(19) Europäisches Patentamt
European Patent Office
Office européen des brevets

(11) EP 4 775 225 A1

(12) **EUROPEAN PATENT APPLICATION**
published in accordance with Art. 153(4) EPC

(43) Date of publication:
**15.07.2026 Bulletin 2026/29**

(21) Application number: **24861967.8**

(22) Date of filing: **03.09.2024**

(51) International Patent Classification (IPC):
***A61K 47/68*** *(2017.01)* ***C07K 7/02*** *(2006.01)*
***A61K 38/08*** *(2019.01)* ***A61P 35/00*** *(2006.01)*
***A61P 35/02*** *(2006.01)*

(86) International application number:
**PCT/CN2024/116631**

(87) International publication number:
**WO 2025/051124 (13.03.2025 Gazette 2025/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.09.2023 CN 202311132387**

(71) Applicant: **Novacyte Therapeutics Co., Ltd**
**Beijing 102206 (CN)**

(72) Inventors:
- **SUN, Binyuan**
  **Beijing 102206 (CN)**
- **REN, Shujian**
  **Beijing 102206 (CN)**
- **HAI, Nan**
  **Beijing 102206 (CN)**
- **HOU, Jingya**
  **Beijing 102206 (CN)**

(74) Representative: **Brann AB**
**P.O. Box 3690**
**Sveavägen 63**
**103 59 Stockholm (SE)**

# (54) AURISTATIN F ANALOGUE, ANTIBODY DRUG CONJUGATE AND USE THEREOF

(57) The invention belongs to the field of biomedicine, and specifically relates to an auristatin F analogue, and an antibody drug conjugate and a use thereof. Specifically, provided are an auristatin F analogue, an antibody drug conjugate thereof, a pharmaceutical composition containing same, a use thereof in preparation of a drug, a preparation method therefor, and an antibody drug conjugate thereof. By means of optimizing the structure of the compound, the tumor cell killing effect of the ADC can be improved, and the therapeutic efficacy and safety of the drug can also be improved.

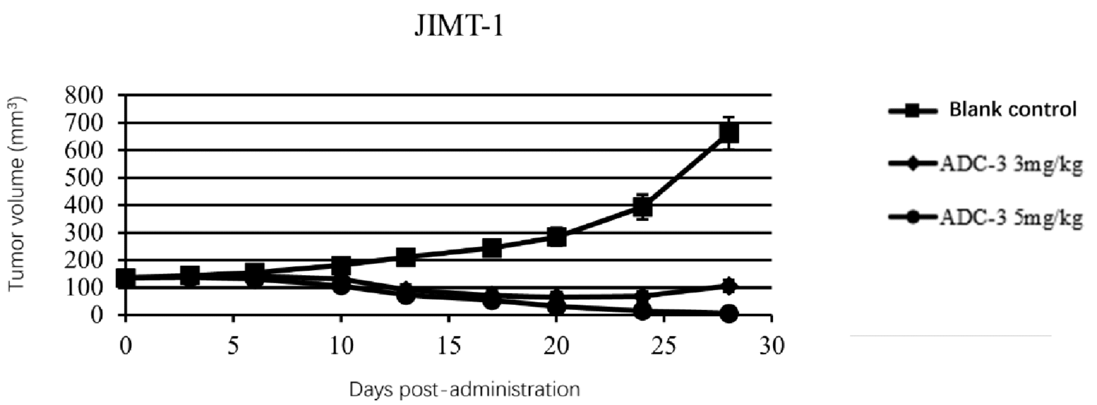


FIG. 1

EP 4 775 225 A1

## Description

### TECHNICAL FIELD

The present disclosure relates to the field of biomedicine, and specifically relates to auristatin F analogs and antibody-drug conjugates and use thereof.

### BACKGROUND

The concept of antibody-drug conjugates (ADCs) was first proposed in 1900 when the ADCs were dubbed "magic bullets". With the advancement of technologies, this technique has been gradually realized and applied to the field of tumor therapy. The ADCs are complexes formed by conjugating, via linkers, small-molecule toxins (payloads) with monoclonal antibodies that specifically target tumor-associated antigens. After entering the body, the ADC molecules can bind to antigens on the surfaces of target cells under the targeting effect of the monoclonal antibodies, and then be internalized into the target cells. Once inside the cells, the ADC molecules can release effector molecules through chemical or enzymatic action to eliminate the target cells. By virtue of their targeting property and intracellular drug release in tumor cells, the ADCs can increase local concentrations of drugs, reduce the toxic and side effects on normal cells, broaden the therapeutic window, and therefore, combine a potent cytotoxic effect of traditional small-molecule chemotherapy with the tumor-targeting ability of antibody-based drugs. This technology has broad application prospects in tumor therapy and has achieved some encouraging results in clinical research.

Linkers in the ADCs are classified into cleavable linkers and non-cleavable linkers. The cleavable linkers can be cleaved by specific enzymes or other mechanisms in the intracellular environment after ADCs enter the cells, and the non-cleavable linkers cannot be cleaved by enzymes or other mechanisms inside the cells. Bystander effect of the ADC drugs refers to a phenomenon that the ADCs can kill not only tumor cells positive for target antigens but also surrounding tumor cells with negative or low expression of target antigens. This phenomenon is of great significance for the treatment of tumor heterogeneous expression, because tumor tissues may contain all cells with high, low or no expression of target antigens.

Auristatin derivatives are depsipeptide derivatives isolated from the Indian Ocean sea hare (Dolabella auricularia), mainly inhibit tumor growth by acting on tubulin, and have activity that is approximately more than 1000 times that of traditional chemotherapeutic drugs. Monomethyl auristatin E (MMAE) and monomethyl auristatin F (MMAF) are the two most commonly used auristatin compounds in research and development of the ADCs. Currently, MMAE or MMAF is used as the payload in five marketed drugs (Adcetris, Polivy, Padcev, Blenrep, and Disitamab Vedotin). Over 20 ADC drugs with auristatin as the payload are under clinical development, accounting for over 50% of the total number of ADC drugs in the research pipeline.

### SUMMARY OF THE INVENTION

A first objective of the present disclosure is to provide a compound (i.e., an auristatin F analog), or an isotopically labeled compound, an optical isomer, a geometric isomer, a tautomer, or a mixture of isomers thereof, or a pharmaceutically acceptable salt or solvate thereof, where the compound includes a structure represented by Formula (I):

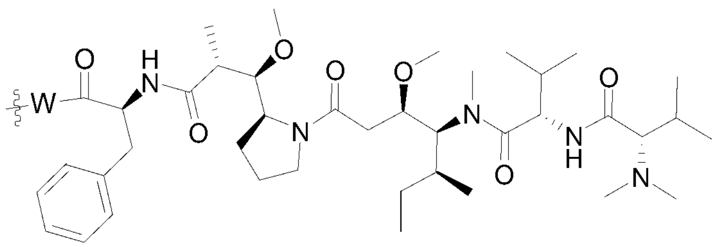

(I)

where W is selected from a single bond, $-X-(CH_2)_{m1}-CR_3R_4-NR_5-$, $-X-CR_3R_4-(CH_2)_{m1}-NR_5-$, $-X-(CH_2O)_{m2}-CR_3R_4-NR_5-$, $-X-CR_3R_4-(CH_2O)_{m2}-NR_5-$, $-X-(CH_2CH_2O)_{m3}-CR_3R_4-NR_5-$, $-X-(CH_2CH_2O)_{m3}-(CH_2)_{m1}-NR_5-$, $-X-CR_3R_4-(CH_2CH_2O)_{m3}-NR_5-$, $-X-(CH_2O)_{m2}-R_3R_4N-$, $-X-(CH_2)_{m1}-R_3R_4N-$, $-XR_5-(CH_2)_{m1}-R_3R_4N-$, $-X-(CH_2)_{m1}-(CH_2O)_{m2}-(CH_2)_{m4}-CR_3R_4-NR_5-$, or $-X-CR_3R_4-(CH_2)_{m1}-(CH_2O)_{m2}-(CH_2)_{m4}-NR_5-$;

X is selected from an alkylene group, an imino group, a nitrogen atom, an oxygen atom, or a sulfur atom;

$R_3$, $R_4$, and $R_5$ are each independently selected from a hydrogen atom, a deuterium atom, halogen, a hydroxyl group, an amino group, a cyano group, a nitro group, a sulfonyl group, a $C_1$ to $C_6$ alkyl group, or a $C_3$ to $C_6$ cycloalkyl group; or

$R_3$ and $R_4$, together with the carbon atom to which they are attached, form a $C_3$ to $C_6$ cycloalkyl group; or $R_3$ and $R_4$, together with the carbon atom and the nitrogen atom to which they are attached, form a 3- to 6-membered heterocyclic group; where in $R_3$, $R_4$, and $R_5$, one or more hydrogen atoms in the $C_1$ to $C_6$ alkyl group or $C_3$ to $C_6$ cycloalkyl group are optionally substituted with a deuterium atom, halogen, a hydroxyl group, a cyano group, an amino group, a $C_1$ to $C_3$ alkyl group, or a phenyl group;
m1, m2, m3, and m4 are each independently selected from an integer from 0 to 6; and
the wavy line attached to W in Formula (I) represents a hydrogen atom or a covalent linkage to a linker unit and/or a structure connecting an antibody or an antigen-binding fragment thereof.

In a preferred embodiment, W is selected from $-X-(CH_2)_{m1}-CR_3R_4-NR_5-$, $-X-CR_3R_4-(CH_2)_{m1}-NR_5-$, $-X-(CH_2CH_2O)_{m3}-(CH_2)_{m1}-NR_5-$, $-X-(CH_2)_{m1}-R_3R_4N-$, $-XR_5-(CH_2)_{m1}-R_3R_4N-$, $-X-(CH_2)_{m1}-(CH_2O)_{m2}-(CH_2)_{m4}-CR_3R_4-NR_5-$, or $-X-CR_3R_4-(CH_2)_{m1}-(CH_2O)_{m2}-(CH_2)_{m4}-NR_5-$;

X is selected from an imino group, a nitrogen atom, or an oxygen atom;
$R_3$ and $R_4$ are each independently selected from a hydrogen atom, a deuterium atom, halogen, a $C_1$ to $C_6$ alkyl group, or a $C_3$ to $C_6$ cycloalkyl group; or $R_3$ and $R_4$, together with the carbon atom to which they are attached, form a $C_3$ to $C_6$ cycloalkyl group; or $R_3$ and $R_4$, together with the carbon atom and the nitrogen atom to which they are attached, form a 3- to 6-membered heterocyclic group; where in $R_3$ and $R_4$, one or more hydrogen atoms in the $C_1$ to $C_6$ alkyl group or $C_3$ to $C_6$ cycloalkyl group are optionally substituted with a deuterium atom, halogen, a $C_1$ to $C_3$ alkyl group, or a phenyl group;
$R_5$ is selected from a hydrogen atom, a deuterium atom, or halogen; and
m1, m2, and m4 are each independently selected from an integer from 1 to 4.

In a preferred embodiment, W is selected from any one of the structures shown below:

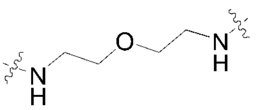 , 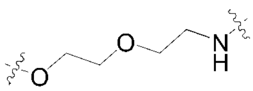 , 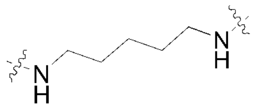 , 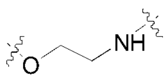 ,

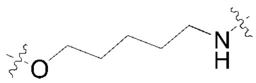 , 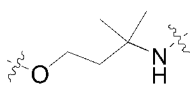 , 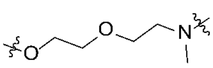 , 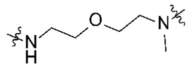 , 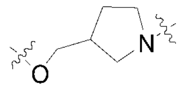 ,

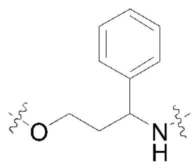 , 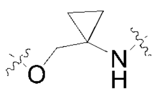 , 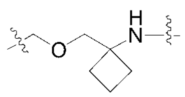 , 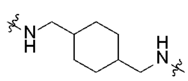 , 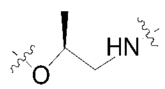 ,

or

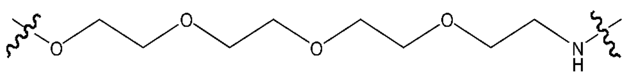 .

In a preferred embodiment, the compound includes a structure represented by Formula (II):

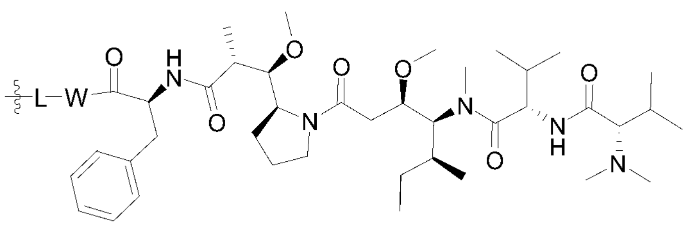

(II)

where W is as defined in any preceding embodiment;

L is selected from $^{NH}$-Val-Cit-$^{C=O}$, $^{NH}$-Val-Ala-$^{C=O}$, $^{NH}$-Val-Lys-$^{C=O}$, $^{NH}$-Val-Lys(Ac)-$^{C=O}$, $^{NH}$-Phe-Lys-$^{C=O}$, $^{NH}$-Phe-Lys(Ac)-$^{C=O}$, $^{NH}$-D-Val-Leu-Lys-$^{C=O}$, $^{NH}$-Gly-Gly-Arg-$^{C=O}$, $^{NH}$-Ala-Ala-Asru-$^{C=O}$, $^{NH}$-Gly-Gly-Gly-$^{C=O}$, $^{NH}$-Gly-Gly-Gly-Gly-Gly-$^{C=O}$, or L1 or L2 shown below, preferably L1 or L2;

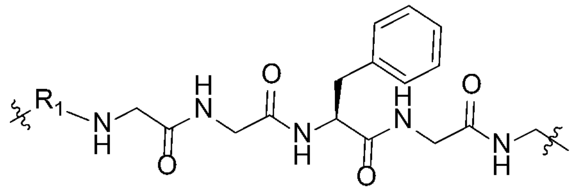

L1

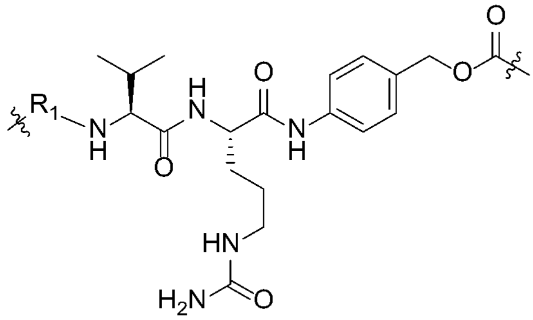

L2

in L1 and L2, $R_1$ is a single bond or has the following structure:

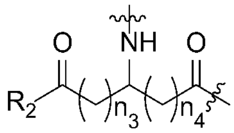

where $n_3$ and $n_4$ are each independently an integer from 0 to 6, and $R_2$ is a hydrophilic group or a structure having a hydrophilic group; and

the wavy line attached to L in Formula (II) represents a hydrogen atom or a covalent linkage to a structure connecting an antibody or an antigen-binding fragment thereof.

In a preferred embodiment, $R_2$ is a hydroxyl group, or a structure having at least three oxygen-containing hydrophilic groups; preferably, the oxygen-containing hydrophilic groups are the same or different and each independently selected from -O- or a hydroxyl group.

Preferably, $R_2$ is any one of the following structures:

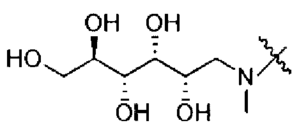

,

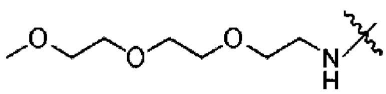

,

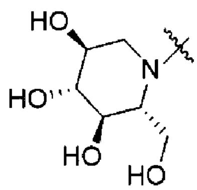

,

or

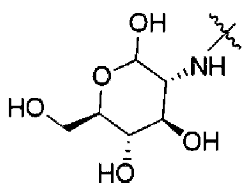

.

In a preferred embodiment, $R_1$ is a single bond or selected from the following structures:

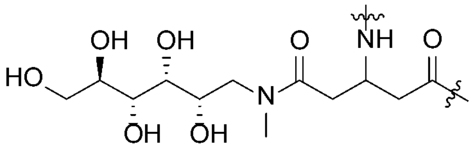,
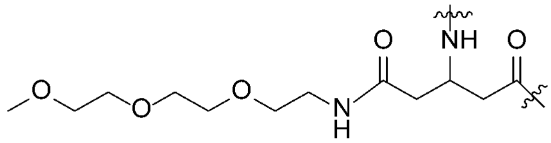,

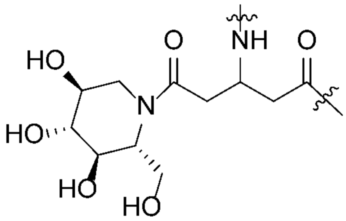,
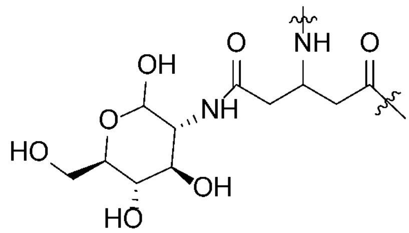,

or

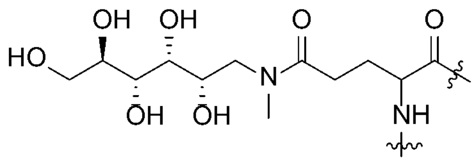.

In a preferred embodiment, when L is L1, X in W is an oxygen atom; in other words, the linking group at the junction of L1 and W is $-CH_2O-$; and when L is L2, X in W is an imino group; in other words, the linking group at the junction of L2 and W is -CONH-. In specific implementation, those skilled in the art can select a matching W structure within the scope of the present disclosure with reference to the description herein.

In a preferred embodiment, the compound includes a structure represented by Formula (III):

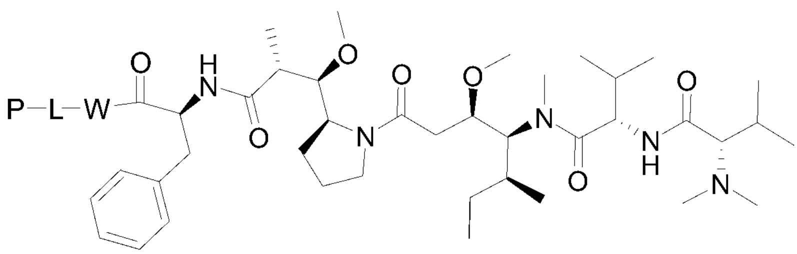

(III)

where P is a structure connecting an antibody or an antigen-binding fragment thereof; and
L and W are as defined in any preceding embodiment.

In a preferred embodiment, P is selected from P1, P2, or P3:

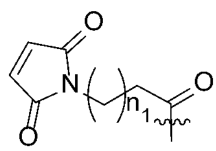

P1

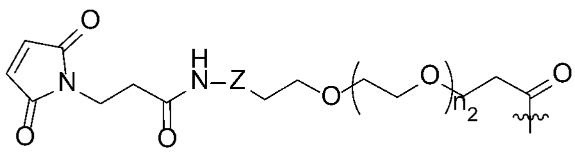

P2

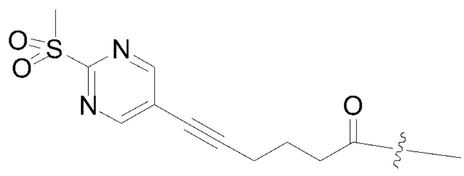

P3

where $n_1$ is an integer from 1 to 10, preferably an integer from 2 to 6;
$n_2$ is an integer from 1 to 12, preferably an integer from 5 to 9; and
Z is selected from a bond, a $C_1$ to $C_6$ alkylene group, an imino group, an ester group, an amide group, a sulfonyl group, a sulfonamide group, a ketone group, a urea group, or a phosphate group.

In a preferred embodiment, the compound has a structure as shown in any one of the following:

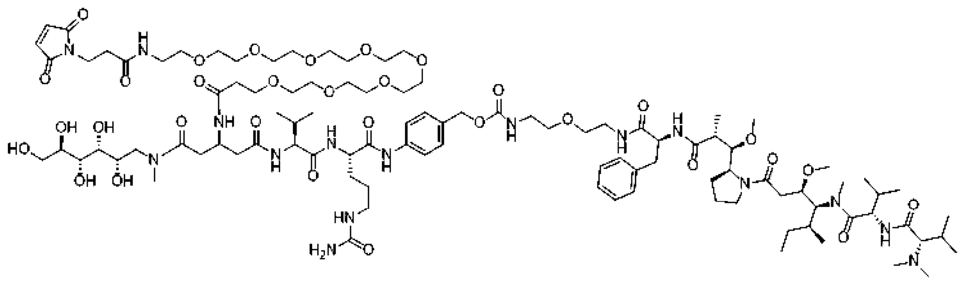

DL-1

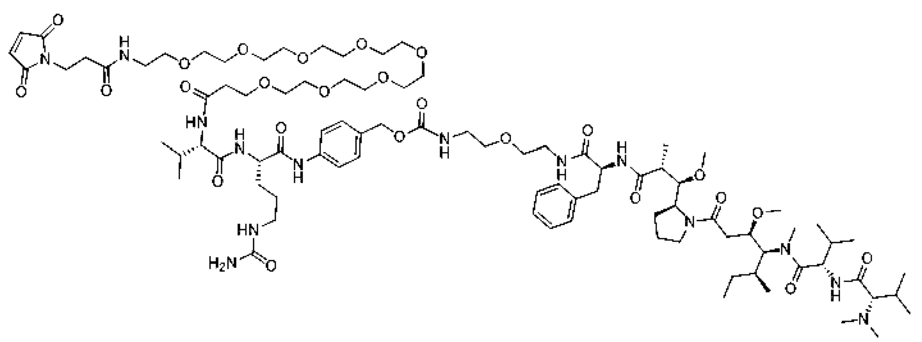

DL-2

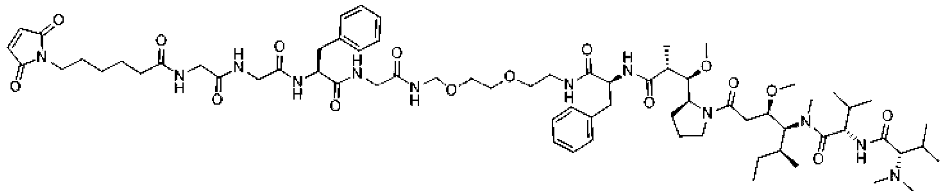

DL-3

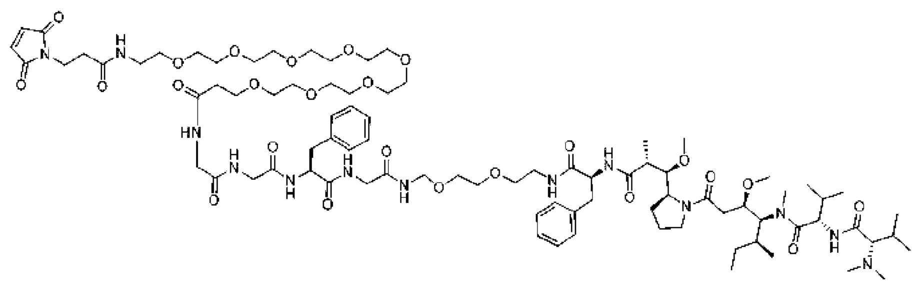

DL-4

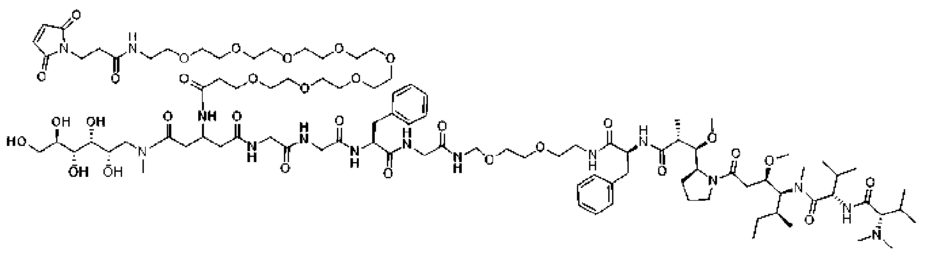

DL-5

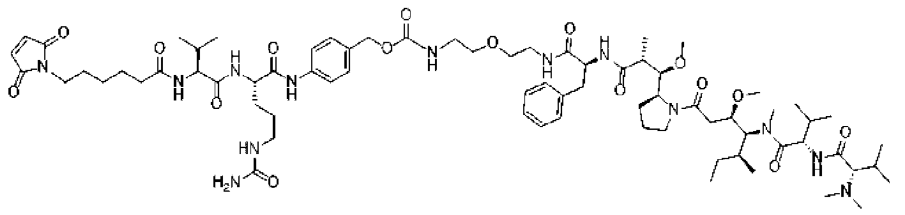

DL-6

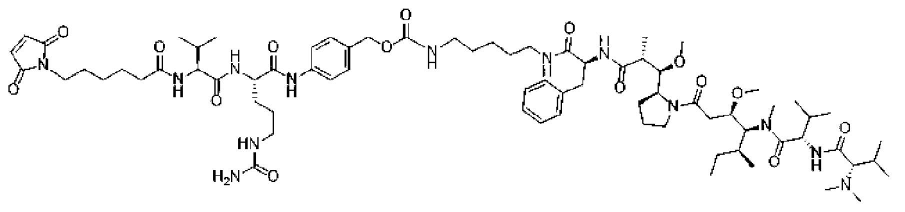

DL-7

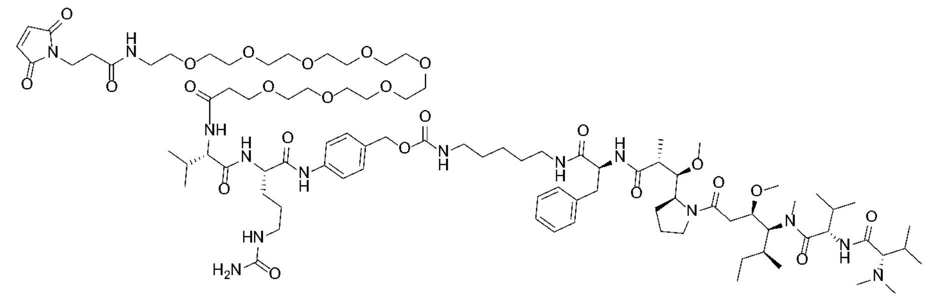

DL-8

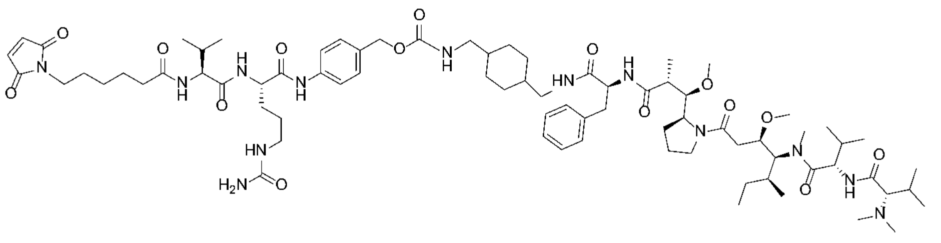

DL-9

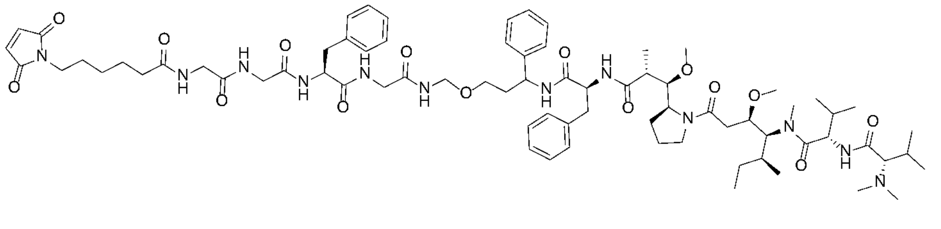

DL-10

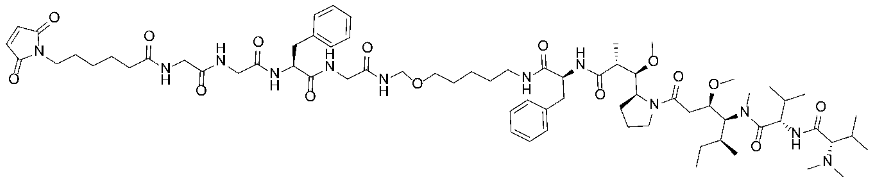

DL-11

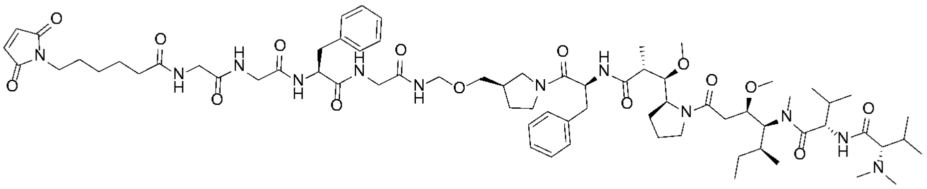

DL-12

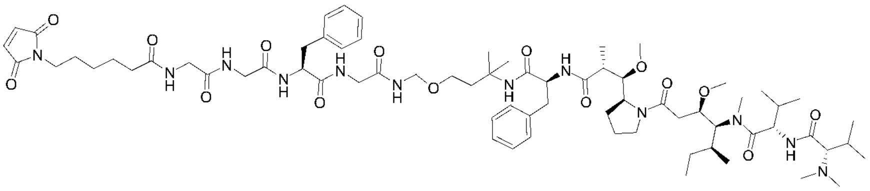

DL-13

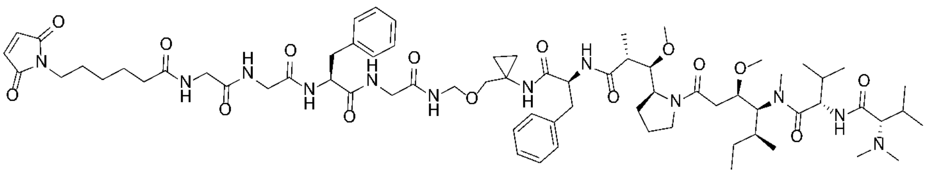

DL-14

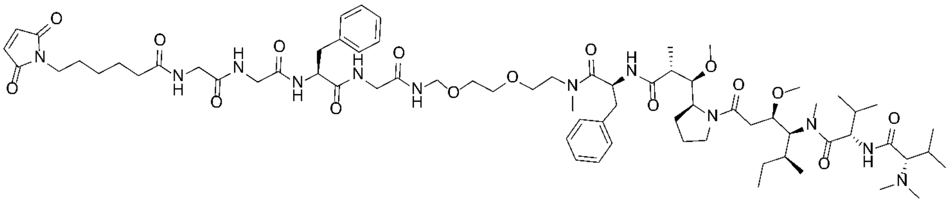

DL-15

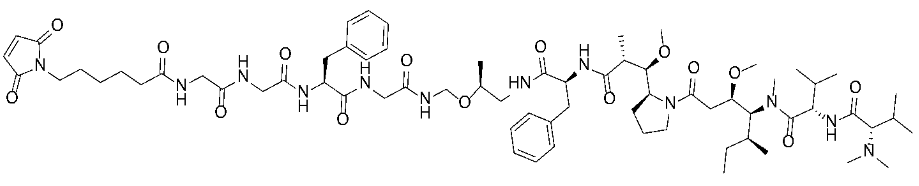

DL-16

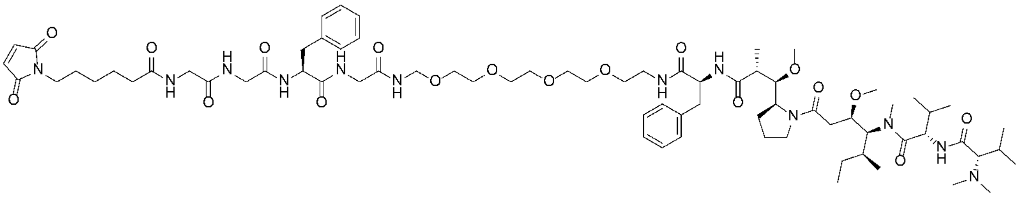

DL-17

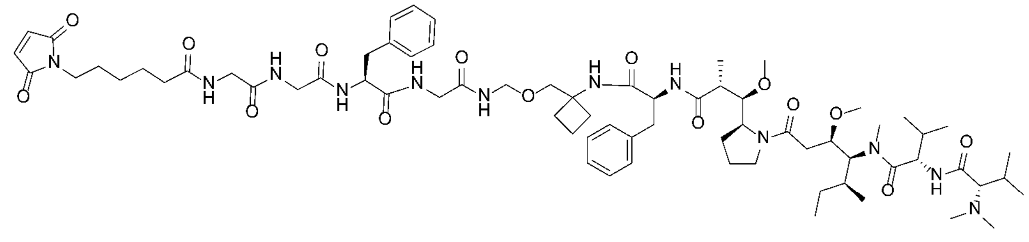

DL-18

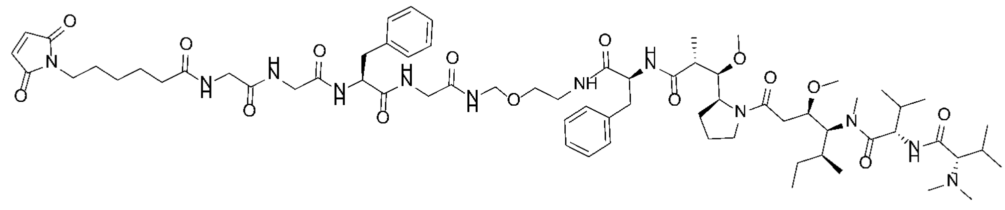

DL-19

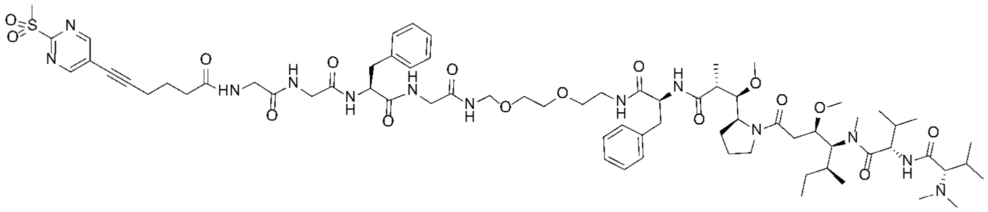

DL-20

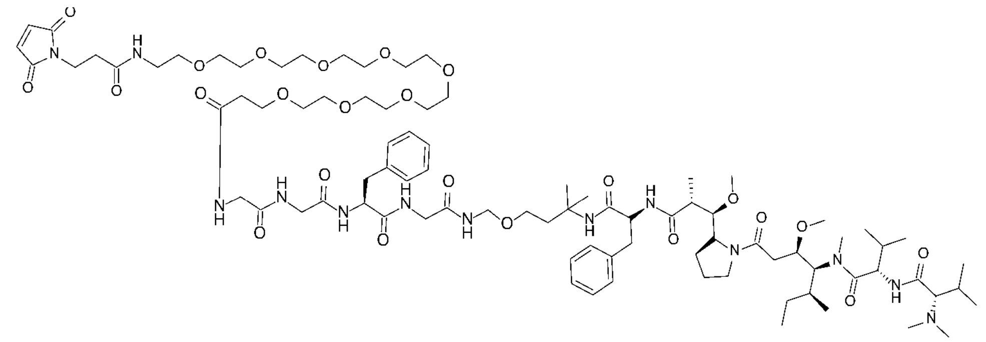

DL-21.

A second objective of the present disclosure is to provide an antibody-drug conjugate, including:

i) an antibody or an antigen-binding fragment thereof; and
ii) the compound, or the isotopically labeled compound, the optical isomer, the geometric isomer, the tautomer, or the mixture of isomers thereof, or the pharmaceutically acceptable salt or solvate thereof as described above.

In a preferred embodiment, the antibody-drug conjugate has a structure represented by Formula (IV):

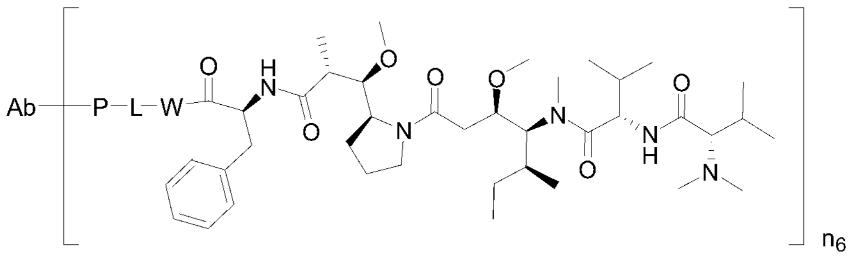

(IV)

where Ab is an antibody or an antigen-binding fragment thereof; and
$n_6$ is selected from an integer or a decimal from 1 to 10.

In a preferred embodiment, $n_6$ is selected from an integer or a decimal from 1 to 8, preferably, $n_6$ is selected from an integer or a decimal from 6 to 8.

In a preferred embodiment, the antibody targets one or more selected from the group consisting of HER2, HER3, B7H3, B7H4, EGFR, Claudin 18.2, c-MET, Nectin-4, ROR1, GPNMB, CD56, TACSTD2 (TROP2), CEACAM5, folate receptor-α, mesothelin, ENPP3, guanylate cyclase C, SLC44A4, NaPi2b, CD70, mucin 1, STEAP1, connexin 4, 5T4, SLTRK6, SC-16, LIV-1, P-cadherin, PSMA, fibronectin extra domain B, endothelin receptor ETB, tenascin C, collagen IV, VEGFR2, periostin, CD30, CD79b, CD19, CD22, CD138, CD37, CD33, CD74, and CD98.

In a preferred embodiment, the antibody is a monoclonal antibody.

A third object of the present disclosure is to provide a pharmaceutical composition, including: the compound, or the isotopically labeled compound, the optical isomer, the geometric isomer, the tautomer, or the mixture of isomers thereof, or the pharmaceutically acceptable salt or solvate thereof as described above, or the antibody-drug conjugate as described above; and one or more pharmaceutically acceptable excipients.

A fourth object of the present disclosure is to provide the use of the compound, or the isotopically labeled compound, the optical isomer, the geometric isomer, the tautomer, or the mixture of isomers thereof, or the pharmaceutically acceptable salt or solvate thereof as described above, or the antibody-drug conjugate as described above in preparation of a medicament.

In a preferred embodiment, the medicament is used for prevention or treatment of a tumor.

Preferably, the tumor is a cancer associated with expression of one or more selected from the group consisting of HER2, HER3, B7H3, B7H4, EGFR, Claudin 18.2, c-MET, Nectin-4, ROR1, GPNMB, CD56, TACSTD2 (TROP2), CEACAM5, folate receptor-α, mesothelin, ENPP3, guanylate cyclase C, SLC44A4, NaPi2b, CD70, mucin 1, STEAP1, connexin 4, 5T4, SLTRK6, SC-16, LIV-1, P-cadherin, PSMA, fibronectin extra domain B, endothelin receptor ETB, tenascin C, collagen IV, VEGFR2, periostin, CD30, CD79b, CD19, CD22, CD138, CD37, CD33, CD74, and CD98.

More preferably, the tumor is one or more selected from the group consisting of breast cancer, ovarian cancer, cervical cancer, uterine cancer, prostate cancer, renal cancer, urethral cancer, bladder cancer, liver cancer, gastric cancer, endometrial cancer, salivary gland cancer, esophageal cancer, melanoma, glioma, neuroblastoma, sarcoma, lung cancer, colon cancer, rectal cancer, colorectal cancer, leukemia, bone cancer, skin cancer, thyroid cancer, pancreatic cancer, and lymphoma.

A fifth object of the present disclosure is to provide a method for preventing or treating a tumor, including: administering an effective amount of the compound, or the isotopically labeled compound, the optical isomer, the geometric isomer, the tautomer, or the mixture of isomers thereof, or the pharmaceutically acceptable salt or solvate thereof as described above, or the antibody-drug conjugate as described above, or the pharmaceutical composition as described above.

In a preferred embodiment, the tumor is a cancer associated with expression of one or more selected from the group consisting of HER2, HER3, B7H3, B7H4, EGFR, Claudin 18.2, c-MET, Nectin-4, ROR1, GPNMB, CD56, TACSTD2 (TROP2), CEACAM5, folate receptor-α, mesothelin, ENPP3, guanylate cyclase C, SLC44A4, NaPi2b, CD70, mucin 1, STEAP1, connexin 4, 5T4, SLTRK6, SC-16, LIV-1, P-cadherin, PSMA, fibronectin extra domain B, endothelin receptor ETB, tenascin C, collagen IV, VEGFR2, periostin, CD30, CD79b, CD19, CD22, CD138, CD37, CD33, CD74, and CD98.

More preferably, the tumor is one or more selected from the group consisting of breast cancer, ovarian cancer, cervical cancer, uterine cancer, prostate cancer, renal cancer, urethral cancer, bladder cancer, liver cancer, gastric cancer, endometrial cancer, salivary gland cancer, esophageal cancer, melanoma, glioma, neuroblastoma, sarcoma, lung cancer, colon cancer, rectal cancer, colorectal cancer, leukemia, bone cancer, skin cancer, thyroid cancer, pancreatic cancer, and lymphoma.

A sixth object of the present disclosure is to provide a method for preparing the compound as described above, where reaction raw materials include a compound containing a structure represented by Formula (I).

In a preferred embodiment, the preparation method includes: reacting a compound containing an L structure and the structure represented by Formula (I) with a compound containing a P structure to obtain the compound as described above; or

reacting a compound containing the P structure and the L structure with a compound containing the structure represented by Formula (I) to obtain the compound as described above.

In a preferred embodiment, the method for preparing the compound as described above includes: reacting a compound containing the L structure with a compound containing the structure represented by Formula (I) to obtain a compound containing the L structure and the structure represented by Formula (I), which is then reacted with a compound containing the P structure to obtain the compound as described above; or

reacting a compound containing the P structure with a compound containing the L structure to obtain a compound containing the P structure and the L structure, which is then reacted with a compound containing the structure represented by Formula (I) to obtain the compound as described above.

A seventh objective of the present disclosure is to provide a method for preparing the antibody-drug conjugate as described above, including: coupling the following i) with ii):

i) an antibody or an antigen-binding fragment thereof; and
ii) the compound, or the isotopically labeled compound, the optical isomer, the geometric isomer, the tautomer, or the mixture of isomers thereof, or the pharmaceutically acceptable salt or solvate thereof as described above.

In a preferred embodiment, the method for preparing the antibody-drug conjugate includes: reducing an interchain disulfide bond of the antibody or the antigen-binding fragment thereof to a free sulfhydryl group, which is then conjugated to a P-terminus of the compound, or the isotopically labeled compound, the optical isomer, the geometric isomer, the tautomer, or the mixture of isomers thereof, or the pharmaceutically acceptable salt or solvate thereof.

In the present disclosure, structural optimization of the compound can not only enhance the killing effect of the ADC on tumor cells, but also improve efficacy and safety of the drug.

## BRIEF DESCRIPTION OF THE DRAWINGS

To describe the technical solutions in the specific embodiments of the present disclosure or in the prior art more clearly, the following briefly describes the accompanying drawings required for describing the specific embodiments or the prior art. Apparently, the accompanying drawings in the following description show some embodiments of the present disclosure, and persons of ordinary skills in the art may still derive other drawings from these accompanying drawings without creative efforts.

FIG. 1 shows efficacy evaluation results of the ADC drug in a JIMT-1 human breast cancer model in Test Example 3 of the present disclosure.
FIG. 2 shows safety evaluation results of the ADC drug in a JIMT-1 human breast cancer model in Test Example 3 of the present disclosure.

## DETAILED DESCRIPTION

The specific embodiments of the present disclosure are described in detail hereinafter. It should be understood that, the specific embodiments described herein are only intended to illustrate and explain the present disclosure, rather than limiting the present disclosure. Persons skilled in the art can make various modifications and changes to the present disclosure without departing from the scope or spirit of the present disclosure. For example, features illustrated or described as a part of an embodiment can be used in another embodiment to create still another embodiment.

Unless otherwise specified, all terms (including technical and scientific terms) used to disclose the present disclosure have the same meanings as commonly understood by persons of ordinary skills in the art to which the present disclosure belongs. With reference to further guidance, the following definitions are used to better understand teachings in the present disclosure. The terms used herein in the description of the present disclosure are only for the purpose of describing specific examples, and are not intended to limit the present disclosure.

The term "and/or" as used herein refers to a selection range that includes any one of the listed items among two or more related items, as well as any and all combinations of those listed items. These combinations may include any two of the related items, any greater number of the related items, or all of the related items. In the present disclosure, it should be understood that when at least two items are connected by "and/or", the technical solutions undoubtedly include technical solutions connected by logical "AND", and also undoubtedly include technical solutions connected by logical "OR". For example, "A and/or B" includes three parallel solutions: A, B, and both A and B. For another example, technical solutions of "A, B, C and/or D" include any one of A, B, C or D (i.e., a technical solution logically conjoined by "or"), and also include a combination of any one and all of A, B, C and D, that is, a combination of any two or three of A, B, C and D and a combination of four of A, B, C and D (i.e., technical solutions logically conjoined by "AND").

The terms "comprise", "include", and "contain", as well as their variations, as used herein are synonymous, and are inclusive or open-ended, and do not exclude additional uncited members, elements, or method steps.

In the present disclosure, a numerical range defined by endpoints includes all values and fractions within the range, as well as the cited endpoints.

In the present disclosure, concentration values refer to values that may fluctuate within a certain range. For example, fluctuations may occur within the corresponding precision range. For instance, a value of 2% may allow fluctuations within $\pm$0.1%. For larger values or values that do not require precise control, the meaning may include greater fluctuations. For example, a value of 100 mM may allow fluctuations within $\pm$1%, $\pm$2%, $\pm$5%, etc. For molecular weight, the meaning may include fluctuations within $\pm$10%.

In the present disclosure, descriptions such as "multiple" and "a plurality of" refer to two or more than two, unless otherwise specified.

In the present disclosure, technical features described in an open-ended manner encompass both closed technical solutions consisting of the listed features and open technical solutions that include the listed features.

In the present disclosure, expressions such as "preferably", "more preferably", "most preferably", or "desirably" are used solely to describe embodiments or examples with better effects, and shall not be construed as limiting the scope of protection of the invention. In the present disclosure, "optionally" and "optional" refer to dispensable or any one of two parallel solutions of "with" and "without". If "optional" appears multiple times in a technical solution, "optional" at each location is independent unless specific explanation is provided and there are contradictions or mutual constraints.

In the present disclosure, the term "antibody" refers to an immunoglobulin, which is a tetrapeptide chain structure formed by bonding two identical heavy chains with two identical light chains through interchain disulfide bonds. Immunoglobulins have heavy chain constant regions with different amino acid compositions and sequences, and therefore, also have different antigenicities.

In the present disclosure, the term "antigen-binding fragment" refers to one or more fragments of an antibody that maintain the ability to bind specifically to the antigen. The antigen-binding ability of the antibody has been demonstrated by using a fragment of a full-length antibody. Examples of binding fragments included in the "antigen-binding fragment" include: (i) a Fab fragment, a monovalent fragment consisting of VL, VH, CL, and CH1 domains; (ii) an $F(ab')_2$ fragment, including a bivalent fragment of two Fab fragments bound by a disulfide bridge in the hinge region; (iii) an Fd fragment consisting of VH and CH1 domains; (iv) an Fv fragment consisting of VH and VL domains on a single arm of the antibody; (v) a single-domain or dAb fragment consisting of a VH domain; and (vi) a separated complementary-determining region (CDR) or (vii) a combination of two or more separated CDRs that can optionally be bound by a synthesized linker. In addition, although both VL and VH domains of the Fv fragment are encoded by separate genes, the VL and VH domains can be bound in a recombinant method through synthesized linkers, so that the VL and VH domains can generate a single protein chain (referred to as single-stranded Fv (scFv)) for pairing the VL and VH regions to form monovalent molecules. Such single-chain antibodies are also incorporated into the term "antigen-binding fragment" of the antibody.

In the present disclosure, the "pharmaceutically acceptable salt" includes its acid addition salts and base addition salts. Suitable acid addition salts are formed from acids that form pharmaceutically acceptable salts. Examples include aspartate, benzoate, bicarbonate/carbonate, glucoheptonate, gluconate, nitrate, orotate, palmitate, and other similar salts. Suitable base addition salts are formed from bases that form pharmaceutically acceptable salts. Examples include aluminum salt, arginine salt, choline salt, magnesium salt, and other similar salts. For a review of suitable salts, see Handbook of Pharmaceutical Salts: Properties, Selection, and Use by Stahl and Wermuth (Wiley-VCH, 2002). Methods for preparing the pharmaceutically acceptable salts of the compounds according to the present disclosure are known to persons skilled in the art.

In the present disclosure, the term "isomer" includes stereoisomers, which refer to isomers formed via at least one asymmetric center. In compounds having one or more (for example, one, two, three, or four) asymmetric centers, racemic mixtures, single enantiomers, diastereomeric mixtures, and individual diastereomers may be formed. Certain individual molecules may also exist as (cis/trans) geometric isomers. Similarly, the compounds according to the present disclosure may exist as a mixture of two or more structurally distinct forms in rapid equilibrium, commonly referred to as tautomers. Representative examples of tautomers include keto-enol tautomers, phenol-keto tautomers, nitroso-oxime tautomers, imine-enamine tautomers, and the like. The scope of this application covers all such isomers or mixtures thereof in any proportion (for example, 60%, 65%, 70%, 75%, 80%, 85%, 90%, 95%, 96%, 97%, 98%, or 99%).

Solid lines, solid wedges, or dashed wedges may be used herein to illustrate the carbon-carbon bonds of the compounds according to the present disclosure. The use of the solid line to illustrate a bond attached to an asymmetric carbon atom is intended to indicate that all possible stereoisomers at the carbon atom are included (for example, specific enantiomers and racemic mixtures). The use of the solid or dashed wedge to illustrate a bond attached to an asymmetric carbon atom indicates the presence of the stereoisomer shown. When present in a racemic mixture, solid and dashed wedges are used to define relative stereochemistry other than absolute stereochemistry. Unless otherwise specified, the compounds according to the present disclosure may exist in forms of stereoisomers, including cis and trans isomers, optical isomers (for example, R and S enantiomers), diastereomers, geometric isomers, rotamers, conformers, atropisomers, and mixtures thereof. The compounds according to the present disclosure may exhibit more than one type of isomerism and may consist of their mixtures (for example, racemic mixtures and pairs of diastereomers).

In the present disclosure, the term "solvate" refers to a pharmaceutically acceptable solvate formed by the ligand-drug conjugate compound according to the present disclosure and one or more solvent molecules. Non-limiting examples of the solvent molecules include water, ethanol, acetonitrile, isopropanol, DMSO, and ethyl acetate.

Pharmaceutically acceptable isotopically-labelled compounds also fall within the scope of the present disclosure and are identical to the compound according to the present disclosure except that one or more of their atoms are replaced by an atom having the same atomic number but an atomic mass or mass number different from that which predominates in nature. Examples of isotopes suitable for incorporation into the compounds according to the present disclosure include, but are not limited to, isotopes of hydrogen (for example, deuterium ($^{2}H$) and tritium ($^{3}H$)), isotopes of carbon (for example, $^{11}C$, $^{13}C$, and $^{14}C$), isotopes of chlorine (for example, $^{36}Cl$), isotopes of fluorine (for example, $^{18}F$); isotopes of iodine (for example, $^{123}I$ and $^{125}I$), isotopes of nitrogen (for example, $^{13}N$ and $^{15}N$), isotopes of oxygen (for example, $^{15}O$, $^{17}O$, and $^{18}O$), isotopes of phosphorus (for example, $^{32}P$), and isotopes of sulfur (for example, $^{35}S$).

Esters of the compounds according to the present disclosure also fall within the scope of the present disclosure, including physiologically hydrolysable esters (which may be hydrolyzed under physiological conditions to release the compounds according to the present disclosure in a form of a free acid or alcohol).

Metabolites of the compounds according to the present disclosure also fall within the scope of the present disclosure, i.e., substances formed in the body upon administration of the compounds according to the present disclosure. Such products may result from, for example, oxidation, reduction, hydrolysis, amidation, deamidation, esterification, and enzymatic hydrolysis of the administered compound. Therefore, the present disclosure includes a metabolite of the compound according to the present disclosure, including a compound prepared by a method of contacting the compound according to the present disclosure with a mammal for a period sufficient to produce a metabolite thereof.

Prodrugs of the compounds according to the present disclosure also fall within the scope of the present disclosure, which are certain derivatives of the compounds according to the present disclosure that may have low or no pharmacological activity per se and that can be converted into the compounds according to the present disclosure with the desired activity through, for example, hydrolytic cleavage when administered to or applied onto the body. Such prodrugs are typically functional group derivatives of the compounds, which are easy to convert *in vivo* into the desired therapeutically active compounds.

The present disclosure also encompasses the compounds according to the present disclosure containing protecting groups. In any process for preparing the compounds according to the present disclosure, it may be necessary and/or desirable to protect sensitive or reactive groups on any relevant molecule, thereby forming a chemically protected form of the compounds according to the present disclosure. This can be implemented by conventional protecting groups, for example protecting groups described by T.W. Greene & 20 P.G.M. Wuts in Protective Groups in Organic Synthesis (John Wiley & Sons, 1991), which is incorporated herein by reference. The protecting groups may be removed at a suitable subsequent stage in methods known in the art.

The term "alkyl group" refers to a saturated aliphatic hydrocarbon group, which includes straight-chain and branched-chain groups.

The term "alkoxy group" refers to -O- (alkyl group) and -O- (non-substituted cycloalkyl group), where the alkyl group or cycloalkyl group is as defined above.

The term "cycloalkyl group" refers to a saturated or partially unsaturated monocyclic or polycyclic hydrocarbon substituent.

The term "heterocyclic group" refers to a saturated or partially unsaturated monocyclic or polycyclic hydrocarbon substituent including 3 to 6 ring atoms, where one or more ring atoms are heteroatoms selected from nitrogen, oxygen, or S(O) m (m is an integer from 0 to 2), excluding ring moieties of -O-O-, -O-S-, or -S-S-, and the other ring atoms are carbon. Preferably, 1 to 4 of the ring atoms are heteroatoms. The term "halogen" refers to fluorine, chlorine, bromine, or iodine.

The term "hydroxyl group" refers to -OH.

The term "cyano group" refers to -CN.

The term "amino group" refers to $-NH_2$.

The term "phenyl group" refers to

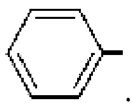

.

The phrase "comprising a maleimide linker" refers to containing a maleimide group.

In the present disclosure, the term "substitution" means that one or more (for example, one, two, three, or four) hydrogen atoms on a specified atom are selectively substituted from the specified group, provided that a normal valence of the specified atom in such a case is not exceeded and such a combination results in a stable compound. A combination of substituents and/or variables is allowable only if such combination results in a stable compound.

In the present disclosure, if a substituent is described as "optionally substituted with...", the substituent may be unsubstituted or substituted. If a carbon atom of the substituent is described as being optionally substituted by one or more substituents from a list of substituents, one or more hydrogen atoms on the carbon atom (to the extent of any hydrogen atoms present) may be replaced, individually and/or collectively, with an independently selected optional substituent. If a nitrogen atom of the substituent is described as being optionally substituted with one or more substituents from the list of substituents, one or more hydrogen atoms on the nitrogen atom (to the extent of any hydrogen atoms present) may each be replaced with an independently selected optional substituent.

In the present disclosure, if the substituent is described as being "independently selected from" a group of groups, each substituent is selected independently of the others. Accordingly, each substituent may be the same as or different from any other substituent.

The present disclosure first provides a compound (i.e., an auristatin F analog), or an isotopically labeled compound, an optical isomer, a geometric isomer, a tautomer, or a mixture of isomers thereof, or a pharmaceutically

acceptable salt or solvate thereof, where the compound includes a structure represented by Formula (I):

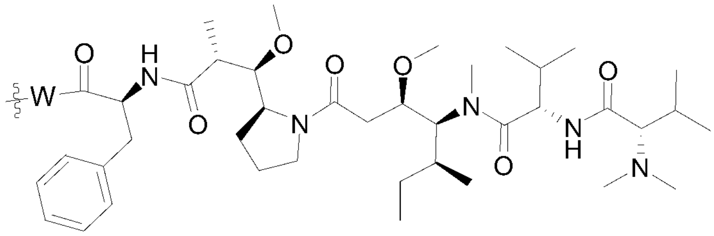

(I)

where W is selected from a single bond, $-X-(CH_2)_{m1}-CR_3R_4-NR_5-$, $-X-CR_3R_4-(CH_2)_{m1}-NR_5-$, $-X-(CH_2O)_{m2}-CR_3R_4-NR_5-$, $-X-CR_3R_4-(CH_2O)_{m2}-NR_5-$, $-X-(CH_2CH_2O)_{m3}-CR_3R_4-NR_5-$, $-X-(CH_2CH_2O)_{m3}-(CH_2)_{m}i-NR_5-$, $-X-CR_3R_4-(CH_2CH_2O)_{m3}-NR_5-$, $-X-(CH_2O)_{m2}-R_3R_4N-$, $-X-(CH_2)_{m1}-R_3R_4N-$, $-XR_5-(CH_2)_{m1}-R_3R_4N-$, $-X-(CH_2)_{m1}-(CH_2O)_{m2}-(CH_2)_{m4}-CR_3R_4-NR_5-$, or $-X-CR_3R_4-(CH_2)_{m1}-(CH_2O)_{m2}-(CH_2)_{m4}-NR_5-$;

X is selected from an alkylene group, an imino group, a nitrogen atom, an oxygen atom, or a sulfur atom;

$R_3$, $R_4$, and $R_5$ are each independently selected from a hydrogen atom, a deuterium atom, halogen, a hydroxyl group, an amino group, a cyano group, a nitro group, a sulfonyl group, a $C_1$ to $C_6$ alkyl group, or a $C_3$ to $C_6$ cycloalkyl group; or $R_3$ and $R_4$, together with the carbon atom to which they are attached, form a $C_3$ to $C_6$ cycloalkyl group; or $R_3$ and $R_4$, together with the carbon atom and the nitrogen atom to which they are attached, form a 3- to 6-membered heterocyclic group; where in $R_3$, $R_4$, and $R_5$, one or more hydrogen atoms in the $C_1$ to $C_6$ alkyl group or $C_3$ to $C_6$ cycloalkyl group are optionally substituted with a deuterium atom, halogen, a hydroxyl group, a cyano group, an amino group, a $C_1$ to $C_3$ alkyl group, or a phenyl group;

m1, m2, m3, and m4 are each independently selected from an integer from 0 to 6; and

the wavy line attached to W in Formula (I) represents a hydrogen atom or a covalent linkage to a linker unit and/or a structure connecting an antibody or an antigen-binding fragment thereof.

In some embodiments, W is selected from $-X-(CH_2)_{m1}-CR_3R_4-NR_5-$, $-X-CR_3R_4-(CH_2)_{m1}-NR_5-$, $-X-(CH_2CH_2O)_{m3}-(CH_2)_{m1}-NR_5-$, $-X-(CH_2)_{m1}-R_3R_4N-$, $-XR_5-(CH_2)_{m1}-R_3R_4N-$, $-X-(CH_2)_{ml}-(CH_2O)_{m2}-(CH_2)_{m4}-CR_3R_4-NR_5-$, or $-X-CR_3R_4-(CH_2)_{m1}-(CH_2O)_{m2}-(CH_2)_{m4}-NR_5-$;

X is selected from an imino group, a nitrogen atom, or an oxygen atom;

$R_3$ and $R_4$ are each independently selected from a hydrogen atom, a deuterium atom, halogen, a $C_1$ to $C_6$ alkyl group, or a $C_3$ to $C_6$ cycloalkyl group; or $R_3$ and $R_4$, together with the carbon atom to which they are attached, form a $C_3$ to $C_6$ cycloalkyl group; or $R_3$ and $R_4$, together with the carbon atom and the nitrogen atom to which they are attached, form a 3- to 6-membered heterocyclic group; where in $R_3$ and $R_4$, one or more hydrogen atoms in the $C_1$ to $C_6$ alkyl group or $C_3$ to $C_6$ cycloalkyl group are optionally substituted with a deuterium atom, halogen, a $C_1$ to $C_3$ alkyl group, or a phenyl group;

$R_5$ is selected from a hydrogen atom, a deuterium atom, or halogen; and

m1, m2, and m4 are each independently selected from an integer from 1 to 4.

In some embodiments, W is selected from any one of the structures shown below:

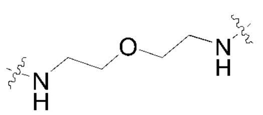 , 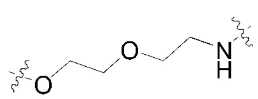 , 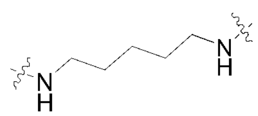 , 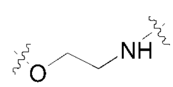 ,

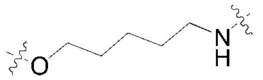 , 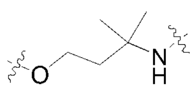 , 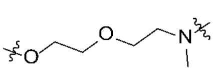 , 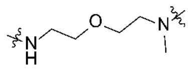 , 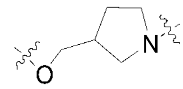 ,

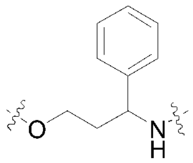, 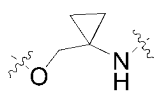, 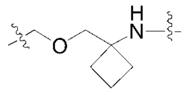, 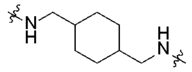, 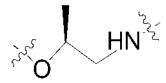,

or

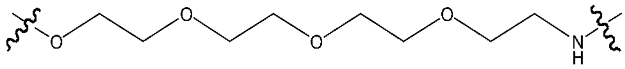.

In some embodiments, the compound includes a structure represented by Formula (II):

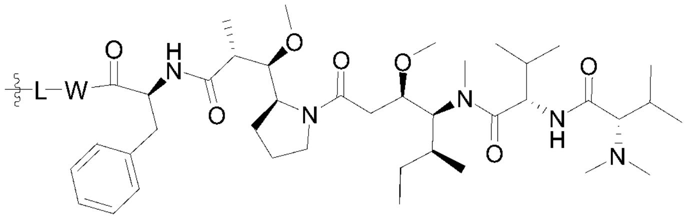

(II)

where W is as defined in any preceding embodiment;

L is selected from $^{NH}$-Val-Cit-$^{C=O}$, $^{NH}$-Val-Ala-$^{C=O}$, $^{NH}$-Val-Lys-$^{C=O}$, $^{NH}$-Val-Lys(Ac)-$^{C=O}$, $^{NH}$-Phe-Lys-$^{C=O}$, $^{NH}$-Phe-Lys(Ac)-$^{C=O}$, $^{NH}$-D-Val-Leu-Lys-$^{C=O}$, $^{NH}$-Gly-Gly-Arg-$^{C=O}$, $^{NH}$-Ala-Ala-Asru-$^{C=O}$, $^{NH}$-Gly-Gly-Gly-$^{C=O}$, $^{NH}$-Gly-Gly-Gly-Gly-Gly-$^{C=O}$, or L1 or L2 shown below, preferably L1 or L2;

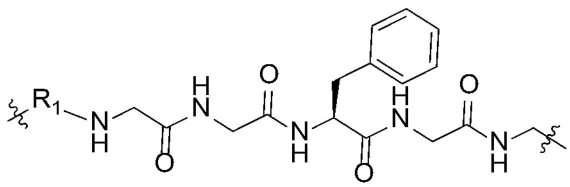

L1

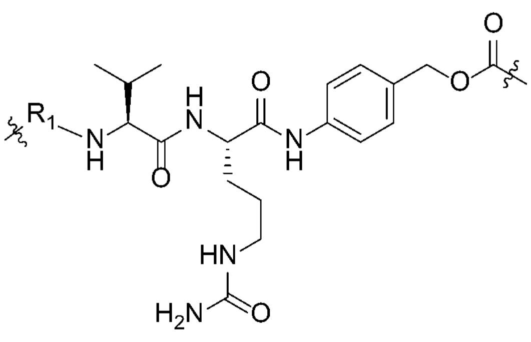

L2

in L1 and L2, $R_1$ is a single bond or has the following structure:

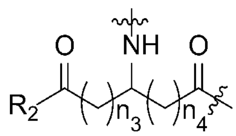

where $n_3$ and $n_4$ are each independently an integer from 0 to 6, and $R_2$ is a hydrophilic group or a structure having a hydrophilic group; and

the wavy line attached to L in Formula (II) represents a hydrogen atom or a covalent linkage to a structure connecting an antibody or an antigen-binding fragment thereof.

In some embodiments, $R_2$ is a hydroxyl group, or a structure having at least three oxygen-containing hydrophilic groups; preferably, the oxygen-containing hydrophilic groups are the same or different and each independently selected from -O- or a hydroxyl group;

preferably, $R_2$ is any one of the following structures:

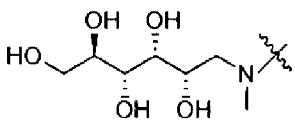
,
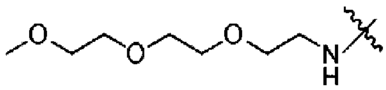
,
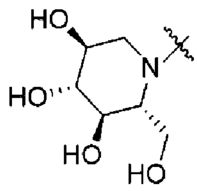
,

or

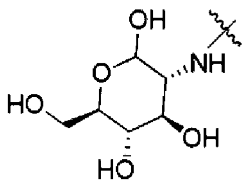
.

In some embodiments, $R_1$ is a single bond or selected from the following structures:

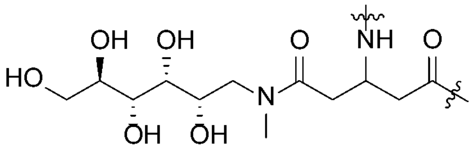
,
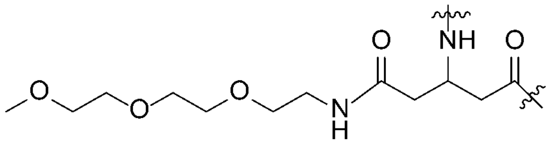
,

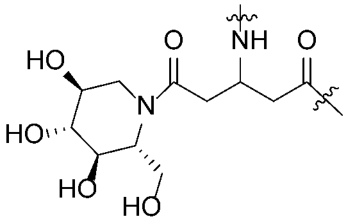
,
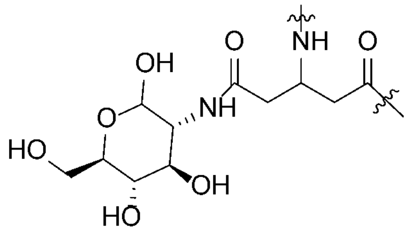
,

or

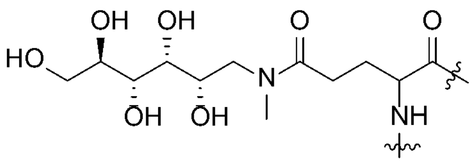
.

In some embodiments, when L is L1, X in W is an oxygen atom; and when L is L2, X in W is an imino group.

In some embodiments, the compound includes a structure represented by Formula (III):

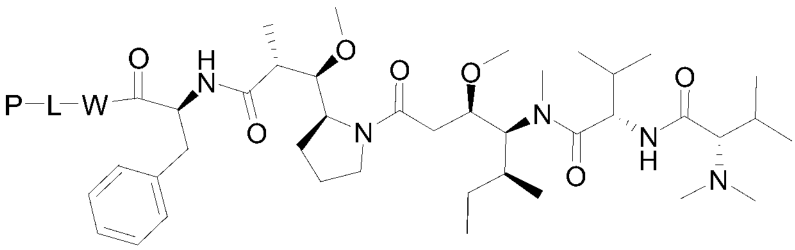

(III)

where P is a structure connecting an antibody or an antigen-binding fragment thereof; and
L and W are as defined in any preceding embodiment.

In some embodiments, P is a moiety containing a maleimide linker. For example, in some embodiments, P is selected from P1 or P2:

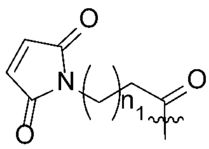

P1

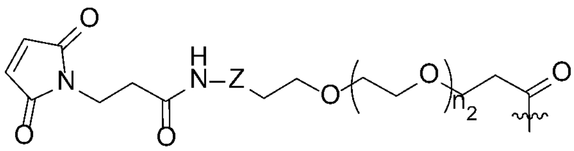

P2;

in some embodiments, P is P3:

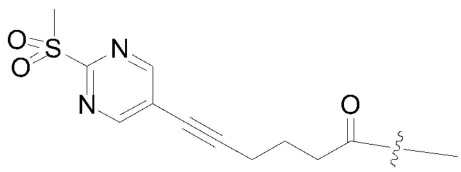

P3,

where $n_1$ is an integer from 1 to 10, preferably an integer from 2 to 6;
$n_2$ is an integer from 1 to 12, preferably an integer from 5 to 9; and
Z is selected from a bond, a $C_1$ to $C_6$ alkylene group, an imino group, an ester group, an amide group, a sulfonyl group, a sulfonamide group, a ketone group, a urea group, or a phosphate group.

In some embodiments, the compound has a structure as shown in any one of the following:

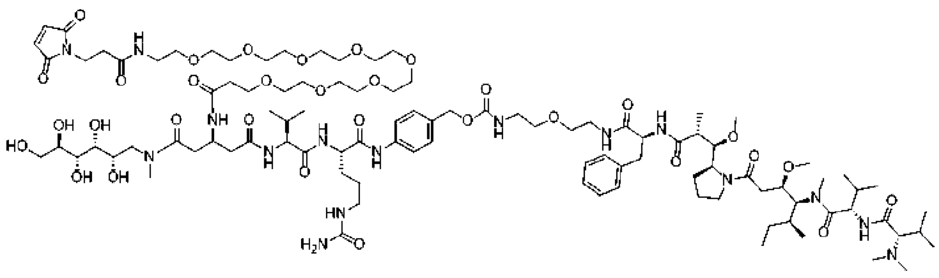

DL-1

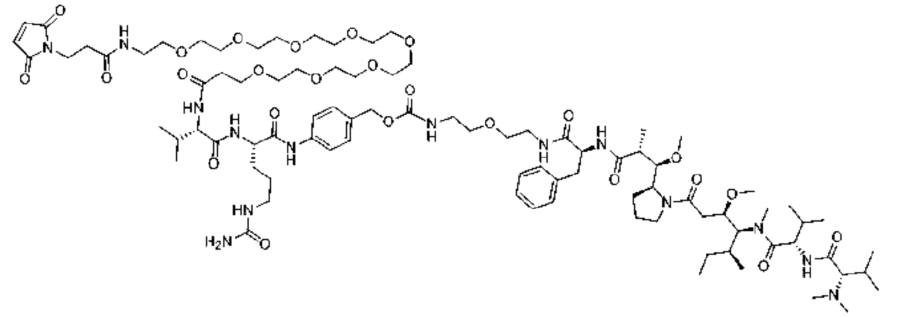

DL-2

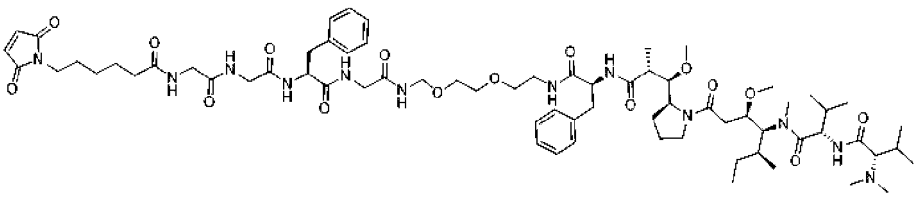

DL-3

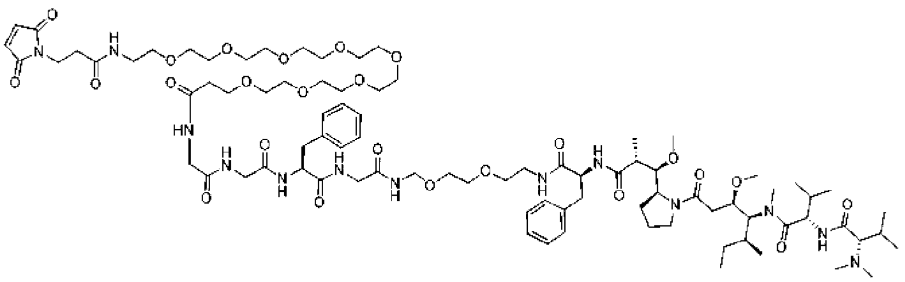

DL-4

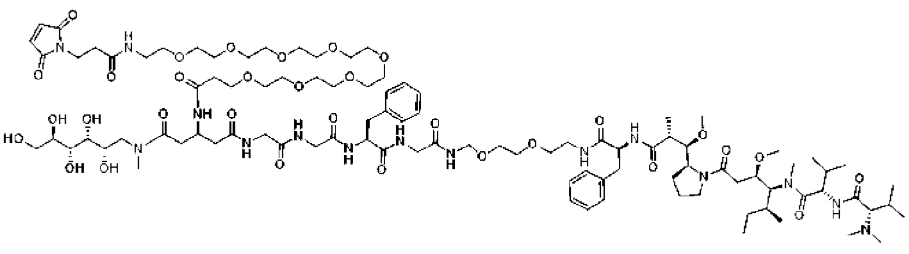

DL-5

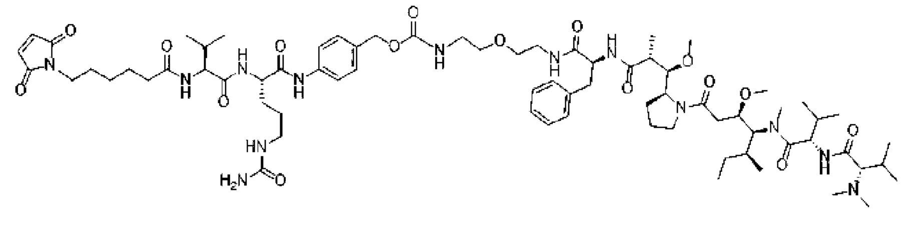

DL-6

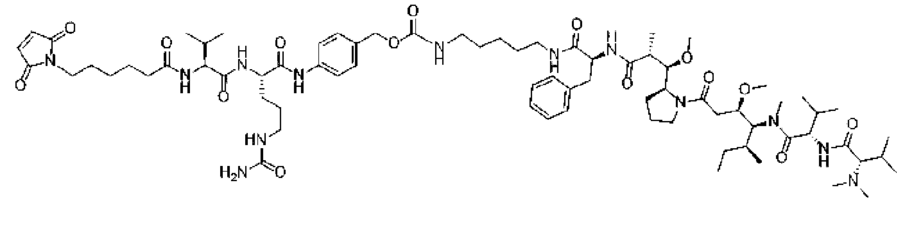

DL-7

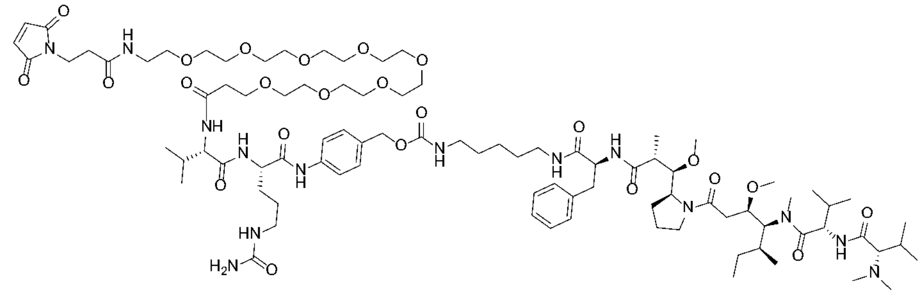

DL-8

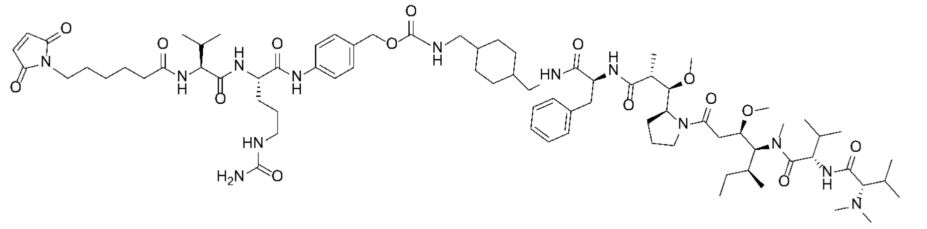

DL-9

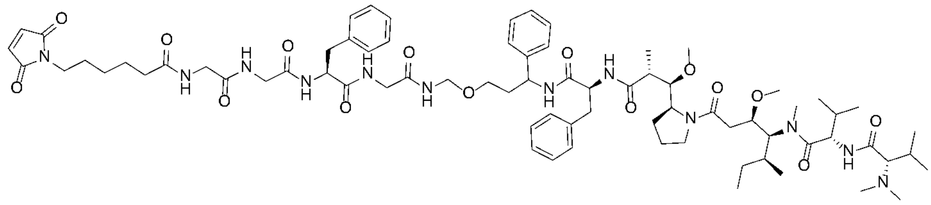

DL-10

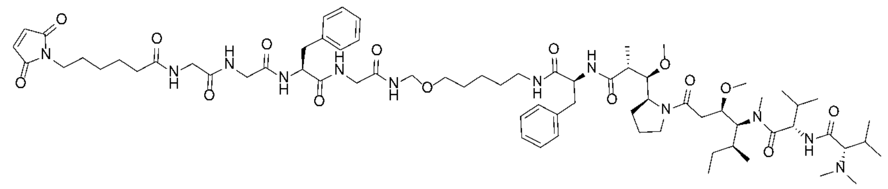

DL-11

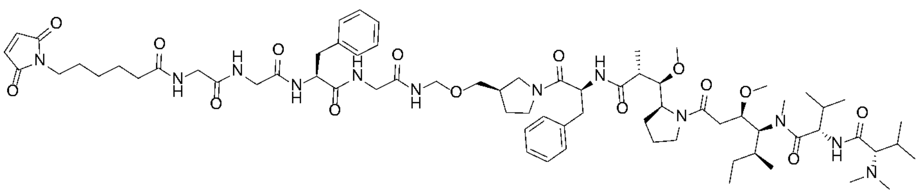

DL-12

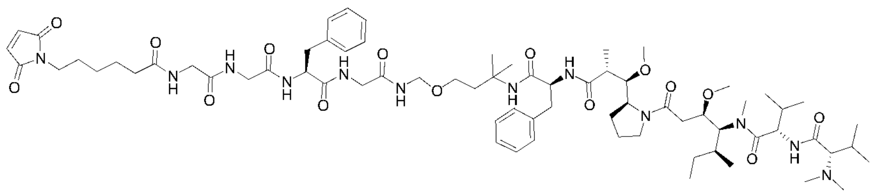

DL-13

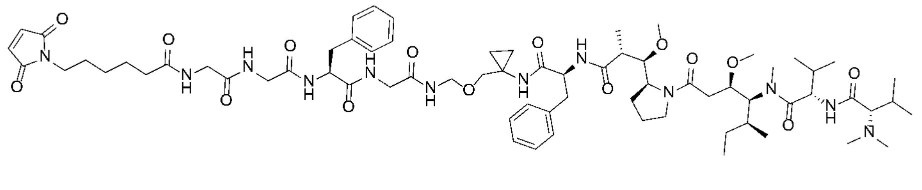

DL-14

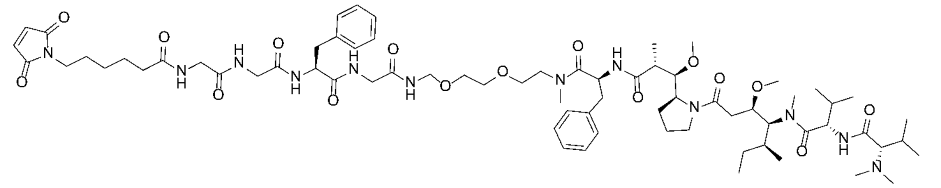

DL-15

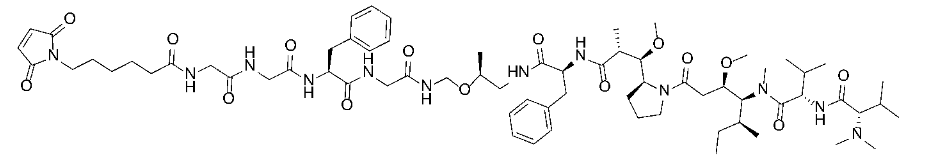

DL-16

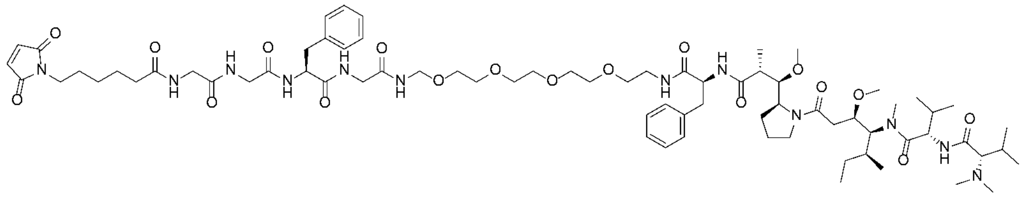

DL-17

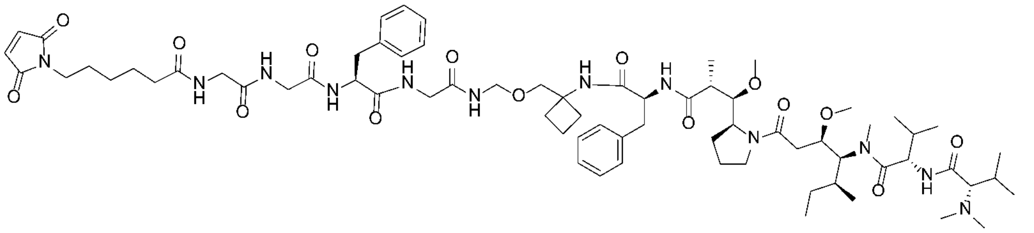

DL-18

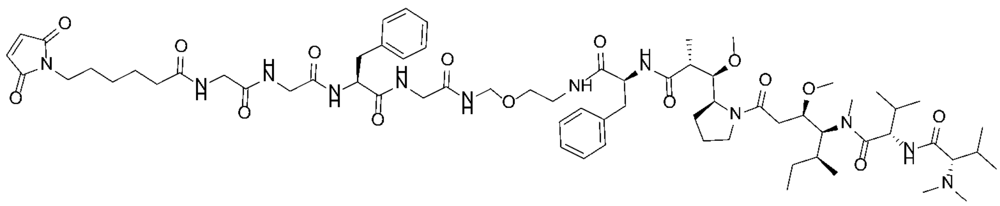

DL-19

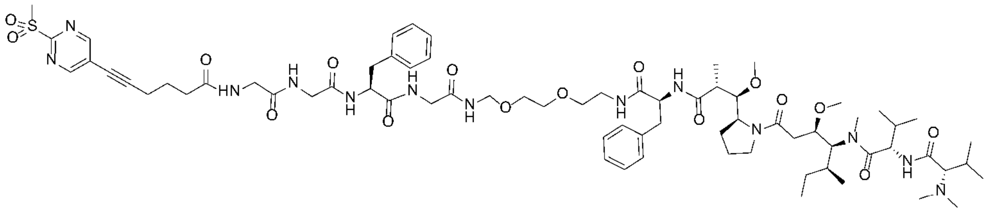

DL-20

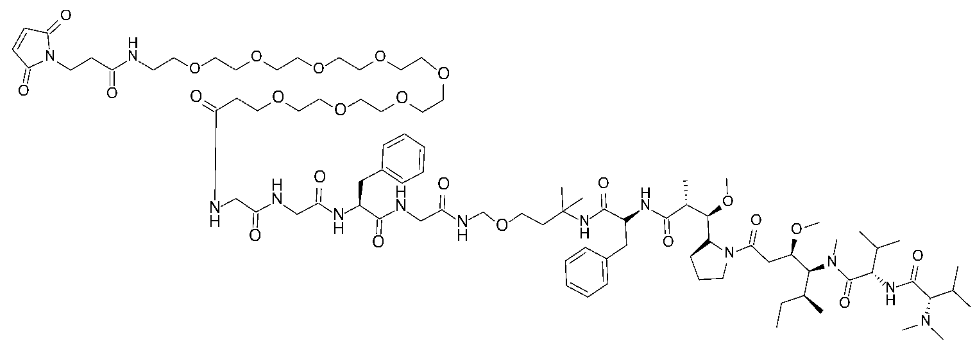

DL-21.

A second objective of the present disclosure is to provide an antibody-drug conjugate, including:

i) an antibody or an antigen-binding fragment thereof; and
ii) the compound, or the isotopically labeled compound, the optical isomer, the geometric isomer, the tautomer, or the mixture of isomers thereof, or the pharmaceutically acceptable salt or solvate thereof as described above.

In some embodiments, the antibody-drug conjugate has a structure represented by Formula (IV):

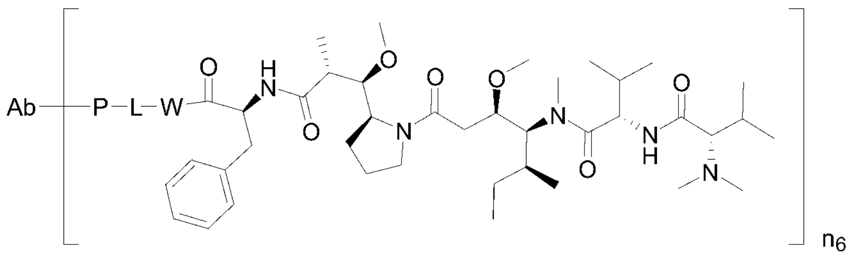

(IV)

where Ab is an antibody or an antigen-binding fragment thereof; and
$n_6$ is selected from an integer or a decimal from 1 to 10.

In some embodiments, $n_6$ is selected from an integer or a decimal from 1 to 8, preferably, $n_6$ is selected from an integer or a decimal from 6 to 8.

For example, in a specific embodiment, $n_6$ may be 1, 2, 3, 4, 5, 6, 6.5, 7, 7.5, 8, 9, or 10.

In some embodiments, the antibody targets one or more selected from the group consisting of HER2, HER3, B7H3, B7H4, EGFR, Claudin 18.2, c-MET, Nectin-4, ROR1, GPNMB, CD56, TACSTD2 (TROP2), CEACAM5, folate receptor-α, mesothelin, ENPP3, guanylate cyclase C, SLC44A4, NaPi2b, CD70, mucin 1, STEAP1, connexin 4, 5T4, SLTRK6, SC-16, LIV-1, P-cadherin, PSMA, fibronectin extra domain B, endothelin receptor ETB, tenascin C, collagen IV, VEGFR2, periostin, CD30, CD79b, CD19, CD22, CD138, CD37, CD33, CD74, and CD98.

In some embodiments, the antibody is a monoclonal antibody.

The antibodies according to the present disclosure include murine antibodies, chimeric antibodies, humanized antibodies and fully human antibodies, preferably humanized antibodies and fully human antibodies.

In a specific embodiment, the antibody or the antigen-binding fragment thereof is Trastuzumab, Pertuzumab, Nimotuzumab, Enoblituzumab, Emibetuzumab, Inotuzumab, Pinatuzumab, Brentuximab, Gemtuzumab, Bivatuzumab, Lorvotuzumab, cBR96, Glematumamab, or an antigen-binding fragment thereof.

A third object of the present disclosure is to provide a pharmaceutical composition, including: the compound, or the isotopically labeled compound, the optical isomer, the geometric isomer, the tautomer, or the mixture of isomers thereof, or the pharmaceutically acceptable salt or solvate thereof as described above, or the antibody-drug conjugate as described above; and one or more pharmaceutically acceptable excipients.

In some embodiments, the dosage form of the pharmaceutical composition is selected from a tablet, a capsule, a granule, a powder, a syrup, an inhalation, or an injection.

In some embodiments, the excipient is one or more selected from the group consisting of a carrier, an excipient, and a diluent. For the purpose of improving the administration effect, persons skilled in the art may select and/or combine excipients and adjust their dosages based on common general knowledge (for example, pharmacopoeias).

A fourth object of the present disclosure is to provide the use of the compound, or the isotopically labeled compound, the optical isomer, the geometric isomer, the tautomer, or the mixture of isomers thereof, or the pharmaceutically acceptable salt or solvate thereof as described above, or the antibody-drug conjugate as described above in preparation of a medicament.

In some embodiments, the medicament is used for prevention or treatment of a tumor; preferably, the tumor is a cancer associated with expression of one or more selected from the group consisting of HER2, HER3, B7H3, B7H4, EGFR, Claudin 18.2, c-MET, Nectin-4, ROR1, GPNMB, CD56, TACSTD2 (TROP2), CEACAM5, folate receptor-$\alpha$, mesothelin, ENPP3, guanylate cyclase C, SLC44A4, NaPi2b, CD70, mucin 1, STEAP1, connexin 4, 5T4, SLTRK6, SC-16, LIV-1, P-cadherin, PSMA, fibronectin extra domain B, endothelin receptor ETB, tenascin C, collagen IV, VEGFR2, periostin, CD30, CD79b, CD19, CD22, CD138, CD37, CD33, CD74, and CD98; more preferably, the tumor is one or more selected from the group consisting of breast cancer, ovarian cancer, cervical cancer, uterine cancer, prostate cancer, renal cancer, urethral cancer, bladder cancer, liver cancer, gastric cancer, endometrial cancer, salivary gland cancer, esophageal cancer, melanoma, glioma, neuroblastoma, sarcoma, lung cancer, colon cancer, rectal cancer, colorectal cancer, leukemia, bone cancer, skin cancer, thyroid cancer, pancreatic cancer, and lymphoma.

A fifth object of the present disclosure is to provide a method for preventing or treating a tumor, including: administering an effective amount of the compound, or the isotopically labeled compound, the optical isomer, the geometric isomer, the tautomer, or the mixture of isomers thereof, or the pharmaceutically acceptable salt or solvate thereof as described above, or the antibody-drug conjugate as described above, or the pharmaceutical composition as described above.

In some embodiments, the tumor is a cancer associated with expression of one or more selected from the group consisting of HER2, HER3, B7H3, B7H4, EGFR, Claudin 18.2, c-MET, Nectin-4, ROR1, GPNMB, CD56, TACSTD2 (TROP2), CEACAM5, folate receptor-$\alpha$, mesothelin, ENPP3, guanylate cyclase C, SLC44A4, NaPi2b, CD70, mucin 1, STEAP1, connexin 4, 5T4, SLTRK6, SC-16, LIV-1, P-cadherin, PSMA, fibronectin extra domain B, endothelin receptor ETB, tenascin C, collagen IV, VEGFR2, periostin, CD30, CD79b, CD19, CD22, CD138, CD37, CD33, CD74, and CD98.

More preferably, the tumor is one or more selected from the group consisting of breast cancer, ovarian cancer, cervical cancer, uterine cancer, prostate cancer, renal cancer, urethral cancer, bladder cancer, liver cancer, gastric cancer, endometrial cancer, salivary gland cancer, esophageal cancer, melanoma, glioma, neuroblastoma, sarcoma, lung cancer, colon cancer, rectal cancer, colorectal cancer, leukemia, bone cancer, skin cancer, thyroid cancer, pancreatic cancer, and lymphoma.

A sixth object of the present disclosure is to provide a method for preparing the compound as described above, where reaction raw materials include a compound containing a structure represented by Formula (I).

In some embodiments, the preparation method includes: reacting a compound containing an L structure and the structure represented by Formula (I) with a compound containing a P structure to obtain the compound as described above; or

reacting a compound containing the P structure and the L structure with a compound containing the structure represented by Formula (I) to obtain the compound as described above.

In some embodiments, the method for preparing the compound includes: reacting a compound containing the L structure with a compound containing the structure represented by Formula (I) to obtain a compound containing the L structure and the structure represented by Formula (I), which is then reacted with a compound containing the P structure to obtain the compound as described above; or

reacting a compound containing the P structure with a compound containing the L structure to obtain a compound containing the P structure and the L structure, which is then reacted with a compound containing the structure represented by Formula (I) to obtain the compound as described above.

In some specific embodiments, persons skilled in the art may select specific compounds based on common general knowledge. In some specific embodiments, protecting groups may be added to or removed from positions of reactive groups in the compound as required by the reaction. In some specific embodiments, the reaction reagents and reaction conditions (including solvents, temperature, pressure, and the like) for each step may be optimized in consideration of the reaction effect. The reaction reagents and reaction conditions are not particularly limited herein.

A seventh objective of the present disclosure is to provide a method for preparing the antibody-drug conjugate as described above, including: coupling the following i) with ii):

i) an antibody or an antigen-binding fragment thereof; and
ii) the compound, or the isotopically labeled compound, the optical isomer, the geometric isomer, the tautomer, or the mixture of isomers thereof, or the pharmaceutically acceptable salt or solvate thereof as described above.

In some embodiments, the method for preparing the antibody-drug conjugate includes: reducing an interchain disulfide bond of the antibody or the antigen-binding fragment thereof to a free sulfhydryl group, which is then conjugated to a P-terminus of the compound, or the isotopically labeled compound, the optical isomer, the geometric isomer, the tautomer, or the mixture of isomers thereof, or the pharmaceutically acceptable salt or solvate thereof.

In some specific embodiments, persons skilled in the art may set a dosage ratio of Ab to the compound based on

common general knowledge. In some specific embodiments, the reaction reagents and reaction conditions (including solvents, temperature, pressure, and the like) for each step may be optimized in consideration of the reaction effect. The reaction reagents and reaction conditions are not particularly limited herein.

The embodiments of the present disclosure are described in detail below with reference to examples. It should be understood that these examples are only used to illustrate the present disclosure, but not to limit the scope of the present disclosure. For experimental methods in the following examples where specific conditions are not indicated, reference should be made to the guidance provided in the present disclosure. Alternatively, standard laboratory manuals, conventional conditions known in the art, or manufacturer-recommended procedures may be adopted.

In the following specific examples, measurement parameters related to raw material components may exhibit slight deviations within the weighing accuracy range unless otherwise specified. Parameters involving temperature and time may allow acceptable deviations resulting from instrument testing accuracy or operational precision.

In each ADC preparation example, an analytical detection method for the ADC sample is as follows:

1. Reversed-Phase Chromatography Detection
Preparation of Mobile Phase A: 0.5‰ TFA in acetonitrile solution, and Mobile Phase B: 0.5‰ TFA in aqueous solution. 20 μL of a to-be-tested sample (1 mg/mL) was taken, 1 μL of DTT (10 mM) was added, and a resulting mixture was allowed to react at room temperature for 30 min for reduction. Chromatographic column used: Agilent PLRP-S 1000A 5 μm, 50*2.1 mm; gradient elution was performed at 70°C (Mobile Phase A: 0% - 100%).
2. SEC Detection

An appropriate amount of the product was taken, and a solvent was added to dissolve and dilute the product to prepare a sample containing approximately 1 to 2 mg of the product in each 1 mL. A diol-bonded gel was used as a filler (Waters Xbridge Premier Protein SEC 7.8*300 mm or a chromatographic column of equivalent efficiency); a solution of 20 mM phosphate buffer and 150 mM sodium chloride was used as the mobile phase; a detection wavelength was 280 nm; column temperature was room temperature; a flow rate was 0.8 mL per minute; and an injection volume was 10 μL.

Specific meanings of the abbreviations involved in the following examples are shown in Table 1 below.

Table 1

| (Abbreviation) | Definitions |
|---|---|
| ADC | Antibody-drug conjugate |
| DMF | N,N-dimethylformamide |
| DCM | Dichloromethane |
| DIEA | Diisopropylethylamine |
| HCl | Hydrogen chloride |
| HATU | 2-(7-azabenzotriazol-1-yl)-N,N,N',N'-tetramethyluronium hexafluorophosphate |
| TFA | Trifluoroacetic acid |
| NaCl | Sodium chloride |
| Boc | Tert-butoxycarbonyl |
| DTT | Dithiothreitol |
| NAc | N-acetylcysteine |
| PBS | Phosphate-buffered saline |

**Example 1: Preparation of Compound DL-1**

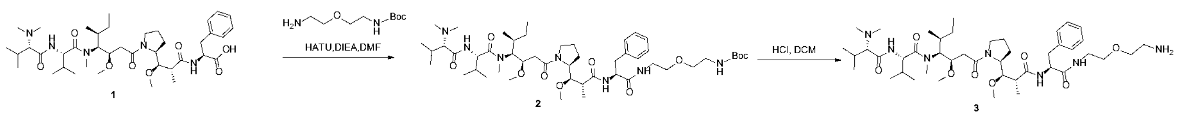

1 2 3

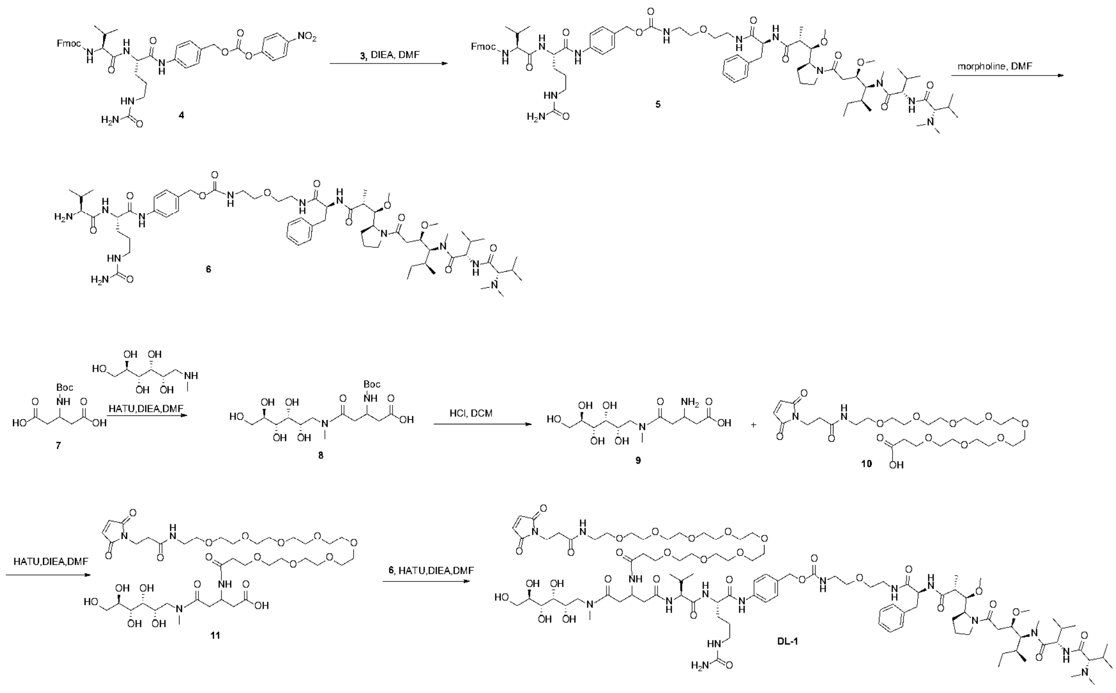


Step 1: Synthesis of tert-butyl ((3R,4R,7S)-7-benzyl-3-((S)-1-((3R,4S,5S)-4-((S)-2-[(S)-2-(dimethylamino)-3-methylbutanamido]-N,3-dimethylbutanamido)-3-methoxy-5-methylheptanoyl) pyrrolidin-2-yl)-4-methyl-5,8-dioxo-2,12-dioxo-6,9-diazatetradecan-14-yl) carbamate

Compound 1 (110 mg, 0.147 mmol) and HATU (62 mg, 0.163 mmol) were added to N,N-dimethylformamide (5 mL), and the mixture was stirred at room temperature for 10 minutes for reaction, then DIEA (70 mg, 0.543 mmol) was added and the mixture was stirred at room temperature for another 10 minutes for reaction. Finally, Compound N-Boc-2-(2-aminoethoxy)ethylamine (30 mg, 0.147 mmol) was added and the mixture was stirred at room temperature for 3 hours for reaction. The reaction was deemed complete when LCMS monitoring showed the content of Compound 1 in the reaction solution was less than 3%. The solvent of the reaction solution was evaporated to dryness under reduced pressure, and the crude product was isolated and purified by preparative liquid chromatography using acetonitrile/water. Compound 2 was obtained (136 mg, 98% yield). MS: 933.26 $[M+H]^+$.

Step 2: Synthesis of (S)-N-((3R,4S,5S)-1-((S)-2-((3R,4R,7S)-14-amino-7-benzyl-4-methyl-5,8-dioxo-2,12-dioxo-6,9-diazatetradecan-3-yl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 2 (136 mg, 0.146 mmol) was added into a 25 mL single-neck flask, dichloromethane (3 mL) and a HCl/1,4-dioxane solution (1 mL, 4M) were added, and the reaction solution reacted at room temperature for 1h. LCMS monitoring showed the complete consumption of starting material Compound 2. After rotary evaporation to dryness, the crude product Compound 3 was obtained (150 mg, 123.6% yield). MS: 833.14$[M+H]^+$.

Step 3: Synthesis of 4-(((S)-2-((S)-2-(((9H-fluoren-9-yl) methoxy)carbonyl) amino)-3-methylbutanamido)-5-ureidopentanamido)benzyl ((3R,4R,7S)-7-benzyl-3-((S)-1-((3R,4S,5S)-4-((S)-2-[(S)-2-(dimethylamino)-3-methylbutanamido]-N,3-dimethylbutanamido)-3-methoxy-5-methylheptanoyl) pyrrolidin-2-yl)-4-methyl-5,8-dioxo-2,12-dioxo-6,9-diazatetradecan-14-yl) carbamate

Compound 3 (100 mg, 0.12 mmol) and Compound 4 (92 mg, 0.12 mmol) were added to N,N-dimethylformamide (5 mL), the mixture was stirred at room temperature for 10 minutes for reaction, then DIEA (62 mg, 0.48 mmol) was added and the mixture was stirred at room temperature for 2h for reaction. The reaction was deemed complete when LCMS monitoring showed the content of Compound 3 in the reaction solution was less than 3%. The solvent of the reaction solution was evaporated to dryness under reduced pressure, and the crude product was isolated and purified by preparative liquid chromatography using acetonitrile/water. Compound 5 (white solid, 182 mg, 103.9% yield) was prepared. MS: 731 $[M+2H]^{2+}$.

Step 4: Synthesis of 4-((S)-2-[(S)-2-amino-3-methylbutanamido)-5-ureidopentanamido]benzyl ((3R,4R,7S)-7-benzyl-3-((S)-1-((3R,4S,5S)-4-((S)-2-[(S)-2-(dimethylamino)-3-methylbutanamido]-N,3-dimethylbutanamido)-3-methoxy-5-methylheptanoyl) pyrrolidin-2-yl)-4-methyl-5,8-dioxo-2,12-dioxo-6,9-diazatetradecan-14-yl) carbamate

Compound 5 (182 mg, 0.125 mmol) was added into a 25 mL single-neck flask, N,N-dimethylformamide (3 mL) and morpholine (2 mL) were added, and the reaction solution reacted at room temperature for 30 min. LCMS monitoring showed the complete consumption of starting material Compound 5. The reaction solution was dried by rotary evaporation, and the crude product was purified by preparative liquid chromatography using acetonitrile/water. Compound 6 (white solid, 130 mg, 84.2% yield) was obtained. MS: 1238.6[M+H]$^+$.

Step 5: Synthesis of 3-((tert-butoxycarbonyl) amino)-5-(methyl((2S,3R,4R,5R)-2,3,4,5,6-pentahydroxyhexyl) amino)-5-oxopentanoic acid

Compound 7 (50 mg, 0.202 mmol) and HATU (76 mg, 0.202 mmol) were added to N,N-dimethylformamide (3 mL), and the mixture was stirred at room temperature for 10 minutes for reaction, then Compound N-methylglucamine (39 mg, 0.202 mmol) was added and the mixture was stirred at room temperature for another 10 minutes for reaction. Finally, DIEA (104 mg, 0.808 mmol) was added and the mixture was stirred at room temperature for 1 hour for reaction. The reaction was deemed complete when LCMS monitoring showed the content of Compound 7 in the reaction solution was less than 3%. The solvent of the reaction solution was evaporated to dryness under reduced pressure, and the crude product was isolated and purified by preparative liquid chromatography using acetonitrile/water. Compound 8 (white solid, 70 mg, 81.5% yield) was prepared. MS: 425.45 [M+H]$^+$.

Step 6: Synthesis of 3-amino-5-(methyl((2S,3R,4R,5R)-2,3,4,5,6-pentahydroxyhexyl) amino)-5-oxopentanoic acid

Compound 8 (30 mg, 0.071 mmol) was added into a 25 mL single-neck flask, dichloromethane (2 mL) and a HCl/1,4-dioxane solution (1 mL, 4M) were added, and the reaction solution reacted at room temperature for 30 min. LCMS monitoring showed the complete consumption of starting material Compound 10. The reaction solution was dried by rotary evaporation, and Compound 9 (white solid, 20 mg, 86.7% yield) was obtained. MS: 325.33[M+H]$^+$.

Step 7: Synthesis of 1-(2,5-dioxo-2,5-dihydro-1H-pyrrol-1-yl)-33-(2-(methyl((2S,3R,4R,5R)-2,3,4,5,6-pentahydroxyhexyl) amino)-2-oxoethyl)-3,31-dioxo-7,10,16,19,22,25,28-octaoxa-4,32-diazapentacont-35-enoic acid

Compound 10 (36 mg, 0.062 mmol) and HATU (28 mg, 0.062 mmol) were added to N,N-dimethylformamide (5 mL), and the mixture was stirred at room temperature for 10 minutes for reaction, then DIEA (32 mg, 0.248 mmol) was added and the mixture was stirred at room temperature for another 10 minutes for reaction. Finally, Compound 9 (20 mg, 0.062 mmol) was added and the mixture was stirred at room temperature for 3 hours for reaction. The reaction was deemed complete when LCMS monitoring showed the content of Compound 10 in the reaction solution was less than 3%. The solvent of the reaction solution was evaporated to dryness under reduced pressure, and the crude product was isolated and purified by preparative liquid chromatography using acetonitrile/water. Compound 11 was obtained (8 mg, 14.3% yield). MS: 899.95 [M+H]$^+$.

Step 8: Synthesis of 4-((2S,5S)-41-(2,5-dioxo-2,5-dihydro-1-dihydro-1-dihydro-1-pyranyl-1-yl)-5-isopropyl-5-isopropyl-9-(2-(methyl-(2S,3R,4R,4R,5R,5R)-2,3,4,4,5,5,6-pentahydroxy-hydroR,4R,7S)-7-benzyl-3-((S)-1-((3R,4S,5S)-4-(S)-2-((dimethylamino)-3-methylbutanamido)-N,3-dimethylbutanamido)-3-methoxy-5-methylheptanoyl) pyrrolidin-2-yl)-4-methyl-5,8-dioxo-2,12-dioxo-6,9-diazatetradecan-14-yl) carbamate

Compound 11 (8 mg, 0.0089 mmol) and HATU (4 mg, 0.011 mmol) were added to N,N-dimethylformamide (3 mL), the mixture was stirred at room temperature for 10 minutes for reaction, then Compound 6 (11 mg, 0.0089 mmol) was added, the mixture was stirred evenly, then DIEA (4.6 mg, 0.036 mmol) was added and the mixture was stirred at room temperature for 3 hours for reaction. The reaction was deemed complete when LCMS monitoring showed the content of Compound 11 in the reaction solution was less than 3%. The solvent of the reaction solution was evaporated to dryness under reduced pressure, and the crude product was isolated and purified by preparative liquid chromatography using acetonitrile/water. Compound DL-1 (white solid, 1.3 mg, 6.9% yield) was prepared after lyophilization. MS: 2119.54 [M+H]$^+$.

$^1$H NMR (400 MHz, DMSO-$d_6$) δ 8.36 - 8.11 (m, 1H), 8.01 (m, 2H), 7.80 (s, 1H), 7.59 (s, 1H), 7.34 - 7.11 (m, 4H), 6.98 (m, 1H), 5.98 (s, 1H), 5.41 (s, 1H), 4.93 (s, 1H), 4.63 (m, 2H), 4.53 (m, 1H), 4.37 (s, 2H), 4.20 (s, 1H), 3.97 (s, 1H), 3.62 - 3.47 (m, 20H), 3.31 (m, 87H), 3.23 - 3.11 (m, 5H), 3.03 (m, 3H), 2.84 - 2.70 (m, 1H), 2.70 - 2.60 (m, 1H), 2.33 (m, 1H), 2.27 (m, 1H), 2.20 (m, 3H), 1.94 (m, 1H), 1.74 (s, 2H), 1.45 (s, 2H), 1.07 - 0.80 (m, 12H), 0.78 - 0.69 (m, 3H).

**Example 2: Preparation of Compound DL-2**

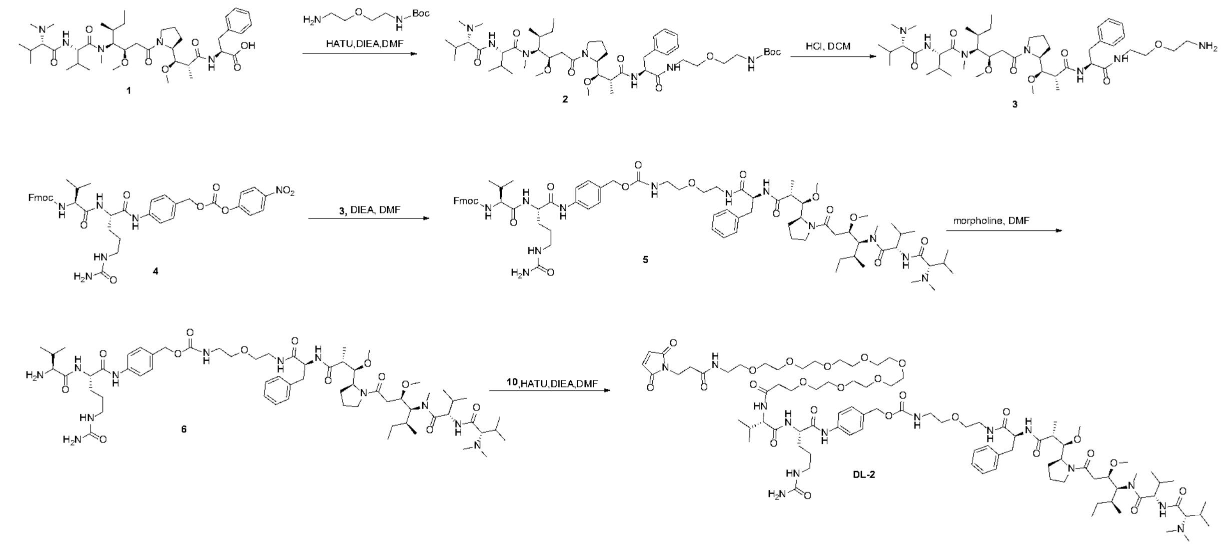


Step 1: Synthesis of tert-butyl ((3R,4R,7S)-7-benzyl-3-((S)-1-(3R,4S,SS)-4-(S)-2-[(S)-2-(dimethylamino)-3-methylbutanamido)-N,3-dimethylbutanamide)-3-methoxy-5-methylheptanoyl) pyrrolidin-2-yl)-4-methyl-5,8-dioxo-2,12-dioxy-6,9-diazatetradecan-14-yl) carbamate

Compound 1 (110 mg, 0.147 mmol) and HATU (62 mg, 0.163 mmol) were added to N,N-dimethylformamide (5 mL), and the mixture was stirred at room temperature for 10 minutes for reaction, then DIEA (70 mg, 0.543 mmol) was added and the mixture was stirred at room temperature for another 10 minutes for reaction. Finally, Compound N-Boc-2-(2-aminoethoxy)ethylamine (30 mg, 0.147 mmol) was added and the mixture was stirred at room temperature for 3 hours for reaction. The reaction was deemed complete when LCMS monitoring showed the content of Compound 1 in the reaction solution was less than 3%. The solvent of the reaction solution was evaporated to dryness under reduced pressure, and the crude product was isolated and purified by preparative liquid chromatography using acetonitrile/water. Compound 2 was obtained (136 mg, 98% yield). MS: 933.26 $[M+H]^+$.

Step 2: Synthesis of (S)-N-((3R,4S,5S)-1-((S)-2-((3R,4R,7S)-14-amino-7-benzyl-4-methyl-5,8-dioxo-2,12-dioxo-6,9-diazatetradecan-3-yl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 2 (136 mg, 0.146 mmol) was added into a 25 mL single-neck flask, dichloromethane (3 mL) and a HCl/1,4-dioxane solution (1 mL, 4M) were added, and the reaction solution reacted at room temperature for 1h. LCMS monitoring showed the complete consumption of starting material Compound 2. After rotary evaporation to dryness, the crude product Compound 3 was obtained (150 mg, 123.6% yield). MS: 833.14$[M+H]^+$.

Step 3: Synthesis of 4-(((S)-2-((S)-2-(((9H-fluoren-9-yl) methoxy)carbonyl) amino)-3-methylbutanamido)-5-ureidopentanamido)benzyl ((3R,4R,7S)-7-benzyl-3-((S)-1-((3R,4S,5S)-4-((S)-2-[(S)-2-(dimethylamino)-3-methylbutanamido]-N,3-dimethylbutanamido)-3-methoxy-5-methylheptanoyl) pyrrolidin-2-yl)-4-methyl-5,8-dioxo-2,12-dioxo-6,9-diazatetradecan-14-yl) carbamate

Compound 3 (100 mg, 0.12 mmol) and Compound 4 (92 mg, 0.12 mmol) were added to N,N-dimethylformamide (5 mL), the mixture was stirred at room temperature for 10 minutes for reaction, then DIEA (62 mg, 0.48 mmol) was added and the mixture was stirred at room temperature for 2h for reaction. The reaction was deemed complete when LCMS monitoring showed the content of Compound 3 in the reaction solution was less than 3%. The solvent of the reaction solution was evaporated to dryness under reduced pressure, and the crude product was isolated and purified by preparative liquid chromatography using acetonitrile/water. Compound 5 (white solid, 182 mg, 103.9% yield) was prepared. MS: 731 $[M+2H]^{2+}$.

Step 4: Synthesis of 4-((S)-2-[(S)-2-amino-3-methylbutanamido)-5-ureidopentanamido]benzyl ((3R,4R,7S)-7-benzyl-3-((S)-1-((3R,4S,5S)-4-((S)-2-[(S)-2-(dimethylamino)-3-methylbutanamido]-N,3-dimethylbutanamido)-3-methoxy-5-methylheptanoyl) pyrrolidin-2-yl)-4-methyl-5,8-dioxo-2,12-dioxo-6,9-diazatetradecan-14-yl) carbamate

Compound 5 (182 mg, 0.125 mmol) was added into a 25 mL single-neck flask, N,N-dimethylformamide (3 mL) and morpholine (2 mL) were added, and the reaction solution reacted at room temperature for 30 min. LCMS monitoring showed the complete consumption of starting material Compound 5. The reaction solution was dried by rotary evaporation, and the crude product was purified by preparative liquid chromatography using acetonitrile/water. Compound 6 (white solid, 130 mg, 84.2% yield) was obtained. MS: 1238.6[M+H]$^+$.

Step 5: Synthesis of 4-((2S,5S)-37-(2,5-dioxo-2,5-dihydro-1H-pyrrol-1-yl)-5-isopropyl-4,7,35-trioxo-2-(3-ureidopropyl)-10,13,16,19,22,25,28,31-octaoxa-3,6,34-triazapentacontanamido)benzyl ((3R,4R,7S)-7-benzyl-3-((S)-1-((3R,4S,5S)-4-((S)-2-[(S)-2-(dimethylamino)-3-methylbutanamido]-N,3-dimethylbutanamido)-3-methoxy-5-methylheptanoyl) pyrrolidin-2-yl)-4-methyl-5,8-dioxo-2,12-dioxo-6,9-diazatetradecan-14-yl) carbamate

Compound 10 (17 mg, 0.028 mmol) and HATU (13 mg, 0.034 mmol) were added to N,N-dimethylformamide (3 mL), the mixture was stirred at room temperature for 10 minutes for reaction, then Compound 6 (35 mg, 0.028 mmol) and DIEA (14 mg, 0.112 mmol) were added and the mixture was stirred at room temperature for 3 hours for reaction. The reaction was deemed complete when LCMS monitoring showed the content of Compound 6 in the reaction solution was less than 3%. The solvent of the reaction solution was evaporated to dryness under reduced pressure, and the crude product was isolated and purified by preparative liquid chromatography using acetonitrile/water. Compound DL-2 (white solid, 5 mg, 9.9% yield) was prepared. MS: 1813.22 [M+H]$^+$.

$^1$H NMR (400 MHz, DMSO-$d_6$) δ 8.12 (m, 1H), 8.08 - 7.97 (m, 3H), 7.93 (m, 1H), 7.86 (m, 1H), 7.58 (m, 2H), 7.29 - 7.16 (m, 7H), 7.00 (s, 2H), 5.99 (m, 1H), 5.41 (s, 2H), 4.93 (s, 2H), 4.72 - 4.56 (m, 2H), 4.56 - 4.47 (m, 1H), 4.38 (m, 1H), 4.29 - 4.18 (m, 1H), 3.97 (s, 1H), 3.78 (m, 1H), 3.61 (m, 4H), 3.53 - 3.46 (m, 26H), 3.37 (m, 8H), 3.25 (s, 1H), 3.24 - 3.10 (m, 12H), 3.07 - 2.88 (m, 7H), 2.76 (m, 1H), 2.69 - 2.59 (m, 1H), 2.46 - 2.25 (m, 6H), 2.20 (m, 6H), 2.04 - 1.84 (m, 5H), 1.81 - 1.54 (m, 5H), 1.35 (m, 6H), 1.05 - 0.80 (m, 22H), 0.77 - 0.66 (m, 6H).

Example 3: Preparation of Compound DL-3

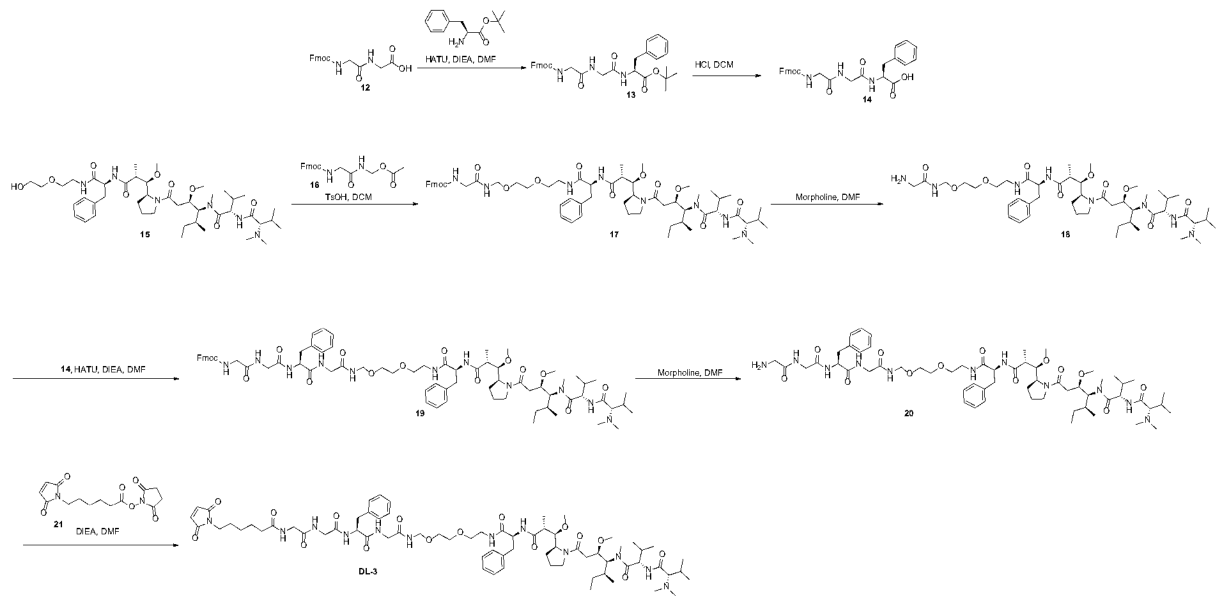


Step 1: Synthesis of tert-butyl ((9H-fluoren-9-yl) methoxycarbonyl)glycylglycyl-L-phenylalaninate

Compound 12 (100.0 mg, 0.282 mmol) and HATU (107.3 mg, 0.282 mmol) were added to N,N-dimethylformamide (3 mL), the mixture was stirred at room temperature for 10 minutes for reaction, then DIEA (140 μL, 0.846 mmol) and tert-Butyl phenylalaninate (62.5 mg, 0.282 mmol) were added and the mixture was stirred at room temperature for 1 hour for reaction. The reaction was deemed complete when LCMS monitoring showed the content of Compound 12 in the

reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound 13 (white solid, 157.0 mg, 99.8% yield) was prepared. MS: 580.17 $[M+Na]^+$.

Step 2: Synthesis of N-[(9H-fluoren-9-yl) methoxy]carbonylglycylglycyl-L-phenylalanine

Compound 13 (157.00 mg, 0.282 mmol) was added to a mixture of DCM (2 mL) and HCl/1,4-dioxane solution (1 mL, 4M), and the reaction solution was allowed to react at room temperature for 60 minutes. The LCMS monitoring showed complete consumption of starting material Compound 13. The reaction solution was subjected to reduced pressure distillation to remove the solvent, followed by isolation and purification via preparative liquid chromatography using acetonitrile/water. Compound 14 (white solid, 124.1 mg, 87.9% yield) was prepared. MS: 502.11 $[M+H]^+$.

Step 3: Synthesis of (9H-fluoren-9-yl) methyl ((3R,4R,7S)-7-benzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8,18-trioxo-2,12,15-trioxo-6,9,17-triazacyclononadecyl) carbamate

Compound 15 (408.0 mg, 0.489 mmol), Compound 16 (90.2 mg, 0.245 mmol) and p-toluenesulfonic acid (126.5 mg, 0.735 mmol) were stirred at room temperature for 10 hours for reaction. The reaction was deemed complete when LCMS monitoring showed the content of Compound 16 in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound 17 (white solid, 162.2 mg, 58.0% yield) was prepared. MS: 1141.59 $[M+H]^+$.

Step 4: Synthesis of (S)-N-((3R,4S,5S)-1-((S)-2-((3R,4R,7S)-19-amino-7-benzyl-4-methyl-5,8,18-trioxo-2,12,15-trioxo-6,9,17-triazacyclononadec-3-yl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 17 (162.2 mg, 0.282 mmol) was added to N,N-dimethylformamide (2 mL) and morpholine (1 mL), and the reaction solution was allowed to react at room temperature for 60 minutes. The LCMS monitoring showed complete consumption of starting material Compound 17. The reaction solution was subjected to reduced pressure distillation to remove the solvent, followed by isolation and purification via preparative liquid chromatography using acetonitrile/water. Compound 18 (white solid, 86.4 mg, 66.2% yield) was prepared. MS: 919.65 $[M+H]^+$.

Step 5: Synthesis of (9H-fluoren-9-yl) methyl ((3R,4R,7S,22S)-7,22-dibenzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8,18,21,24,27-hexaoxo-2,12,15-trioxo-6,9,17,20,23,26-hexaazacyclooctacosan-28-yl) carbamate

Compound 14 (47.1 mg, 0.094 mmol) and HATU (35.7 mg, 0.094 mmol) were added to N,N-dimethylformamide (3 mL), the mixture was stirred at room temperature for 10 minutes for reaction, then DIEA (47 $\mu$L, 0.282 mmol) and Compound 18 (86.4 mg, 0.094 mmol) were added and the mixture was stirred at room temperature for 1 hour for reaction. The reaction was deemed complete when LCMS monitoring showed the content of Compound 14 in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound 19 (white solid, 64.2 mg, 48.7% yield) was prepared. MS: 702.19 $[M+2H]^{2+}$.

Step 6: Synthesis of (S)-N-((3R,4S,5S)-1-((S)-2-((3R,4R,7S,22S)-28-amino-7,22-dibenzyl-4-methyl-5,8,18,21,24,27-hexaoxo-2,12,15-trioxa-6,9,17,20,23,26-hexaazacyclooctacosan-3-yl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 19 (64.2 mg, 0.046 mmol) was added to N,N-dimethylformamide (2 mL) and morpholine (1 mL), and the reaction solution was allowed to react at room temperature for 60 minutes. The LCMS monitoring showed complete consumption of starting material Compound 19. The reaction solution was subjected to reduced pressure distillation to remove the solvent, followed by isolation and purification via preparative liquid chromatography using acetonitrile/water.

Compound 20 (white solid, 22.1 mg, 40.9% yield) was prepared. MS: 1180.73 [M+H]+.

Step 7: Synthesis of N-(3R,4R,7S,22S)-7,22-dibenzyl-3-((S)-1-((3R,4S,5S)-4-((S)-pyrrolidin-2-yl)-4-methyl-5,8,18,21,24,27-hexaoxo-2,12,15-trioxo-6,9,17,20,23,26-hexaazacyclooctacosan-28-yl))-6-(2,5-dioxo-2,5-dihydro-1H-pyrrol-1-yl)hexanamide

Compound 20 (10.0 mg, 0.0085 mmol) was added to N,N-dimethylformamide (2 mL) and DIEA (5 μL, 0.025 mmol), and the reaction solution was allowed to react at room temperature for 2 hours. The reaction was deemed complete when LCMS monitoring showed the content of Compound 20 in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound DL-3 (white solid, 9.2 mg, 79.1% yield) was prepared. MS: 1373.69 [M+H]+.

$^1$H NMR (400 MHz, DMSO-$d_6$) δ 8.48 (m, 1H), 8.18 - 8.10 (m, 1H), 8.07 (m, 1H), 8.01 (m, 2H), 7.95 (m, 2H), 7.31 - 7.10 (m, 10H), 6.99 (s, 2H), 4.61 - 4.39 (m, 5H), 3.86 - 3.64 (m, 7H), 3.59 (m, 2H), 3.54 - 3.43 (m, 6H), 3.26 - 3.09 (m, 11H), 3.09 - 2.92 (m, 6H), 2.78 (m, 3H), 2.69 - 2.57 (m, 2H), 2.39 - 2.23 (m, 3H), 2.20 (m, 6H), 2.11 (m, 2H), 1.89 (s, 7H), 1.47 (m, 5H), 1.30 (s, 1H), 1.20 (m, 3H), 1.00 - 0.79 (m, 16H), 0.79 - 0.63 (m, 6H).

Example 4: Preparation of Compound DL-4

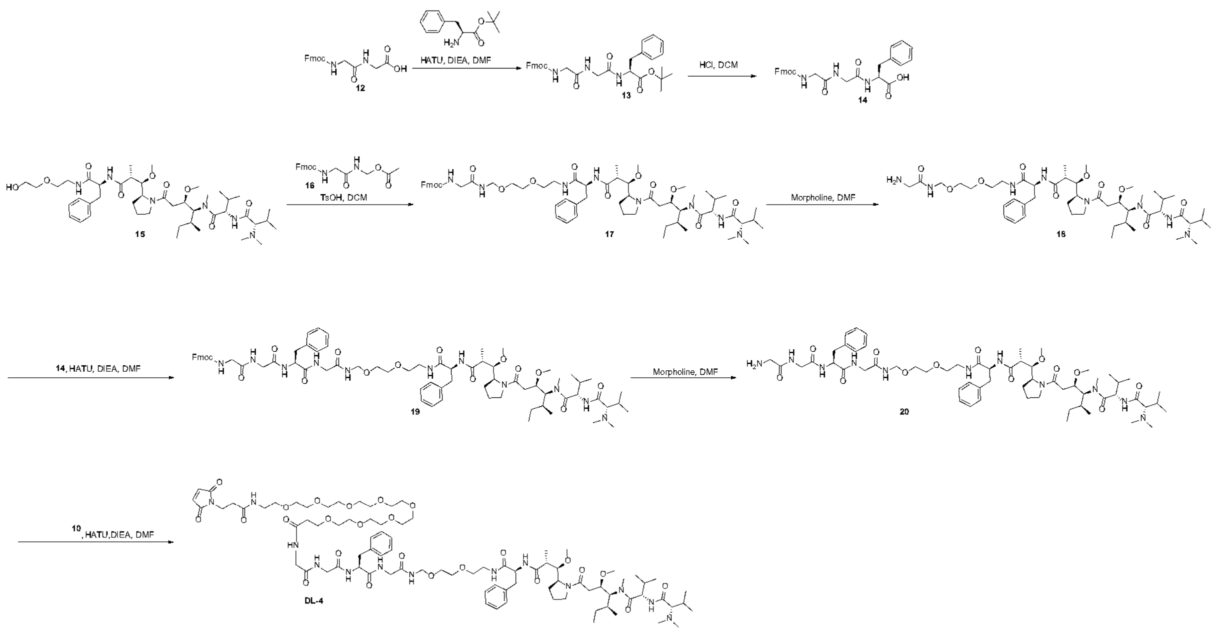


Step 1: Synthesis of tert-butyl ((9H-fluoren-9-yl) methoxycarbonyl)glycylglycyl-L-phenylalaninate

Compound 12 (100.0 mg, 0.282 mmol) and HATU (107.3 mg, 0.282 mmol) were added to N,N-dimethylformamide (3 mL), the mixture was stirred at room temperature for 10 minutes for reaction, then DIEA (140 μL, 0.846 mmol) and tert-Butyl phenylalaninate (62.5 mg, 0.282 mmol) were added and the mixture was stirred at room temperature for 1 hour for reaction. The reaction was deemed complete when LCMS monitoring showed the content of Compound 12 in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound 13 (white solid, 157.0 mg, 99.8% yield) was prepared. MS: 580.17 [M+Na]+.

Step 2: Synthesis of N-[(9H-fluoren-9-yl) methoxy]carbonylglycylglycyl-L-phenylalanine

Compound 13 (157.00 mg, 0.282 mmol) was added to a mixture of DCM (2 mL) and HCl/1,4-dioxane solution (1

mL, 4M), and the reaction solution was allowed to react at room temperature for 60 minutes. The LCMS monitoring showed complete consumption of starting material Compound 13. The reaction solution was subjected to reduced pressure distillation to remove the solvent, followed by isolation and purification via preparative liquid chromatography using acetonitrile/water. Compound 14 (white solid, 124.1 mg, 87.9% yield) was prepared. MS: 502.11 $[M+H]^+$.

Step 3: Synthesis of (9H-fluoren-9-yl) methyl ((3R,4R,7S)-7-benzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8,18-trioxo-2,12,15-trioxo-6,9,17-triazacyclononadecyl) carbamate

Compound 15 (408.0 mg, 0.489 mmol), Compound 16 (90.2 mg, 0.245 mmol) and p-toluenesulfonic acid (126.5 mg, 0.735 mmol) were stirred at room temperature for 10 hours for reaction. The reaction was deemed complete when LCMS monitoring showed the content of Compound 16 in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound 17 (white solid, 162.2 mg, 58.0% yield) was prepared. MS: 1141.59 $[M+H]^+$.

Step 4: Synthesis of (S)-N-((3R,4S,5S)-1-((S)-2-((3R,4R,7S)-19-amino-7-benzyl-4-methyl-5,8,18-trioxo-2,12,15-trioxo-6,9,17-triazacyclononadec-3-yl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 17 (162.2 mg, 0.282 mmol) was added to N,N-dimethylformamide (2 mL) and morpholine (1 mL), and the reaction solution was allowed to react at room temperature for 60 minutes. The LCMS monitoring showed complete consumption of starting material Compound 17. The reaction solution was subjected to reduced pressure distillation to remove the solvent, followed by isolation and purification via preparative liquid chromatography using acetonitrile/water. Compound 18 (white solid, 86.4 mg, 66.2% yield) was prepared. MS: 919.65 $[M+H]^+$.

Step 5: Synthesis of (9H-fluoren-9-yl) methyl ((3R,4R,7S,22S)-7,22-dibenzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8,18,21,24,27-hexaoxo-2,12,15-trioxo-6,9,17,20,23,26-hexaazacyclooctacosan-28-yl) carbamate

Compound 14 (47.1 mg, 0.094 mmol) and HATU (35.7 mg, 0.094 mmol) were added to N,N-dimethylformamide (3 mL), the mixture was stirred at room temperature for 10 minutes for reaction, then DIEA (47 μL, 0.282 mmol) and Compound 18 (86.4 mg, 0.094 mmol) were added and the mixture was stirred at room temperature for 1 hour for reaction. The reaction was deemed complete when LCMS monitoring showed the content of Compound 14 in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound 19 (white solid, 64.2 mg, 48.7% yield) was prepared. MS: 702.19 $[M+2H]^{2+}$.

Step 6: Synthesis of (S)-N-((3R,4S,5S)-1-((S)-2-((3R,4R,7S,22S)-28-amino-7,22-dibenzyl-4-methyl-5,8,18,21,24,27-hexaoxo-2,12,15-trioxa-6,9,17,20,23,26-hexaazacyclooctacosan-3-yl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 19 (64.2 mg, 0.046 mmol) was added to N,N-dimethylformamide (2 mL) and morpholine (1 mL), and the reaction solution was allowed to react at room temperature for 60 minutes. The LCMS monitoring showed complete consumption of starting material Compound 19. The reaction solution was subjected to reduced pressure distillation to remove the solvent, followed by isolation and purification via preparative liquid chromatography using acetonitrile/water. Compound 20 (white solid, 22.1 mg, 40.9% yield) was prepared. MS: 1180.73 $[M+H]^+$.

Step 7: Synthesis of N-(3R,4R,7S,22S)-7,22-dibenzyl-3-((S)-1-((3R,4S,55)-4-((S)-methylheptanoyl) pyrrolidin-2-yl)-4-methyl-5,8,18,21,24,27-hexaoxo-2,12,15-trioxo-6,9,17,20,23,26-hexaazacycloheptacosan-28-yl)-1-(3-(2,5-dioxo-2,5-dihydro-1H-pyrrol-1-yl) propanamido)-3,6,9,12,15,18,21,124-octaoxaheptacosan-27-amide

Compound 10 (5.1 mg, 0.0085 mmol) and HATU (3.5 mg, 0.0085 mmol) were added to N,N-dimethylformamide (3 mL), the mixture was stirred at room temperature for 10 minutes for reaction, then DIEA (5 μL, 0.025 mmol) and Compound 20 (10.0 mg, 0.094 mmol) were added and the mixture was stirred at room temperature for 1 hour for reaction.

The reaction was deemed complete when LCMS monitoring showed the content of Compound 10 in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound 12 (white solid, 6.4 mg, 43.1% yield) was prepared. MS: 1755.12 [M+H]$^+$.

$^1$H NMR (400 MHz, DMSO-$d_6$) δ 8.35 - 8.22 (m, 2H), 8.19 - 8.08 (m, 2H), 8.07 - 7.90 (m, 4H), 7.32 - 7.09 (m, 10H), 7.00 (s, 2H), 4.53 (m, 5H), 3.84 - 3.64 (m, 7H), 3.60 (m, 6H), 3.56 - 3.45 (m, 31H), 3.25 (m, 2H), 3.24 - 3.18 (m, 6H), 3.18 - 3.10 (m, 6H), 3.07 (m, 2H), 3.05 - 2.94 (m, 5H), 2.78 (m, 3H), 2.43 - 2.24 (m, 6H), 2.20 (m, 7H), 2.01 (s, 3H), 1.30 (s, 4H), 1.04 (m, 1H), 0.90 (m, 15H), 0.79 - 0.62 (m, 6H).

**Example 5: Preparation of Compound DL-5**

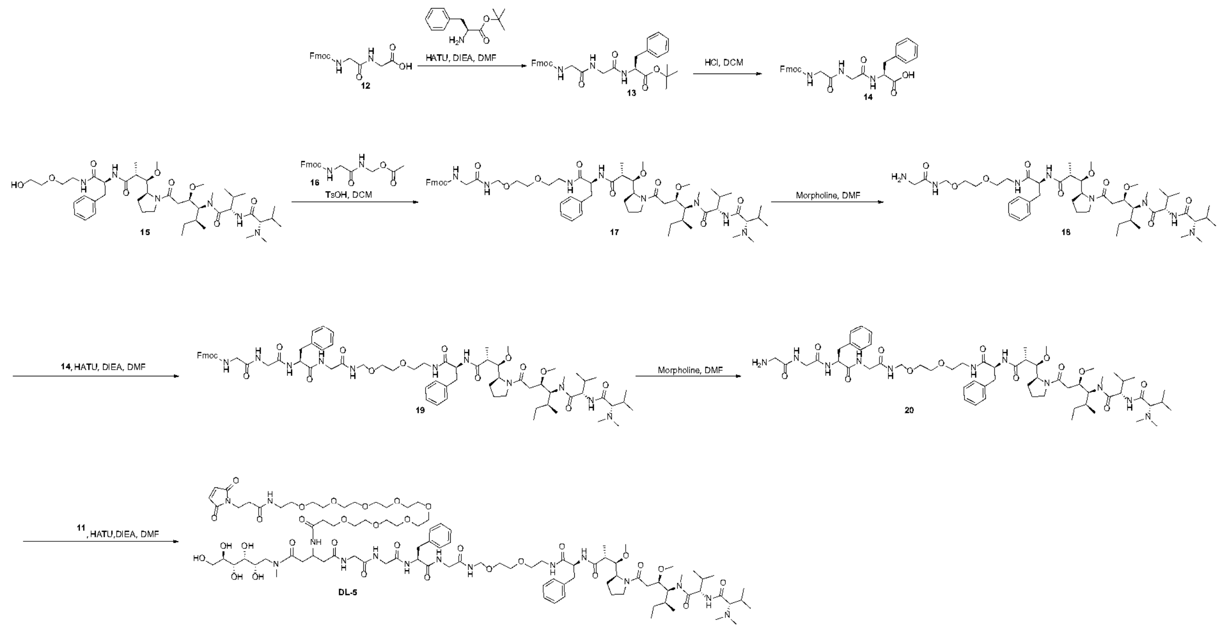


Step 1: Synthesis of tert-butyl ((9H-fluoren-9-yl) methoxycarbonyl)glycylglycyl-L-phenylalaninate

Compound 12 (100.0 mg, 0.282 mmol) and HATU (107.3 mg, 0.282 mmol) were added to N,N-dimethylformamide (3 mL), the mixture was stirred at room temperature for 10 minutes for reaction, then DIEA (140 μL, 0.846 mmol) and tert-Butyl phenylalaninate (62.5 mg, 0.282 mmol) were added and the mixture was stirred at room temperature for 1 hour for reaction. The reaction was deemed complete when LCMS monitoring showed the content of Compound 12 in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound 13 (white solid, 157.0 mg, 99.8% yield) was prepared. MS: 580.17 [M+Na]$^+$.

Step 2: Synthesis of N-[(9H-fluoren-9-yl) methoxy]carbonylglycylglycyl-L-phenylalanine

Compound 13 (157.00 mg, 0.282 mmol) was added to a mixture of DCM (2 mL) and HCl/1,4-dioxane solution (1 mL, 4M), and the reaction solution was allowed to react at room temperature for 60 minutes. The LCMS monitoring showed complete consumption of starting material Compound 13. The reaction solution was subjected to reduced pressure distillation to remove the solvent, followed by isolation and purification via preparative liquid chromatography using acetonitrile/water. Compound 14 (white solid, 124.1 mg, 87.9% yield) was prepared. MS: 502.11 [M+H]$^+$.

Step 3: Synthesis of (9H-fluoren-9-yl) methyl ((3R,4R,7S)-7-benzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8,18-trioxo-2,12,15-trioxo-6,9,17-triazacyclononadecyl) carbamate

Compound 15 (408.0 mg, 0.489 mmol), Compound 16 (90.2 mg, 0.245 mmol) and p-toluenesulfonic acid (126.5 mg, 0.735 mmol) were stirred at room temperature for 10 hours for reaction. The reaction was deemed complete when LCMS monitoring showed the content of Compound 16 in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound 17 (white solid, 162.2 mg, 58.0% yield) was prepared. MS: 1141.59 $[M+H]^+$.

Step 4: Synthesis of (S)-N-((3R,4S,5S)-1-((S)-2-((3R,4R,7S)-19-amino-7-benzyl-4-methyl-5,8,18-trioxo-2,12,15-trioxo-6,9,17-triazacyclononadec-3-yl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 17 (162.2 mg, 0.282 mmol) was added to N,N-dimethylformamide (2 mL) and morpholine (1 mL), and the reaction solution was allowed to react at room temperature for 60 minutes. The LCMS monitoring showed complete consumption of starting material Compound 17. The reaction solution was subjected to reduced pressure distillation to remove the solvent, followed by isolation and purification via preparative liquid chromatography using acetonitrile/water. Compound 18 (white solid, 86.4 mg, 66.2% yield) was prepared. MS: 919.65 $[M+H]^+$.

Step 5: Synthesis of (9H-fluoren-9-yl) methyl ((3R,4R,7S,22S)-7,22-dibenzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8,18,21,24,27-hexaoxo-2,12,15-trioxo-6,9,17,20,23,26-hexaazacyclooctacosan-28-yl) carbamate

Compound 14 (47.1 mg, 0.094 mmol) and HATU (35.7 mg, 0.094 mmol) were added to N,N-dimethylformamide (3 mL), the mixture was stirred at room temperature for 10 minutes for reaction, then DIEA (47 μL, 0.282 mmol) and Compound 18 (86.4 mg, 0.094 mmol) were added and the mixture was stirred at room temperature for 1 hour for reaction. The reaction was deemed complete when LCMS monitoring showed the content of Compound 14 in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound 19 (white solid, 64.2 mg, 48.7% yield) was prepared. MS: 702.19 $[M+2H]^{2+}$.

Step 6: Synthesis of (S)-N-((3R,4S,5S)-1-((S)-2-((3R,4R,7S,22S)-28-amino-7,22-dibenzyl-4-methyl-5,8,18,21,24,27-hexaoxo-2,12,15-trioxa-6,9,17,20,23,26-hexaazacyclooctacosan-3-yl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 19 (64.2 mg, 0.046 mmol) was added to N,N-dimethylformamide (2 mL) and morpholine (1 mL), and the reaction solution was allowed to react at room temperature for 60 minutes. The LCMS monitoring showed complete consumption of starting material Compound 19. The reaction solution was subjected to reduced pressure distillation to remove the solvent, followed by isolation and purification via preparative liquid chromatography using acetonitrile/water. Compound 20 (white solid, 22.1 mg, 40.9% yield) was prepared. MS: 1180.73 $[M+H]^+$.

Step 7: Synthesis of N-(3R,4R,7S,22S)-7,22-dibenzyl-3-((S)-1-((3R,4S,5S)-4-((S)-methylheptanoyl) pyrrolidin-2-yl)-4-methyl-5,8,18,21,24,27-hexaoxo-2,12,15-trioxa-6,9,17,20,23,26-hexaazacyclooctacosan-28-yl)-3-(1-(2,5-dioxo-2,5-dihydro-1H-pyrrol-1-yl)-3-oxo-7,10,13,16,19,25,28-octaoxa-4-azatritriacontan-31-ylamino)-N5-methyl-N5-((2S,3R,4R,5R)-2,3,4,5,6-pentahydroxyhexyl)glutaramide

Compound 11 (4.2 mg, 0.0085 mmol) and HATU (4 mg, 0.011 mmol) were added to N,N-dimethylformamide (3 mL), the mixture was stirred at room temperature for 10 minutes for reaction, then Compound 20 (5.0 mg, 0.0085 mmol) was added, the mixture was stirred evenly, then DIEA (4.6 mg, 0.036 mmol) was added and the mixture was stirred at room temperature for 3 hours for reaction. The reaction was deemed complete when LCMS monitoring showed the content of Compound 11 in the reaction solution was less than 3%. The solvent of the reaction solution was evaporated to dryness under reduced pressure, and the crude product was isolated and purified by preparative liquid chromatography using acetonitrile/water. Compound DL-5 (white solid, 1.5 mg, 8.5% yield) was prepared after lyophilization. MS: 1042.84

$[M+2H]^{2+}$.

$^1$H NMR (400 MHz, DMSO-$d_6$) δ 8.53 - 8.43 (m, 1H), 8.19 - 7.89 (m, 7H), 7.30 - 7.07 (m, 10H), 6.99 (d, $J$ = 1.5 Hz, 2H), 4.59 (s, 3H), 4.52 (m, 6H), 4.37 (s, 3H), 3.96 (s, 2H), 3.88 - 3.65 (m, 8H), 3.64 - 3.54 (m, 7H), 3.54 - 3.43 (m, 31H), 3.26 - 3.19 (m, 10H), 3.18 - 3.12 (m, 6H), 3.09 - 2.93 (m, 8H), 2.87 - 2.70 (m, 5H), 2.69 - 2.60 (m, 2H), 2.30 (m, 9H), 2.24 - 2.11 (m, 7H), 1.09 - 0.95 (m, 2H), 0.94 - 0.80 (m, 14H), 0.72 (m, 6H).

**Example 6: Preparation of Compound DL-6**

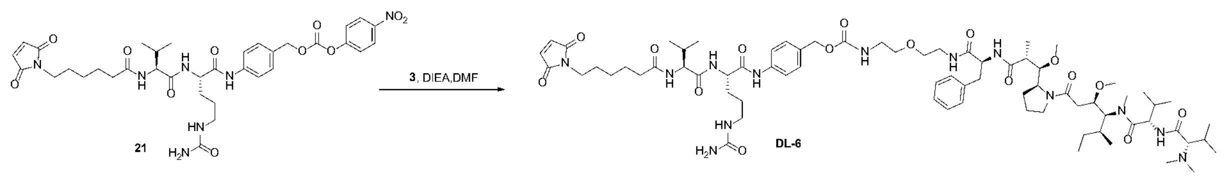

21 DL-6

Step 1: Synthesis of 4-((S)-2-((S)-2-(6-(2,5-dioxo-2,5-dihydro-1H-pyrrol-1-yl)hexanamido)-3-methylbutanamido)-5-ureidopentanamido)benzyl ((3R,4R,7S)-7-benzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8-dioxo-2,12-dioxa-6,9-diazatetradecan-14-yl) carbamate

Compound 21 (4.4 mg, 0.006 mmol) and Compound 3 (5 mg, 0.006 mmol) were added to N,N-dimethylformamide (3 mL), then DIEA (3 mg, 0.024 mmol) was added and the mixture was stirred at room temperature for 1h for reaction. The reaction was deemed complete when LCMS monitoring showed the content of Compound 2 in the reaction solution was less than 3%. The solvent of the reaction solution was evaporated to dryness under reduced pressure, and the crude product was isolated and purified by preparative liquid chromatography using acetonitrile/water. Compound DL-6 (white solid, 6 mg, 69.7% yield) was prepared after lyophilization. MS: 1431.8 $[M+H]^+$.

$^1$H NMR (400 MHz, DMSO-$d_6$) δ 9.97 (s, 1H), 8.10 - 7.98 (m, 3H), 7.94 (m, 1H), 7.79 (m, 1H), 7.57 (m, 2H), 7.23 (m, 6H), 7.14 (m, 1H), 7.00 (s, 2H), 5.97 (m, 1H), 5.37 (m, 2H), 4.93 (s, 2H), 4.76 - 4.57 (m, 2H), 4.55 - 4.46 (m, 1H), 4.38 (m, 1H), 4.23 - 4.14 (m, 1H), 3.96 (s, 1H), 3.78 (m, 1H), 3.50 (m, 1H), 3.37 (m, 7H), 3.23 - 3.15 (m, 7H), 3.08 - 2.89 (m, 7H), 2.75 (m, 1H), 2.68 - 2.60 (m, 1H), 2.45 - 2.35 (m, 1H), 2.34 - 2.24 (m, 2H), 2.23 - 2.06 (m, 9H), 2.03 - 1.84 (m, 4H), 1.82 - 1.63 (m, 4H), 1.53 - 1.35 (m, 7H), 1.30 - 1.13 (m, 4H), 1.03 (m, 1H), 1.00 - 0.79 (m, 20H), 0.78 - 0.65 (m, 6H).

**Example 7: Preparation of Compound DL-7**

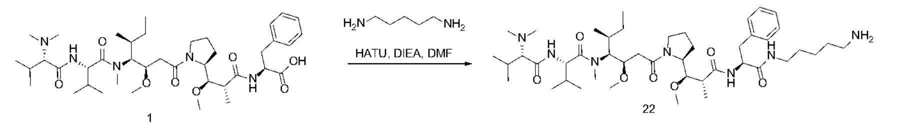

1 22

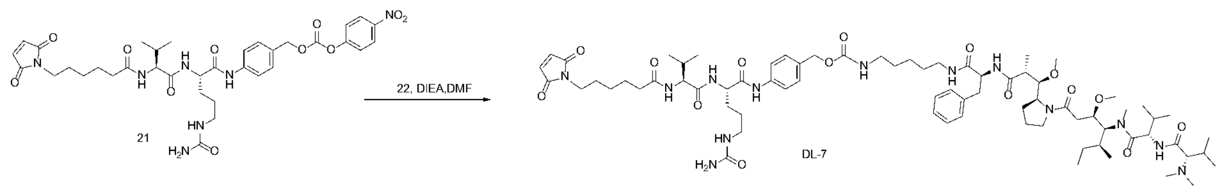

21 DL-7

Step 1: Synthesis of (S)-N-((3R,4S,5S)-1-((S)-2-(((1R,2R)-3-((S)-1-((5-aminopentyl) amino)-1-oxo-3-phenylpropan-2-yl) amino)-1-methoxy-2-methyl-3-oxopropyl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 1 (50 mg, 0.067 mmol) and HATU (31 mg, 0.08 mmol) were added to N,N-dimethylformamide (3 mL), and the mixture was stirred at room temperature for 10 minutes for reaction, then 1,5-pentanediamine (14 mg, 0.137 mmol) was added and the mixture was stirred at room temperature for another 10 minutes for reaction. Finally, DIEA (35 mg,

0.268 mmol) was added and the mixture was stirred at room temperature for 3 hours for reaction. The reaction was deemed complete when LCMS monitoring showed the content of Compound 1 in the reaction solution was less than 3%. The solvent of the reaction solution was evaporated to dryness under reduced pressure, and the crude product was isolated and purified by preparative liquid chromatography using acetonitrile/water. Compound 22 (white solid, 40 mg, 71.9% yield) was prepared. MS: 831.17 $[M+H]^+$.

Step 2: Synthesis of 4-((S)-2-((S)-2-(6-(2,5-dioxo-2,5-dihydro-1H-pyrrol-1-yl)hexanamido)-3-methylbutanamido)-5-ureidopentanamido)benzyl (5-((S)-2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-3-ethoxy-2-methylpropanamido)-3-phenylpropanamido) pentyl) carbamate

Compound 21 (10 mg, 0.012 mmol) and Compound 22 (9 mg, 0.012 mmol) were added to N,N-dimethylformamide (3 mL), then DIEA (6 mg, 0.048 mmol) was added and the mixture was stirred at room temperature for 1h for reaction. The reaction was deemed complete when LCMS monitoring showed the content of Compound 21 in the reaction solution was less than 3%. The solvent of the reaction solution was evaporated to dryness under reduced pressure, and the crude product was isolated and purified by preparative liquid chromatography using acetonitrile/water. Compound DL-7 (white solid, 13 mg, 76.5% yield) was prepared after lyophilization. MS: 1429.83 $[M+H]^+$.

$^1$H NMR (400 MHz, DMSO-$d_6$) δ 9.96 (s, 1H), 8.31 - 8.21 (m, 1H), 8.10 - 7.97 (m, 3H), 7.86 (m, 1H), 7.79 (m, 1H), 7.58 (m, 2H), 7.30 - 7.18 (m, 5H), 7.15 (m, 2H), 7.00 (s, 2H), 5.96 (m, 1H), 5.40 (s, 2H), 4.92 (s, 2H), 4.61 (m, 2H), 4.55 - 4.42 (m, 2H), 4.38 (m, 1H), 4.23 - 4.15 (m, 1H), 3.97 (s, 1H), 3.79 (m, 1H), 3.52 (m, 2H), 3.36 (m, 3H), 3.24 (m, 3H), 3.17 (m, 3H), 3.10 - 2.89 (m, 9H), 2.81 - 2.71 (m, 1H), 2.68 - 2.60 (m, 1H), 2.33 (m, 4H), 2.23 - 2.07 (m, 8H), 1.95 (m, 4H), 1.82 - 1.65 (m, 4H), 1.58 (m, 1H), 1.49 (m, 5H), 1.36 (m, 5H), 1.26 - 1.12 (m, 5H), 1.04 (m, 1H), 0.97 - 0.79 (m, 17H), 0.73 (m, 6H).

**Example 8: Preparation of Compound DL-8**

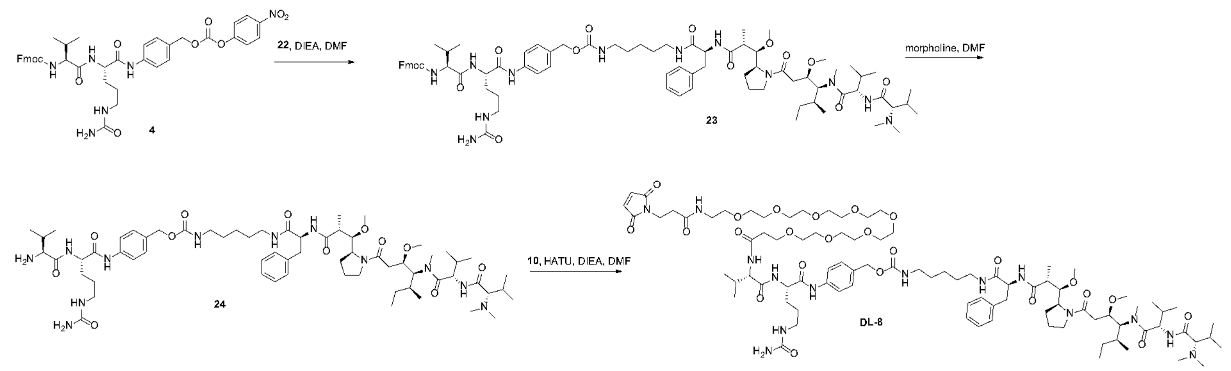


Step 1: Synthesis of 4-(((S)-2-((S)-2-(((9H-fluoren-9-yl) methoxy)carbonyl) amino)-3-methylbutanamido)-5-ureidopentanamido)benzyl (5-((S)-2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-3-methoxy-2-methylpropanamido)-3-phenylpropanamido) pentyl) carbamate

Compound 22 (30 mg, 0.036 mmol) and Compound 4 (28 mg, 0.036 mmol) were added to N,N-dimethylformamide (5 mL), the mixture was stirred at room temperature for 10 minutes, then DIEA (18 mg, 0.144 mmol) was added and the mixture was stirred at room temperature for 2h for reaction. The reaction was deemed complete when LCMS monitoring showed the content of Compound 22 in the reaction solution was less than 3%. The solvent of the reaction solution was evaporated to dryness under reduced pressure, and the crude product was isolated and purified by preparative liquid chromatography using acetonitrile/water. Compound 23 (white solid, 51 mg, 97.2% yield) was prepared. MS: 729.94 $[M+2H]^{2+}$.

Step 2: Preparation of 4-((S)-2-((S)-2-amino-3-methylbutanamido)-5-ureidopentanamido)benzyl (5-((S)-2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-3-methoxy-2-methylpropanamido)-3-phenylpropanamido) pentyl) carbamate

Compound 23 (51 mg, 0.035 mmol) was added into a 25 mL single-neck flask, N,N-dimethylformamide (5 mL) and morpholine (2 mL) were added, and the reaction solution reacted at room temperature for 30 min. LCMS monitoring showed the complete consumption of starting material Compound 23. The reaction solution was dried by rotary evaporation, and the crude product was purified by preparative liquid chromatography using acetonitrile/water. Compound 24 (white solid, 13 mg, 30.2% yield) was obtained. MS: 618.81$[M+2H]^{2+}$.

Step 3: Synthesis of 4-((2S,5S)-37-(2,5-dioxo-2,5-dihydro-1H-pyrrol-1-yl)-5-isopropyl-4,7,35-trioxo-2-(3-ureidopropyl)-10,13,16,19,22,25,28,31-octaoxa-3,6,34-triazatritriacontanamido)benzyl (5-((S)-2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-3-methoxy-2-methylpropanamido)-3-phenylpropanamido) pentyl) carbamate

Compound 10 (6 mg, 0.01 mmol) and HATU (5 mg, 0.012 mmol) were added to N,N-dimethylformamide (3 mL), the mixture was stirred at room temperature for 10 minutes for reaction, then Compound 24 (13 mg, 0.01 mmol) and DIEA (5 mg, 0.04 mmol) were added and the mixture was stirred at room temperature for 3 hours for reaction. The reaction was deemed complete when LCMS monitoring showed the content of Compound 24 in the reaction solution was less than 3%. The solvent of the reaction solution was evaporated to dryness under reduced pressure, and the crude product was isolated and purified by preparative liquid chromatography using acetonitrile/water. Compound DL-8 (white solid, 9 mg, 49.7% yield) was prepared after lyophilization. MS: 1811.25 $[M+H]^+$.

$^1H$ NMR (400 MHz, DMSO-$d_6$) δ 9.96 (s, 1H), 8.27 - 8.11 (m, 1H), 8.12 - 7.95 (m, 3H), 7.83 (m, 1H), 7.58 (m, 2H), 7.26 (m, 3H), 7.24 - 7.19 (m, 3H), 7.18 - 7.12 (m, 1H), 7.00 (m, 1H), 5.96 (m, 1H), 5.36 (m, 2H), 4.92 (s, 2H), 4.74 - 4.56 (m, 1H), 4.53 - 4.41 (m, 1H), 4.43 - 4.32 (m, 1H), 4.23 (m, 1H), 3.97 (s, 1H), 3.79 (m, 1H), 3.67 - 3.55 (m, 4H), 3.56 - 3.41 (m, 27H), 3.36 (m, 3H), 3.24 (m, 4H), 3.15 (m, 5H), 3.08 (s, 1H), 3.06 - 2.88 (m, 9H), 2.81 - 2.72 (m, 1H), 2.70 - 2.62 (m, 1H), 2.45 (m, 1H), 2.40 (m, 1H), 2.36 - 2.28 (m, 3H), 2.26 - 2.12 (m, 7H), 2.04 - 1.84 (m, 4H), 1.83 - 1.65 (m, 3H), 1.56 (m, 1H), 1.41 - 1.26 (m, 7H), 1.22 (m, 2H), 1.21 - 1.12 (m, 2H), 1.02 (m, 1H), 0.89 (m, 21H), 0.74 (m, 6H).

**Example 9: Preparation of Compound DL-9**

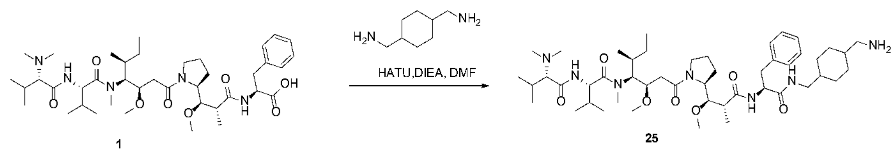


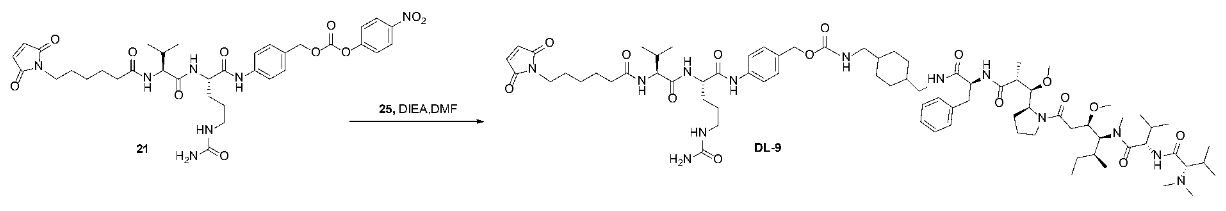


Step 1: Synthesis of ((S)-N-((3R,4S,55)-1-((S)-2-(((1R,2R)-3-((S)-1-((4-(aminomethyl)cyclohexyl) methyl) amino)-1-oxo-3-phenylpropan-2-yl) amino)-1-methoxy-2-methyl-3-oxopropyl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 1 (20 mg, 0.027 mmol) and HATU (13 mg, 0.032 mmol) were added to N,N-dimethylformamide (3 mL), and the mixture was stirred at room temperature for 10 minutes for reaction, then Compound 1,4-cyclohexanedimethylamine (8 mg, 0.054 mmol) was added and the mixture was stirred at room temperature for another 10 minutes for reaction. Finally, DIEA (14 mg, 0.108 mmol) was added and the mixture was stirred at room temperature for 3 hours for reaction. The reaction was deemed complete when LCMS monitoring showed the content of Compound 1 in the reaction

solution was less than 3%. The solvent of the reaction solution was evaporated to dryness under reduced pressure, and the crude product was isolated and purified by preparative liquid chromatography using acetonitrile/water. Compound 25 (white solid, 17 mg, 72.3% yield) was prepared. MS: 871.23 [M+H]+.

Step 2: Synthesis of 4-(S)-2-((S)-2-(6-(2,5-dioxo-2,5-dihydro-1H-pyrrol-1-yl)hexanamido)-3-methylbutanamido)-5-ureidopentanamido)benzyl ((4-(((S)-2-((2R,3R)-3-((S)-1-((3R,4S,SS)-4-((S)-2-((S)-2-(dimethylamino)-3-methylbutanamido)-N,3-dimethylbutanamido)-3-methoxy-5-methylheptanoyl) pyrrolidin-2-yl)-3-methoxy-2-methylpropanamido)-3-phenylpropanamido) methyl)cyclohexyl) methyl) carbamate

Compound 25 (17 mg, 0.02 mmol) and Compound 21 (14 mg, 0.02 mmol) were added to N,N-dimethylformamide (3 mL), then DIEA (10 mg, 0.08 mmol) was added and the mixture was stirred at room temperature for 1h for reaction. The reaction was deemed complete when LCMS monitoring showed the content of Compound 25 in the reaction solution was less than 3%. The solvent of the reaction solution was evaporated to dryness under reduced pressure, and the crude product was isolated and purified by preparative liquid chromatography using acetonitrile/water. Compound DL-9 (white solid, 7.4 mg, 25.2% yield) was prepared after lyophilization. MS: 1469.89 [M+H]+.

$^1$H NMR (400 MHz, DMSO-$d_6$) δ 9.97 (s, 1H), 8.08 (m, 1H), 8.05 - 8.01 (m, 1H), 7.80 (m, 1H), 7.57 (m, 2H), 7.32 - 7.11 (m, 8H), 6.99 (s, 2H), 6.73 (m, 1H), 6.05 - 5.95 (m, 1H), 5.41 (s, 2H), 4.93 (s, 2H), 4.63 (m, 1H), 4.52 (m, 1H), 4.38 (m, 1H), 4.24 - 4.11 (m, 1H), 3.97 (s, 1H), 3.81 (m, 2H), 3.23 (m, 6H), 3.18 (m, 5H), 3.11 - 2.86 (m, 9H), 2.86 - 2.71 (m, 2H), 2.69 - 2.59 (m, 1H), 2.26 - 2.06 (m, 8H), 2.05 - 1.85 (m, 4H), 1.80 - 1.70 (m, 2H), 1.66 (m, 1H), 1.63 - 1.57 (m, 1H), 1.48 (m, 7H), 1.37 - 1.14 (m, 15H), 1.04 (m, 1H), 0.96 (m, 1H), 0.94 - 0.79 (m, 20H), 0.78 - 0.68 (m, 7H).

**Example 10: Preparation of Compound DL-10**

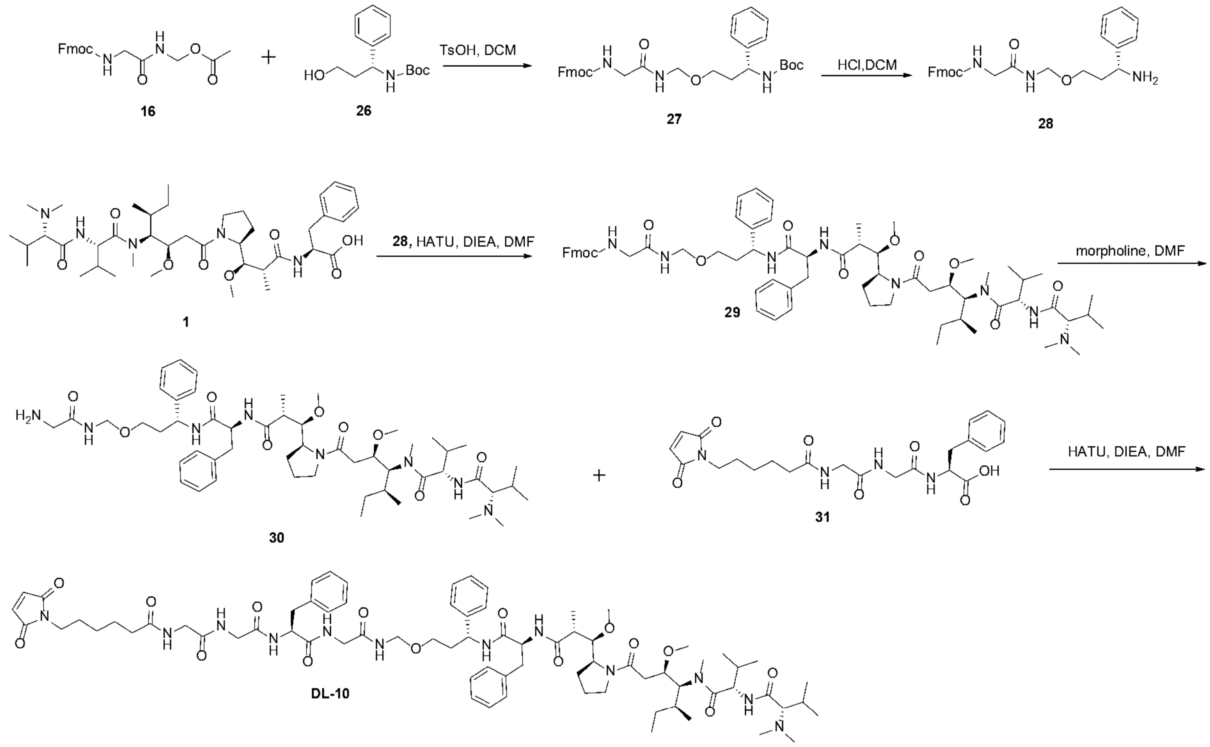


16 26 27 28 1 29 30 31 DL-10

Step 1: Synthesis of (9H-fluoren-9-yl) methyl (R)-(12,12-dimethyl-2,10-dioxo-8-phenyl-5,11-dioxo-3,9-diazatridecyl) carbamate

Compound 16 (100 mg, 0.272 mmol), Compound 26 (200 mg, 0.797 mmol), and TsOH (137 mg, 0.797 mmol) were added to dichloromethane (3 mL), and the mixture was stirred at room temperature for 3 hours for reaction. The reaction was deemed complete when LCMS monitoring showed the content of Compound 26 in the reaction solution was less than 3%. The solvent of the reaction solution was evaporated to dryness under reduced pressure, and the crude product was isolated and purified by preparative liquid chromatography using acetonitrile/water. Compound 27 (white

solid, 71 mg, 46.6% yield) was prepared. MS: 560.66 $[M+H]^+$.

Step 2: Synthesis of (9H-fluoren-9-yl) methyl (R)-(2-(((3-amino-3-phenylpropoxy) methyl) amino)-2-oxoethyl) carbamate

Compound 27 (71 mg, 0.127 mmol) was added into a 25 mL single-neck flask, dichloromethane (3 mL) and a HCl/1,4-dioxane solution (1 mL, 4M) were added, and the reaction solution reacted at room temperature for 1h. LCMS monitoring showed the complete consumption of starting material Compound 27. The crude product was obtained after rotary evaporation, and then isolated and purified by preparative liquid chromatography using acetonitrile/water to obtain Compound 28 (28 mg, 47.9% yield). MS: 460.55$[M+H]^+$.

Step 3: Synthesis of (9H-fluoren-9-yl) methyl ((3R,4R,7S,10R)-7-benzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8,16-trioxo-10-phenyl-2,13-dioxa-6,9,15-triazaheptan-17-yl) carbamate

Compound 1 (45 mg, 0.061 mmol) and HATU (28 mg, 0.073 mmol) were added to N,N-dimethylformamide (5 mL), and the mixture was stirred at room temperature for 10 minutes for reaction, then DIEA (31 mg, 0.244 mmol) was added and the mixture was stirred at room temperature for another 10 minutes for reaction. Finally, Compound 28 (28 mg, 0.061 mmol) was added and the mixture was stirred at room temperature for 3 hours for reaction. The reaction was deemed complete when LCMS monitoring showed the content of Compound 1 in the reaction solution was less than 3%. The solvent of the reaction solution was evaporated to dryness under reduced pressure, and the crude product was isolated and purified by preparative liquid chromatography using acetonitrile/water. Compound 29 was obtained (16 mg, 22.1% yield). MS: 1188.53 $[M+H]^+$.

Step 4: Synthesis of (S)-N-((3R,4S,5S)-1-((S)-2-((3R,4R,7S,10R)-17-amino-7-benzyl-4-methyl-5,8,16-trioxo-10-phenyl-2,13-dioxa-6,9,15-triazaheptan-3-yl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 29 (16 mg, 0.013 mmol) was added into a 25 mL single-neck flask, N,N-dimethylformamide (3 mL) and morpholine (1 mL) were added, and the reaction solution reacted at room temperature for 30 min. LCMS monitoring showed the complete consumption of starting material Compound 29. The reaction solution was dried by rotary evaporation, and the crude product was purified by preparative liquid chromatography using acetonitrile/water. Compound 30 (white solid, 6 mg, 46.2% yield) was obtained. MS: 966.29$[M+H]^+$.

Step 5: Synthesis of N-((3R,4R,7S,10R,20S)-7,20-dibenzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8,16,19,22,25-hexaoxo-10-phenyl-2,13-dioxo-6,9,15,18,21,24-hexaazahexan-26-yl)-6-(2,5-dioxo-2,5-dihydro-1H-pyrrol-1-yl)hexanamide

Compound 31 (3 mg, 0.006 mmol) and HATU (2.7 mg, 0.007 mmol) were added to N,N-dimethylformamide (3 mL), and the mixture was stirred at room temperature for 10 minutes for reaction, then DIEA (3.1 mg, 0.024 mmol) was added and the mixture was stirred at room temperature for another 10 minutes for reaction. Finally, Compound 30 (6 mg, 0.006 mmol) was added and the mixture was stirred at room temperature for 3 hours for reaction. The reaction was deemed complete when LCMS monitoring showed the content of Compound 31 in the reaction solution was less than 3%. The solvent of the reaction solution was evaporated to dryness under reduced pressure, and the crude product was isolated and purified by preparative liquid chromatography using acetonitrile/water. Compound DL-10 (5 mg, 58.7% yield) was obtained after lyophilization. MS: 1420.77 $[M+H]^+$.

$^1H$ NMR (400 MHz, DMSO) $\delta$ 8.16 - 8.03 (m, 2H), 7.97 (m, 1H), 7.74 (m, 1H), 7.68 (m, 1H), 7.27 - 7.14 (m, 15H), 7.00 - 6.95 (m, 2H), 6.64 (s, 1H), 6.51 (s, 1H), 5.32 (m, 1H), 4.52 (m, 3H), 3.74 - 3.63 (m, 5H), 3.25 - 3.13 (m, 9H), 2.98 (m, 5H), 2.84 - 2.69 (m, 8H), 2.32 (m, 3H), 2.10 (m, 2H), 2.00 (m, 7H), 1.85 (s, 4H), 1.47 (m, 10H), 1.30 (m, 5H), 0.98 - 0.81 (m, 21H), 0.76 (m, 2H).

**Example 11: Preparation of Compound DL-11**

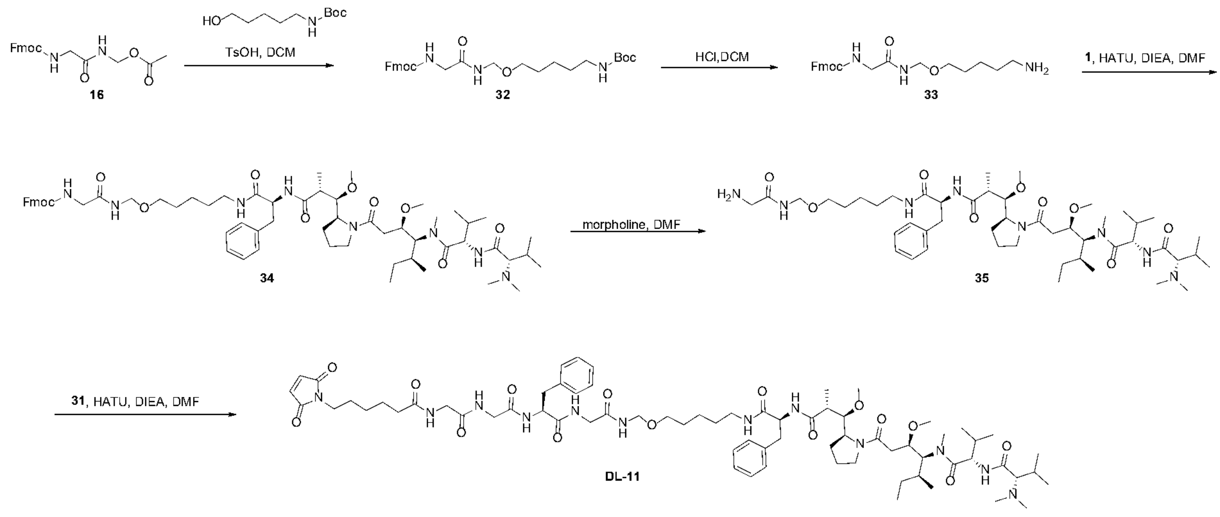


16, 32, 33, 34, 35, DL-11

Step 1: Synthesis of (9H-fluoren-9-yl) methyl (14,14-dimethyl-2,12-dioxo-5,13-dioxo-3,11-diazapentadecyl) carbamate

Compound 16 (60.0 mg, 0.163 mmol), Compound 5-Boc-amino-1-pentanol (99.33 mg, 0.489 mmol) and p-toluenesulfonic acid (84.21 mg, 0.489 mmol) were added to dichloromethane (3 mL), and the mixture was stirred at room temperature for 2 hours for reaction. The reaction was deemed complete when LCMS monitoring showed the content of Compound 16 in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound 32 (white solid, 32.0 mg, 46% yield) was prepared. MS: 534.29 $[M+Na]^+$.

Step 2: Synthesis of (9H-fluoren-9-yl) methyl (2-((((5-aminopentyl)oxy) methyl) amino)-2-oxoethyl) carbamate

Compound 32 (32.00 mg, 0.063 mmol) was added to dichloromethane (2 mL) and HCl (4M dioxane, 1 mL), and the reaction solution was allowed to react at room temperature for 30 minutes. The LCMS monitoring showed complete consumption of starting material Compound 32. The reaction solution was subjected to reduced pressure distillation to remove the solvent, followed by isolation and purification via preparative liquid chromatography using acetonitrile/water. Compound 33 (white solid, 21.0 mg, 81.7% yield) was prepared. MS: 412.25 $[M+H]^+$.

Step 3: Synthesis of (9H-fluoren-9-yl) methyl ((3R,4R,7S)-7-benzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8,18-trioxo-2,15-dioxo-6,9,17-triazanonadecyl) carbamate

Compound 1 (38.1 mg, 0.051 mmol) and HATU (19.4 mg, 0.051 mmol) were added to N,N-dimethylformamide (3 mL), the mixture was stirred at room temperature for 10 minutes for reaction, then DIEA (26 μL, 0.153 mmol) and Compound 33 (21.0 mg, 0.051 mmol) were added and the mixture was stirred at room temperature for 1 hour for reaction. The reaction was deemed complete when LCMS monitoring showed the content of Compound 1 in the reaction solution was less than 3%. The solvent was evaporated to dryness under reduced pressure, and the crude product was isolated and purified by preparative liquid chromatography using acetonitrile/water. Compound 34 (white solid, 22.0 mg, 37.9% yield) was prepared. MS: 1139.99 $[M+H]^+$.

Step 4: Synthesis of (S)-N-((3R,4S,5S)-1-((S)-2-((3R,4R,7S)-19-amino-7-benzyl-4-methyl-5,8,18-trioxo-2,15-dioxa-6,9,17-triazanonadecyl-3-yl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 34 (22.0 mg, 0.019 mmol) was added to N,N-dimethylformamide (2 mL) and morpholine (1 mL), and the reaction solution was allowed to react at room temperature for 30 minutes. The LCMS monitoring showed complete consumption of starting material Compound 34. The reaction solution was subjected to reduced pressure distillation to remove the solvent, followed by isolation and purification via preparative liquid chromatography using acetonitrile/water. Compound 35 (white solid, 12.0 mg, 67.8% yield) was prepared. MS: 917.79 $[M+H]^+$.

Step 5: Synthesis of N-((3R,4R,7S,22S)-7,22-dibenzyl-3-(S)-1-((3R,4S,5S)-4-(S) pyrrolidin-2-yl)-4-methyl-5,8,18,21,24,27-hexaoxo-2,15-dioxo-6,9,17,20,23,26-hexaazaoctacosan-28-yl)-6-(2,5-dioxo-2,5-dihydro-1H-pyrrol-1-yl)hexanamide

Compound 31 (6.2 mg, 0.013 mmol) and HATU (5.0 mg, 0.013 mmol) were added to N,N-dimethylformamide (2 mL), the mixture was stirred at room temperature for 10 minutes for reaction, then DIEA (7 μL, 0.039 mmol) and Compound 35 (12.0 mg, 0.013 mmol) were added and the mixture was stirred at room temperature for 1 hour for reaction. The reaction was deemed complete when LCMS monitoring showed the content of Compound 31 in the reaction solution was less than 3%. The solvent of the reaction solution was evaporated to dryness under reduced pressure, and the crude product was isolated and purified by preparative liquid chromatography using acetonitrile/water. Compound DL-11 (white solid, 2.5 mg, 14.0% yield) was prepared. MS: 1371.70 $[M+H]^+$.

$^1H$ NMR (400 MHz, DMSO-$d_6$) δ 8.43 (s, 3H), 8.27 (d, *J* = 7.3 Hz, 2H), 8.18 - 7.97 (m, 5H), 7.95 (s, 1H), 7.30 - 7.14 (m, 10H), 6.99 (s, 2H), 6.75 (s, 2H), 4.63 (m, 3H), 4.50 (m, 5H), 3.96 (s, 2H), 3.85 - 3.60 (m, 9H), 3.56 (s, 3H), 3.22 (m, 4H), 3.20 - 3.14 (m, 4H), 3.11 - 2.93 (m, 9H), 2.89 (s, 2H), 2.80 (m, 3H), 2.73 (s, 2H), 2.64 (m, 2H), 2.20 (m, 7H), 2.11 (m, 3H), 1.92 (m, 4H), 1.76 (m, 5H), 1.60 - 1.31 (m, 16H), 1.30 - 1.11 (m, 9H), 1.00 - 0.80 (m, 16H), 0.80 - 0.64 (m, 7H).

**Example 12: Preparation of Compound DL-12**

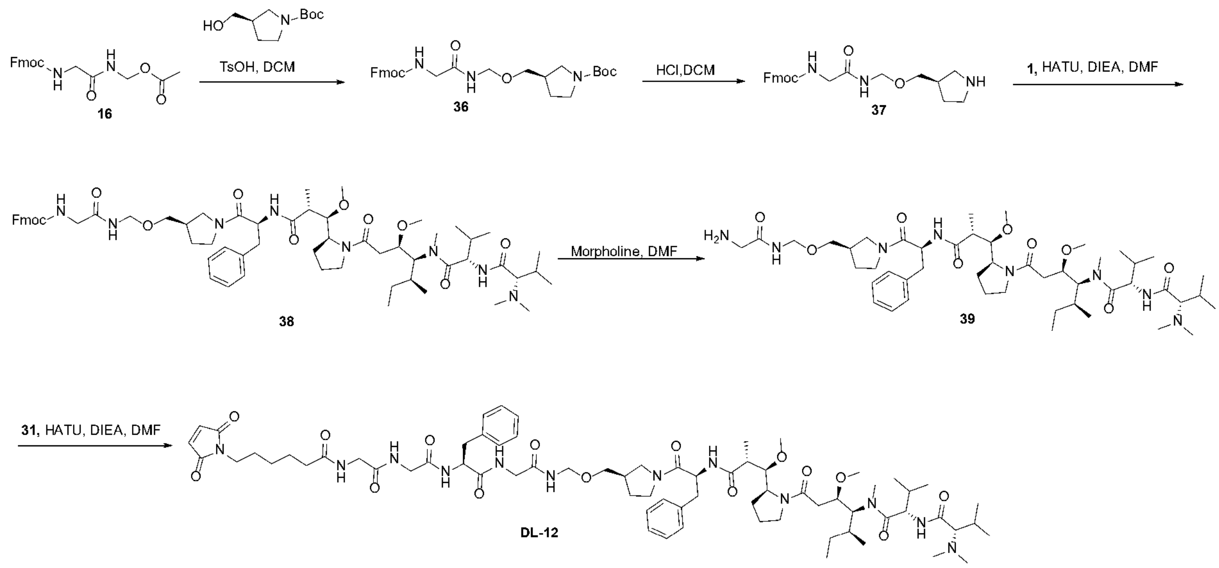


Step 1: Synthesis of tert-butyl (R)-3-(10-(9H-fluoren-9-yl)-5,8-dioxo-2,9-dioxo-4,7-diazadecyl) pyrrolidine-1-carboxylate

Compound 16 (50.0 mg, 0.135 mmol), Compound (R)-1-Boc-3-(hydroxymethyl) pyrrolidine (54.3 mg, 0.270 mmol) and p-toluenesulfonic acid (69.8 mg, 0.405mmol) were added to dichloromethane (3 mL), and the mixture was stirred at room temperature for 2 hours for reaction. The reaction was deemed complete when LCMS monitoring showed the content of Compound 16 in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound 36 (white solid, 23.0 mg, 33.2% yield) was prepared. MS: 510.29 $[M+H]^+$.

Step 2: Synthesis of (9H-fluoren-9-yl) methyl (R)-(2-oxo-2-(((pyrrolidin-3-ylmethoxy) methyl) amino)ethyl) carbamate

Compound 36 (32.00 mg, 0.063 mmol) was added to dichloromethane (2 mL) and HCl (4M dioxane, 1 mL), and the reaction solution was allowed to react at room temperature for 30 minutes. The LCMS monitoring showed complete consumption of starting material Compound 36. The reaction solution was subjected to reduced pressure distillation to remove the solvent, followed by isolation and purification via preparative liquid chromatography using acetonitrile/water. Compound 37 (white solid, 21.0 mg, 81.7% yield) was prepared. MS: 410.29 $[M+H]^+$.

Step 3: Synthesis of (9H-fluoren-9-yl) methyl (2-(((R)-1-(((2R,3R,3R)-3-(((S)-1-((3R,4S,4S,5S)-4-((S)-2-((S)-2-(dimethylamino)-3-methylbutanamido)-N,3-dimethylbutanamido)-3-methoxy-5-methylheptanoyl) pyrrolidin-2-yl)-3-ethoxy-2-methylpropanoyl)-L-phenylalanyl) pyrrolidin-3-yl) methoxy) methyl) amino)-2-oxoethyl) carbamate

Compound 1 (38.1 mg, 0.051 mmol) and HATU (19.4 mg, 0.051 mmol) were added to N,N-dimethylformamide (3 mL), the mixture was stirred at room temperature for 10 minutes for reaction, then DIEA (26 μL, 0.153 mmol) and Compound 37 (21.0 mg, 0.051 mmol) were added and the mixture was stirred at room temperature for 1 hour for reaction. The reaction was deemed complete when LCMS monitoring showed the content of Compound 37 in the reaction solution was less than 3%. The solvent of the reaction solution was evaporated to dryness under reduced pressure, and the crude product was isolated and purified by preparative liquid chromatography using acetonitrile/water. Compound 38 (white solid, 22.0 mg, 37.9% yield) was prepared. MS:1137.98 $[M+H]^+$.

Step 4: Synthesis of (S)-N-((3R,4S,5S)-1-((S)-2-((1R,2R)-3-(((S)-1-((R)-3-(((2-aminoacetamido) methoxy) methyl) pyrrolidin-1-yl)-1-oxo-3-phenylpropan-2-yl) amino)-1-methoxy-2-methyl-3-oxoprop yl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 38 (22.0 mg, 0.019 mmol) was added to N,N-dimethylformamide (2 mL) and morpholine (1 mL), and the reaction solution was allowed to react at room temperature for 30 minutes. The LCMS monitoring showed complete consumption of starting material Compound 38. The reaction solution was subjected to reduced pressure distillation to remove the solvent, followed by isolation and purification via preparative liquid chromatography using acetonitrile/water. Compound 39 (white solid, 12.0 mg, 67.8% yield) was prepared. MS: 915.83 $[M+H]^+$.

Step 5: Synthesis of N-((S)-9-benzyl-1-((R)-1-(((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-3-methoxy-2-methylpropanoyl)-L-phenylalanyl) pyrrolidin-3-yl)-5,8,11,14-tetraoxo-2-oxa-4,7,10,13-tetraazapentadecyl-15-yl)-6-(2,5-dioxo-2,5-dihydro-1H-pyrrol-1-yl)hexanamide

Compound 31 (6.2 mg, 0.013 mmol) and HATU (5.0 mg, 0.013 mmol) were added to N,N-dimethylformamide (3 mL), the mixture was stirred at room temperature for 10 minutes for reaction, then DIEA (7 μL, 0.039 mmol) and Compound 39 (12.0 mg, 0.013 mmol) were added and the mixture was stirred at room temperature for 1 hour for reaction. The reaction was deemed complete when LCMS monitoring showed the content of Compound 31 in the reaction solution was less than 3%. The solvent of the reaction solution was evaporated to dryness under reduced pressure, and the crude product was isolated and purified by preparative liquid chromatography using acetonitrile/water. Compound DL-12 (white solid, 6.4 mg, 35.6% yield) was prepared. MS: 1369.69 $[M+H]^+$.

$^1H$ NMR (400 MHz, DMSO-$d_6$) δ 9.50 (s, 2H), 8.92 (m, 2H), 8.49 (m, 3H), 8.27 (m, 3H), 8.17 - 7.92 (m, 5H), 7.41 - 7.31 (m, 2H), 7.27 - 7.17 (m, 10H), 6.99 (s, 2H), 4.73 - 4.44 (m, 8H), 3.98 (s, 2H), 3.83 (m, 1H), 3.80 - 3.64 (m, 9H), 3.65 - 3.59 (m, 2H), 3.58 - 3.54 (m, 2H), 3.28 - 3.21 (m, 6H), 3.21 - 3.12 (m, 7H), 3.12 - 3.03 (m, 4H), 3.00 (s, 4H), 2.86 - 2.71 (m, 10H), 2.70 (s, 1H), 2.39 - 2.19 (m, 7H), 2.18 - 1.98 (m, 6H), 1.73 (m, 6H), 1.48 (m, 9H), 1.39 - 1.09 (m, 8H), 1.10 - 0.81 (m, 22H), 0.77 (m, 4H).

**Example 13: Preparation of Compound DL-13**

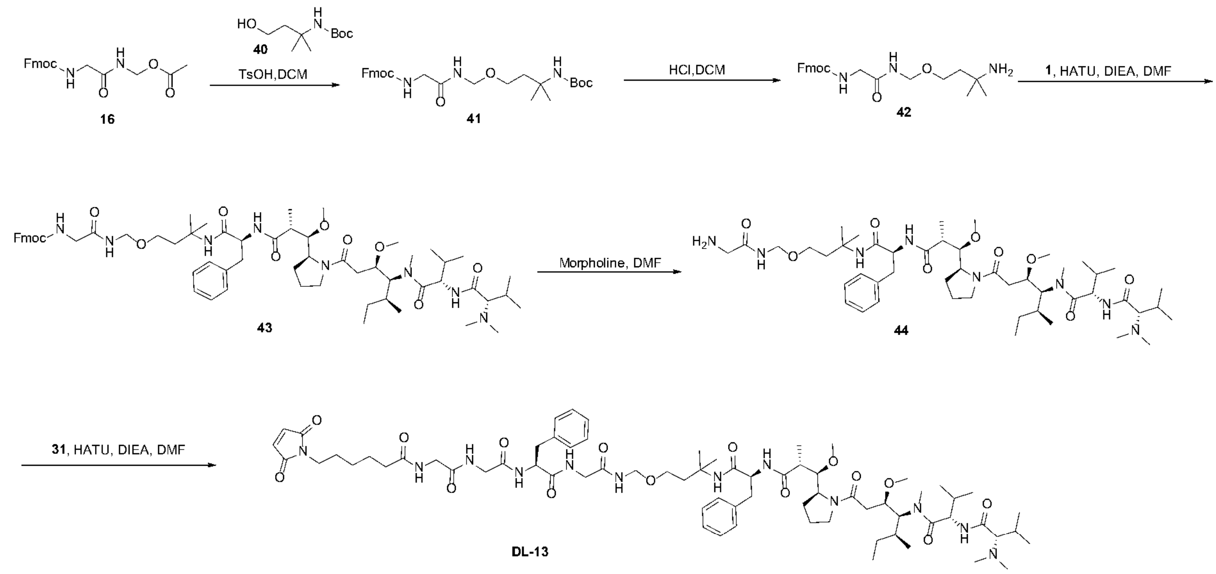


Step 1: Synthesis of (9H-fluoren-9-yl) methyl (8,8,12,12-tetramethyl-2,10-dioxo-5,11-dioxo-3,9-diazatridecyl) carbamate

Compound 16 (60 mg, 0.163 mmol) and Compound 40 (66.67 mg, 0.326 mmol) were added into a 10 mL single-neck flask, dichloromethane (3 mL) and TsOH (2.8 mg, 0.0163 mmol) were added, and the mixture were allowed to react at room temperature for 2 hours. LCMS monitoring showed complete consumption of starting material Compound 16. The crude product was obtained after rotary evaporation, and then isolated and purified by preparative liquid chromatography using acetonitrile/water. Compound 41 (white solid, 38 mg, 56.7% yield) was obtained. MS: 512.26[M+H]$^+$.

Step 2: Synthesis of (9H-fluoren-9-yl) methyl (2-(((3-amino-3-methylbutoxy) methyl) amino)-2-oxoethyl) carbamate

Compound 41 (38 mg, 0.074 mmol) was added to a 5 mL solution of 4 M HCl in dioxane, and the reaction solution was stirred at room temperature for 20 minutes for reaction. LCMS monitoring showed complete consumption of Compound 41 in the reaction solution. The solvent of the reaction solution was evaporated to dryness under reduced pressure, and the crude product was isolated and purified by preparative liquid chromatography using acetonitrile/water, to obtain Compound 42 (30 mg, 97.2% yield). MS: 412.14 [M+H]$^+$.

Step 3: Synthesis of (9H-fluoren-9-yl) methyl ((3R,4R,7R)-7-benzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4,10,10-trimethyl-5,8,16-trioxo-2,13-dioxo-6,9,15-triazaheptadecan-17-yl) carbamate

Compound 1 (30 mg, 0.072 mmol), HATU (54.72 mg, 0.144 mmol), and Compound 42 (53 mg, 0.072 mmol) were added to N,N-dimethylformamide (3 mL), the mixture was stirred at room temperature for 10 minutes for reaction, finally DIEA (46.44 mg, 0.36 mmol) were added and the mixture was stirred at room temperature for 1 hour for reaction. The reaction was deemed complete when LCMS monitoring showed the content of Compound 1 in the reaction solution was less than 3%. The solvent of the reaction solution was evaporated to dryness under reduced pressure, and the crude product was isolated and purified by preparative liquid chromatography using acetonitrile/water. Compound 43 was obtained (30 mg, 36.5% yield). MS: 580.74 [M+H+Na]$^{2+}$.

Step 4: Synthesis of (S)-N-((3R,4S,5S)-1-((S)-2-((3R,4R,7R)-17-amino-7-benzyl-4,10-trimethyl-5,8,16-trioxo-2,13-dioxo-6,9,15-triazaheptadecan-3-yl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 43 (30 mg, 0.0263 mmol) was added into a 25 mL single-neck flask, N,N-dimethylformamide (3 mL) and morpholine (2 mL) were added, and the reaction solution reacted at room temperature for 30 min. LCMS monitoring showed the complete consumption of starting material Compound 43. The reaction solution was dried by rotary evaporation, and the crude product was purified by preparative liquid chromatography using acetonitrile/water. Compound 44 (white solid, 18 mg, 74.5% yield) was obtained. MS: 917.25 [M+H]$^+$.

Step 5: Synthesis of N-((3R,4R,7R,20S)-7,20-dibenzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4,10,10-trimethyl-5,8,16,19,22,5-hexoxo-2,13-dioxo-6,9,15,18,21,24-hexaazaoctacosan-26-yl)-6-(2,5-dioxo-2,5-dihydro-1H-pyrrol-1-yl)hexanamide

Compound 31 (9.25 mg, 0.0196 mmol), HATU (14.89 mg, 0.0392 mmol), and Compound 44 (18 mg, 0.0196 mmol) were added to N,N-dimethylformamide (3 mL), finally DIEA (12.64 mg, 0.098 mmol) were added and the mixture was stirred at room temperature for 1 hour for reaction. The reaction was deemed complete when LCMS monitoring showed the content of Compound 44 in the reaction solution was less than 3%. The solvent of the reaction solution was evaporated to dryness under reduced pressure, and the crude product was isolated and purified by preparative liquid chromatography using acetonitrile/water. Compound DL-12 (white solid, 2.8 mg, 10.4% yield) was prepared. MS: 1371.7 $[M+H]^+$.

$^1H$ NMR (400 MHz, DMSO-$d^6$) δ 9.52 (s, 1H), 8.90 (s, 1H), 8.79 (s, 1H), 8.46 (m, 1H), 8.33 - 8.23 (m, 1H), 8.09 (m, 2H), 7.98 (m, 2H), 7.29 - 7.11 (m, 10H), 6.98 (s, 2H), 4.72 - 4.55 (m, 3H), 4.47 (m, 4H), 3.97 (s, 2H), 3.82 (m, 1H), 3.79 - 3.69 (m, 4H), 3.66 (m, 2H), 3.59 (m, 2H), 3.32 - 3.28 (m, 3H), 3.23 (s, 2H), 3.22 - 3.14 (m, 5H), 3.06 (m, 3H), 2.98 (m, 2H), 2.76 (m, 6H), 2.36 - 2.18 (m, 4H), 2.11 (m, 2H), 2.03 (m, 2H), 1.82 (m, 5H), 1.61 (m, 1H), 1.46 (m, 5H), 1.25 - 1.16 (m, 6H), 1.15 (s, 2H), 1.09 - 1.04 (m, 1H), 1.04 - 0.99 (m, 2H), 0.98 - 0.81 (m, 15H), 0.80 - 0.70 (m, 3H).

**Example 14: Preparation of Compound DL-14**

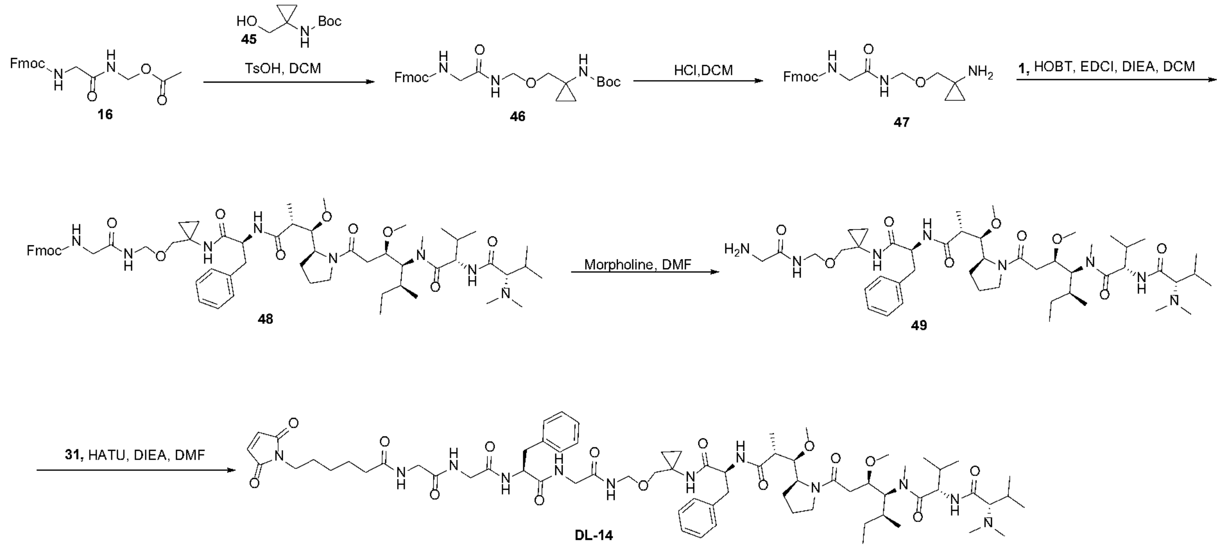


16 46 47 48 49 DL-14

Step 1: Synthesis of (9H-fluoren-9-yl) methyl ((2-((((1-(tert-butoxycarbonyl) amino)cyclopropyl) methoxy) methyl) amino)-2-oxoethyl) carbamate

Compound 16 (60.0 mg, 0.163 mmol), Compound 45 (91.6 mg, 0.489 mmol), and p-toluenesulfonic acid (2.8 mg, 0.016 mmol) were added to dichloromethane (3 mL), and the mixture was stirred at room temperature for 2 hours for reaction. The reaction was deemed complete when LCMS monitoring showed the content of Compound 16 in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound 46 (white solid, 53.0 mg, 65.6% yield) was prepared. MS: 496.45 $[M+H]^+$.

Step 2: Synthesis of (9H-fluoren-9-yl) methyl (2-((((1-aminocyclopropyl) methoxy) methyl) amino)-2-oxoethyl) carbamate

Compound 46 (53.0 mg, 0.107 mmol) was added to dichloromethane (2 mL) and TFA (0.5 mL), and the reaction solution was allowed to react at room temperature for 30 minutes. The LCMS monitoring showed complete consumption of

starting material Compound 46. The reaction solution was subjected to reduced pressure distillation to remove the solvent, followed by isolation and purification via preparative liquid chromatography using acetonitrile/water. Compound 47 (white solid, 15.1 mg, 35.5% yield) was prepared. MS: 396.58 $[M+H]^+$.

Step 3: Synthesis of (9H-fluoren-9-yl) methyl (2-((2R,3R)-3-(S)-1-(3R,4S,4S,5S)-4-(S)-2-((S)-2-(dimethylamino)-3-methylbutanamido)-3-methoxymethylbutenyl-methyl-methyl-(methyl) amino)-2-oxoethyl) carbamate

Compound 1 (28.0 mg, 0.038 mmol), HOBT (5.1 mg, 0.038 mmol), and EDCI (7.2 mg, 0.038 mmol) were added to dichloromethane (3 mL), the mixture was stirred at room temperature for 20 minutes for reaction, then DIEA (19 μL, 0.114 mmol) and Compound 47 (15.0 mg, 0.038 mmol) were added and the mixture was stirred at room temperature for 1 hour for reaction. The reaction was deemed complete when LCMS monitoring showed the content of Compound 47 in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound 48 (white solid, 23.6 mg, 55.3% yield) was prepared. MS: 1124.67 $[M+H]^+$.

Step 4: Synthesis of (S)-N-((3R,4S,5S)-1-((S)-2-((1R,2R)-3-(((S)-1-((1-(((2-aminoacetamido) methoxy) methyl)cyclopropyl) amino)-1-oxo-3-phenylpropan-2-yl) amino)-1-methoxy-2-methyl-3-oxopropyl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 48 (23.6 mg, 0.021 mmol) was added to N,N-dimethylformamide (2 mL) and morpholine (1 mL), and the reaction solution was allowed to react at room temperature for 30 minutes. The LCMS monitoring showed complete consumption of starting material Compound 48. The reaction solution was subjected to reduced pressure distillation to remove the solvent, followed by isolation and purification via preparative liquid chromatography using acetonitrile/water. Compound 49 (white solid, 13.2 mg, 71.4% yield) was prepared. MS: 902.37 $[M+H]^+$.

Step 5: Synthesis of N-((S)-9-benzyl-1-(1-((S)-2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-3-ethoxy-2-methylpropanamido)-3-phenylpropanamido)cyclopropyl)-5,8,11,14-tetraoxo-2-oxa-4,7,10,13-tetraazapentadecan-15-yl)-6-(2,5-dioxo-2,5-dihydro-1H-pyrrol-1-yl)hexanamide

Compound 31 (7.1 mg, 0.015 mmol) and HATU (5.7 mg, 0.015 mmol) were added to N,N-dimethylformamide (3 mL), the mixture was stirred at room temperature for 10 minutes for reaction, then DIEA (8 μL, 0.045 mmol) and Compound 49 (13.2 mg, 0.015 mmol) were added and the mixture was stirred at room temperature for 1 hour for reaction. The reaction was deemed complete when LCMS monitoring showed the content of Compound 49 in the reaction solution was less than 3%. The solvent of the reaction solution was evaporated to dryness under reduced pressure, and the crude product was isolated and purified by preparative liquid chromatography using acetonitrile/water. Compound DL-14 (white solid, 7.8 mg, 38.3% yield) was prepared. MS: 689.56 $[M+Na+H]^{2+}$.

$^1$H NMR (400 MHz, DMSO-$d_6$) δ 9.57 (s, 1H), 8.44 (m, 1H), 8.29 - 8.17 (m, 2H), 8.10 (m, 2H), 8.07 - 7.92 (m, 3H), 7.30 - 7.16 (m, 10H), 7.13 (m, 1H), 6.99 (s, 2H), 4.65 (m, 1H), 4.62 - 4.45 (m, 5H), 3.79 - 3.69 (m, 6H), 3.69 - 3.46 (m, 8H), 3.43 - 3.33 (m, 5H), 3.33 - 3.14 (m, 9H), 3.10 - 2.97 (m, 5H), 2.85 - 2.67 (m, 9H), 2.33 - 2.19 (m, 3H), 2.15 - 1.98 (m, 4H), 1.90 - 1.70 (m, 0H), 1.65 (m, 3H), 1.48 (m, 6H), 1.35 - 1.27 (m, 2H), 1.19 (m, 3H), 1.03 (m, 2H), 1.00 - 0.85 (m, 17H), 0.85 - 0.71 (m, 6H), 0.65 (m, 2H), 0.54 (m, 2H).

**Example 15: Preparation of Compound DL-15**

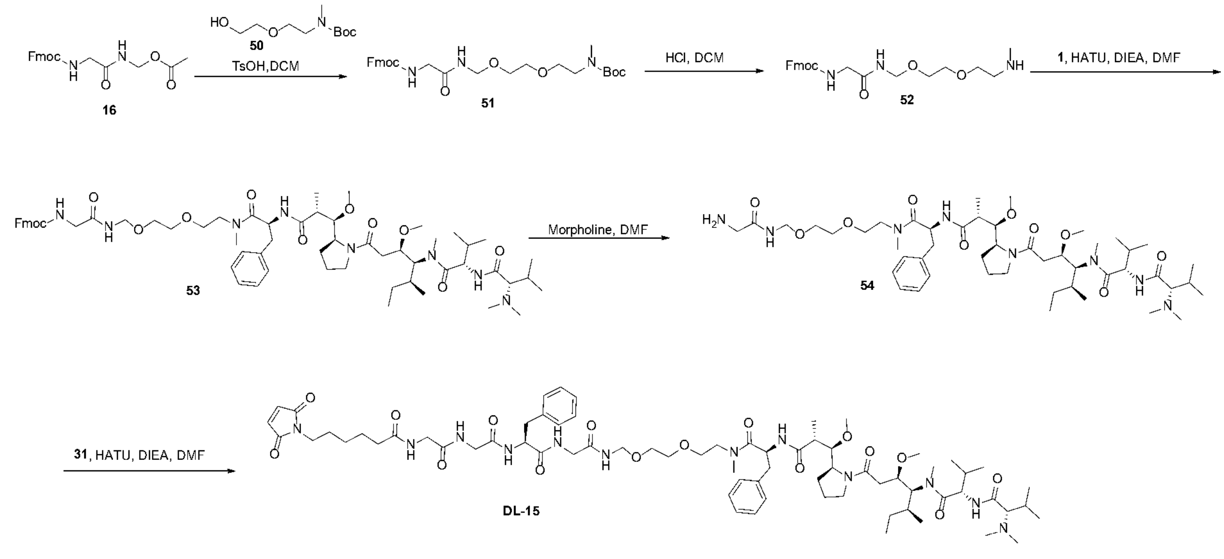


Step 1: Synthesis of tert-butyl (1-(9H-fluoren-9-yl)-3,6-dioxo-2,9,12-trioxo-4,7-diazatetradecan-14-yl)(methyl) carbamate

Compound 16 (67 mg, 0.1825 mmol) and Compound 50 (100 mg, 0.456 mmol) were added into a 10 mL single-neck flask, dichloromethane (3 mL) and TsOH (2.8 mg, 0.0163 mmol) were added, and the mixture were allowed to react at room temperature for 2 hours. LCMS monitoring showed complete consumption of starting material Compound 16. The crude product, Compound 51, was obtained after rotary evaporation and used directly as a starting material for the next step. MS: 528.35[M+H]$^+$.

Step 2: Synthesis of (9H-fluoren-9-yl) methyl (11-oxo-5,8-dioxo-2,10-diazadodecan-12-yl) carbamate

The crude product, Compound 51, was added to a 5 mL solution of 4 M HCl in dioxane, and the reaction solution was stirred at room temperature for 20 minutes for reaction. LCMS monitoring showed complete consumption of Compound 51 in the reaction solution. The solvent of the reaction solution was evaporated to dryness under reduced pressure, and the crude product was isolated and purified by preparative liquid chromatography using acetonitrile/water, to obtain Compound 52 (17 mg, 97.2% yield). MS: 428.29 [M+H]$^+$.

Step 3: Synthesis of (9H-fluoren-9-yl) methyl (((3R,4R,7R)-7-benzyl-3-(((S)-1-(3-methylbutanamido)-N,3-dimethylbutanamido)-3-methoxy-5-methylheptanoyl) pyrrolidin-2-yl)-4,9-dimethyl-5,8,18-trioxo-2,12,15-trioxo-6,9,17-triazapentadecan-19-yl) carbamate

Compound 52 (17 mg, 0.039 mmol), HATU (17.78 mg, 0.0468 mmol), and Compound 1 (32 mg, 0.0429 mmol) were added to N,N-dimethylformamide (3 mL), the mixture was stirred at room temperature for 10 minutes for reaction, finally DIEA (25.155 mg, 0.195 mmol) were added and the mixture was stirred at room temperature for 1 hour for reaction. The reaction was deemed complete when LCMS monitoring showed the content of Compound 52 in the reaction solution was less than 3%. The solvent of the reaction solution was evaporated to dryness under reduced pressure, and the crude product was isolated and purified by preparative liquid chromatography using acetonitrile/water. Compound 53 was obtained (32 mg, 71.0% yield). MS: 1155.99 [M+H]$^+$.

Step 4: Synthesis of (S)-N-((3R,4S,5S)-1-((S)-2-((3R,4R,7R)-19-amino-7-benzyl-4,9-dimethyl-5,8,18-trioxo-2,12,15-trioxa-6,9,17-triazapentadecan-3-yl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 53 (32 mg, 0.0277 mmol) was added into a 25 mL single-neck flask, N,N-dimethylformamide (3 mL) and morpholine (2 mL) were added, and the reaction solution reacted at room temperature for 30 min. LCMS monitoring showed the complete consumption of starting material Compound 53. The reaction solution was dried by rotary evaporation, and the crude product was purified by preparative liquid chromatography using acetonitrile/water. Compound 54 (white solid, 16.5 mg, 63.89% yield) was obtained. MS: 933.82 [M+H]$^+$.

Step 5: Synthesis of N-((3S,4S,7S,22R)-7,22-dibenzyl-3-((R)-1-(((3S,4R,5R)-4-((R, (R)-2-(dimethylamino)-3-methylbutanamido)-3-methylbutanamido)-N,3-dimethylbutanamido)-3-methoxy-5-methylheptanoyl) pyrrolidin-2-yl)-4,9-dimethyl-5,8,18,21,24,27-hexoxo-2,12,15-trioxo-6,9,17,20,23,26-hexaazaoctacosan-28-yl)-6-(2,5-dioxo-2,5-dihydro-1H-pyrrol-1-yl)hexanamide

Compound 31 (16.5 mg, 0.0177 mmol), HATU (8.11 mg, 0.0213 mmol), and Compound 54 (9.23 mg, 0.0195 mmol) were added to N,N-dimethylformamide (3 mL), finally DIEA (11.47 mg, 0.0889 mmol) were added and the mixture was stirred at room temperature for 4 hour for reaction. The reaction was deemed complete when LCMS monitoring showed the content of Compound 54 in the reaction solution was less than 3%. The solvent of the reaction solution was evaporated to dryness under reduced pressure, and the crude product was isolated and purified by preparative liquid chromatography using acetonitrile/water. Compound DL-15 (white solid, 13 mg, 10.4% yield) was prepared. MS: 705.8 $[M+H+Na]^{2+}$.

$^1$H NMR (400 MHz, DMSO-$d_6$) δ 9.51 (s, 1H), 8.96 - 8.86 (m, 1H), 8.54 - 8.38 (m, 2H), 8.24 (m, 2H), 8.08 (m, 2H), 8.03 - 7.94 (m, 1H), 7.28 - 7.11 (m, 9H), 6.98 (m, 2H), 5.05 (m, 1H), 4.89 (m, 1H), 4.70 (s, 1H), 4.66 - 4.57 (m, 2H), 4.56 - 4.38 (m, 4H), 3.71 (m, 8H), 3.63 - 3.53 (m, 3H), 3.40 - 3.32 (m, 3H), 3.31 - 3.13 (m, 8H), 3.03 (m, 7H), 2.92 - 2.86 (m, 1H), 2.86 - 2.70 (m, 8H), 2.30 (m, 4H), 2.11 (m, 3H), 2.06 - 1.97 (m, 1H), 1.75 (m, 4H), 1.47 (m, 5H), 1.32 - 1.24 (m, 2H), 1.18 (m, 3H), 1.07 - 0.91 (m, 11H), 0.90 - 0.81 (m, 6H), 0.77 (m, 3H).

**Example 16: Preparation of Compound DL-16**

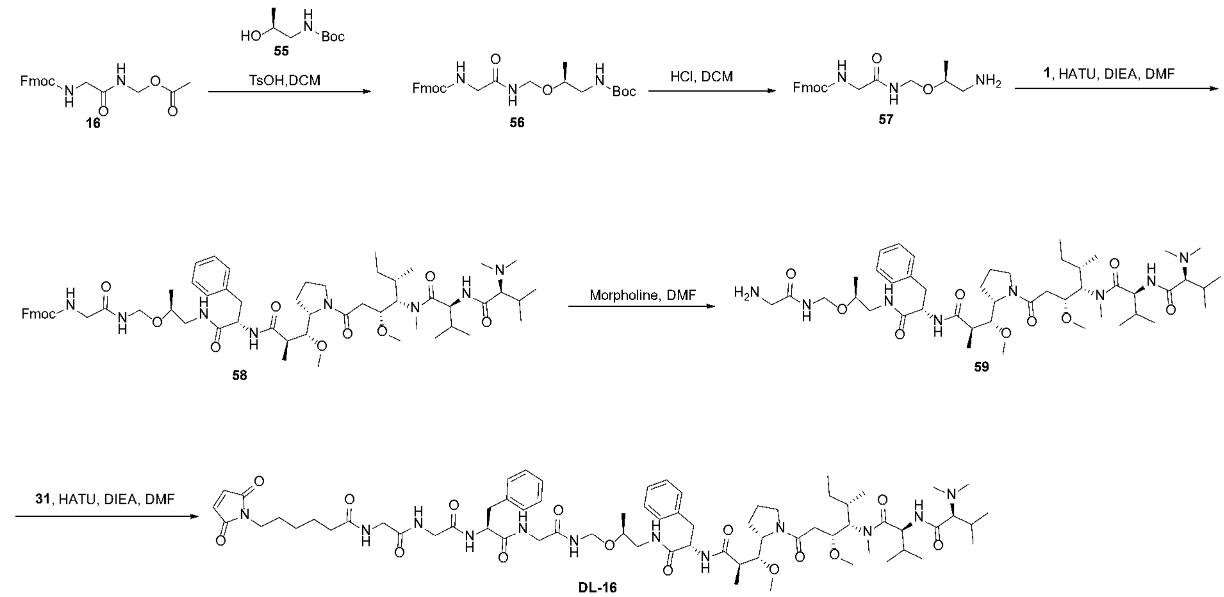


Step 1: Synthesis of (9H-fluoren-9-yl) methyl (S)-(6,11,11-trimethyl-2,9-dioxo-5,10-dioxa-3,8-diazadodecyl) carbamate

Compound 16 (143 mg, 0.815 mmol), Compound 55 (100 mg, 0.272 mmol), and TsOH (5 mg, 0.027 mmol) were added to dichloromethane (4 mL), and the mixture was stirred at room temperature for 3 hours for reaction. The reaction was deemed complete when LCMS monitoring showed the content of Compound 16 in the reaction solution was less than 3%. The solvent of the reaction solution was evaporated to dryness under reduced pressure, and the crude product was isolated and purified by preparative liquid chromatography using acetonitrile/water. Compound 56 (white solid, 86 mg, 65.4% yield) was prepared. MS: 484.57 $[M+H]^+$.

Step 2: Synthesis of (9H-fluoren-9-yl) methyl (S)-(2-((((1-aminopropan-2-yl)oxy) methyl) amino)-2-oxoethyl) carbamate

Compound 56 (86 mg, 0.178 mmol) was added into a 25 mL single-neck flask, dichloromethane (4 mL) and trifluoroacetate (2 mL) were added, and the reaction solution reacted at room temperature for 1h. LCMS monitoring showed the complete consumption of starting material Compound 56. The crude product was obtained after rotary evaporation, and then isolated and purified by preparative liquid chromatography using acetonitrile/water to obtain

Compound 57 (50 mg, 73.3% yield). MS: 384.45[M+H]$^+$.

Step 3: Synthesis of (9H-fluoren-9-yl) methyl ((3R,4R,7S,11S)-7-benzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4,11-dimethyl-5,8,15-trioxo-2,12-dioxa-6,9,14-triazahexadecan-16-yl) carbamate

Compound 1 (78 mg, 0.104 mmol), HOBT (24 mg, 0.177 mmol), and EDCI (30 mg, 0.156 mmol) were added to dichloromethane (5 mL), the mixture was stirred at room temperature for 10 minutes, finally Compound 57 (40 mg, 0.104 mmol) was added and the mixture was stirred at room temperature for 3 hours for reaction. The reaction was deemed complete when LCMS monitoring showed the content of Compound 57 in the reaction solution was less than 3%. The solvent of the reaction solution was evaporated to dryness under reduced pressure, and the crude product was isolated and purified by preparative liquid chromatography using acetonitrile/water. Compound 58 was obtained (39 mg, 32.8% yield). MS: 1112.44 [M+H]$^+$.

Step 4: Synthesis of (S)-N-((3R,4S,5S)-1-((S)-2-((3R,4R,7S,11S)-16-amino-7-benzyl-4,11-dimethyl-5,8,15-trioxo-2,12-dioxa-6,9,14-triazahexadecan-3-yl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 58 (39 mg, 0.035 mmol) was added into a 25 mL single-neck flask, N,N-dimethylformamide (3 mL) and morpholine (1 mL) were added, and the reaction solution reacted at room temperature for 30 min. LCMS monitoring showed the complete consumption of starting material Compound 58. The reaction solution was dried by rotary evaporation, and the crude product was purified by preparative liquid chromatography using acetonitrile/water. Compound 59 (white solid, 26 mg, 83.5% yield) was obtained. MS: 890.19[M+H]$^+$.

Step 5: Synthesis of N-((3R,4R,7S,11S,19S)-7,19-dibenzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4,11-dimethyl-5,8,15,18,21,24-hexoxo-2,12-dioxa-6,9,14,17,20,23-hexaazacyclopentan-25-yl)-6-(2,5-dioxo-2,5-dihydro-1H-pyrrol-1-yl)hexanamide

Compound 31 (14 mg, 0.029 mmol) and HATU (13 mg, 0.035 mmol) were added to N,N-dimethylformamide (3 mL), the mixture was stirred at room temperature for 10 minutes for reaction, then Compound 59 (26 mg, 0.029 mmol) and DIEA (15 mg, 0.116 mmol) were added and the mixture was stirred at room temperature for 3 hours for reaction. The reaction was deemed complete when LCMS monitoring showed the content of Compound 59 in the reaction solution was less than 3%. The solvent of the reaction solution was evaporated to dryness under reduced pressure, and the crude product was isolated and purified by preparative liquid chromatography using acetonitrile/water. Compound DL-16 (19 mg, 48.7% yield) was obtained after lyophilization. MS: 1344.68 [M+H]$^+$.

$^1$H NMR (400 MHz, DMSO-$d_6$) δ 9.54 (s, 1H), 8.88 (m, 1H), 8.45 m, 1H), 8.31 - 8.24 (m, 1H), 8.13 - 8.05 (m, 2H), 7.99 (m, 1H), 7.27 - 7.21 (m, 8H), 7.19 (m, 2H), 6.99 (s, 2H), 4.71 - 4.44 (m, 6H), 3.84 - 3.64 (m, 10H), 3.29 - 3.12 (m, 8H), 3.09 - 2.95 (m, 6H), 2.77 m, 8H), 2.37 - 2.21 (m, 3H), 2.11 (t, *J* = 7.4 Hz, 3H), 2.04 - 1.95 (m, 2H), 1.91 - 1.73 (m, 3H), 1.63 (m, 1H), 1.47 (m, 5H), 1.32 - 1.14 (m, 8H), 1.07 - 0.90 (m, 13H), 0.90 - 0.81 (m, 9H), 0.80 - 0.72 (m, 3H).

**Example 17: Preparation of Compound DL-17**

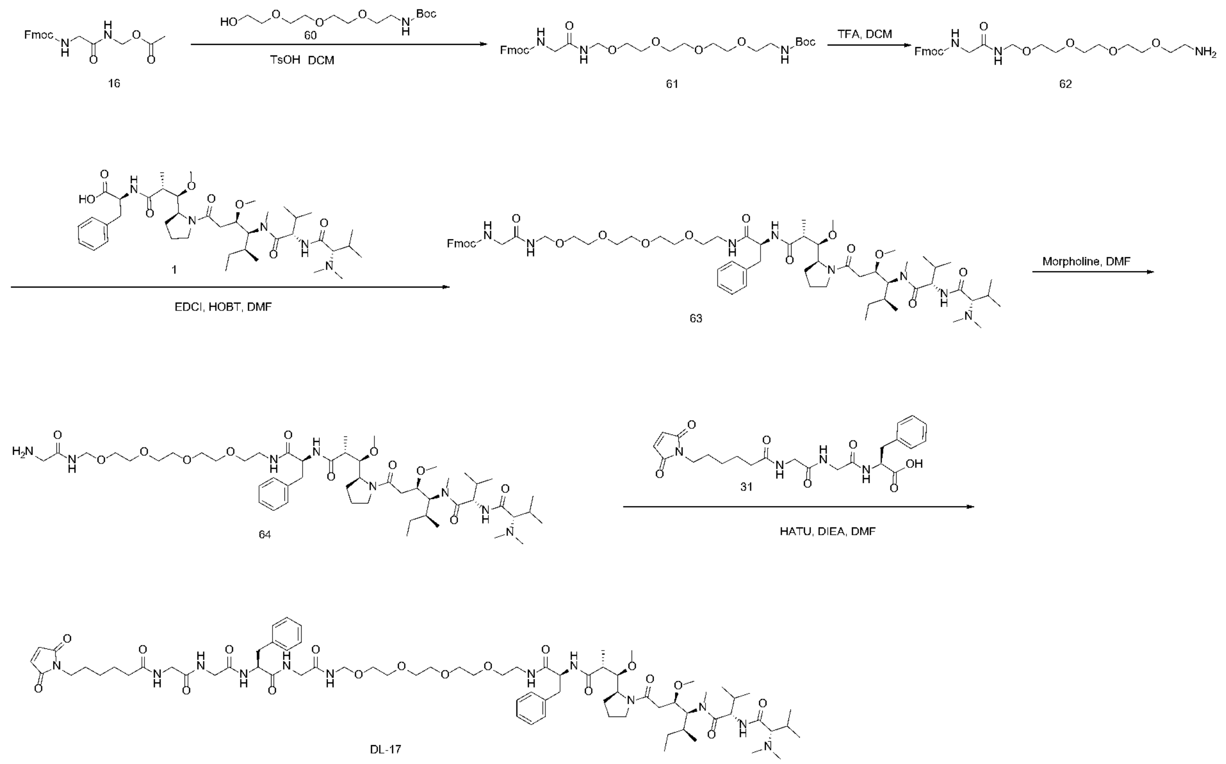


Step 1: Synthesis of (9H-fluoren-9-yl) methyl (2-oxo-5,8,11,14-tetraoxa-3-azahexadecan-1,16-diyl)dicarbamate

Compound **16** (200.0 mg, 0.543 mmol), Compound **60** (637.1 mg, 2.172 mmol), and p-toluenesulfonic acid (18.7 mg, 0.109 mmol) were added to dichloromethane (3 mL), and the mixture was stirred at room temperature for 2 hours for reaction. The reaction was deemed complete when LCMS monitoring showed the content of Compound **16** in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound **61** (white solid, 163.3 mg, 50.0% yield) was prepared. MS: 602.71 $[M+H]^+$.

Step 2: Synthesis of (9H-fluoren-9-yl) methyl (16-amino-2-oxo-5,8,11,14-tetraoxa-3-azahexadecyl) carbamate

Compound **61** (163.3 mg, 0.271 mmol) was added to dichloromethane (2 mL) and TFA (0.5 mL), and the reaction solution was allowed to react at 4°C for 1 hour. The LCMS monitoring showed complete consumption of starting material Compound 61. The reaction solution was subjected to reduced pressure distillation to remove the solvent, followed by isolation and purification via preparative liquid chromatography using acetonitrile/water. Compound **62** (white solid, 92.2 mg, 67.7% yield) was prepared. MS: 502.64 $[M+H]^+$.

Step 3: Synthesis of (9H-fluoren-9-yl) methyl ((3R,4R,7S)-7-benzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8,24-trioxo-2,12,15,18,21-pentaoxa-6,9,23-triazapentacosanyl-25-yl) carbamate

Compound 1 (86.9 mg, 0.184 mmol), HOBT (49.7 mg, 0.368 mmol), EDCI (70.5 mg, 0.368 mmol), and Compound 62 (92.2 mg, 0.184 mmol) were added to N,N-dimethylformamide (3 mL), and the mixture was stirred at room temperature for 1 hour for reaction. The reaction was deemed complete when LCMS monitoring showed the content of Compound **62** in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound **63** (white solid, 55.3 mg, 24.5% yield) was prepared. MS: 626.58 $[M+Na+H]^{2+}$.

Step 4: Synthesis of (S)-N-((3R,4S,5S)-1-((S)-2-((3R,4R,7S)-25-amino-7-benzyl-4-methyl-5,8,24-trioxo-2,12,15,18,21-pentaoxa-6,9,23-triazapentacosanyl-3-yl) pyrrolidin-1-yl)-3-methoxy-5-methyl-1-oxocycloheptan-4-yl)-2-((S)-2-(dimethylamino)-3-methylbutanamido)-N,3-dimethylbutanamide

Compound **63** (55.3 mg, 0.0450 mmol) was added to N,N-dimethylformamide (2 mL) and triethylamine (1 mL), and the reaction solution was allowed to react at room temperature for 3 hours. The LCMS monitoring showed complete consumption of starting material Compound **63.** The reaction solution was subjected to reduced pressure distillation to remove the solvent, followed by isolation and purification via preparative liquid chromatography using acetonitrile/water. Compound **64** (white solid, 40.2 mg, 88.7% yield) was prepared.

Step 5: Synthesis of N-((3R,4R,7S,28S)-7,28-dibenzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8,24,27,30,33-hexoxo-2,12,15,18,21-pentaoxa-6,9,23,26,29,32-hexaazatriacontan-34-yl)-6-(2,5-dioxo-2,5-dihydro-1H-pyrrol-1-yl)hexanamide

Compound 31 (18.8 mg, 0.0397 mmol), HOBT (10.7 mg, 0.0794 mmol), EDCI (15.2 mg, 0.0794 mmol), and Compound 64 (40.2 mg, 0.0397 mmol) were added to N,N-dimethylformamide (3 mL), and the mixture was stirred at room temperature for 1 hour for reaction. The reaction was deemed complete when LCMS monitoring showed the content of Compound **64** in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound **DL-17** (white solid, 19.1 mg, 32.7% yield) was prepared. MS: 742.48 $[M+Na+H]^{2+}$.

$^1$H NMR (400 MHz, DMSO-$d_6$) δ 8.47 (d, 1H), 8.31 - 8.24 (m, 2H), 8.11 (d, 1H), 8.08 - 8.02 (m, 3H), 8.00 (d, 2H), 7.96 (t, 1H), 7.29 - 7.13 (m, 10H), 7.00 (d, 2H), 4.67 (d, 3H), 4.58 - 4.48 (m, 4H), 3.98 (s, 2H), 3.78 (t, 2H), 3.70 (dd, 5H), 3.62 (d, 1H), 3.50 (dd, 11H), 3.23 - 3.16 (m, 8H), 3.08 (s, 2H), 3.05 (d, 2H), 3.00 (s, 3H), 2.70 - 2.62 (m, 3H), 2.36 - 2.31 (m, 2H), 2.21 (d, 6H), 2.12 (t, 3H), 1.92 (s, 4H), 1.75 (s, 4H), 1.48 (q, 6H), 1.21 (q, 4H), 1.04 (d, 2H), 1.00 - 0.81 (m, 14H), 0.74 (dt, 6H).

**Example 18: Preparation of Compound DL-18**

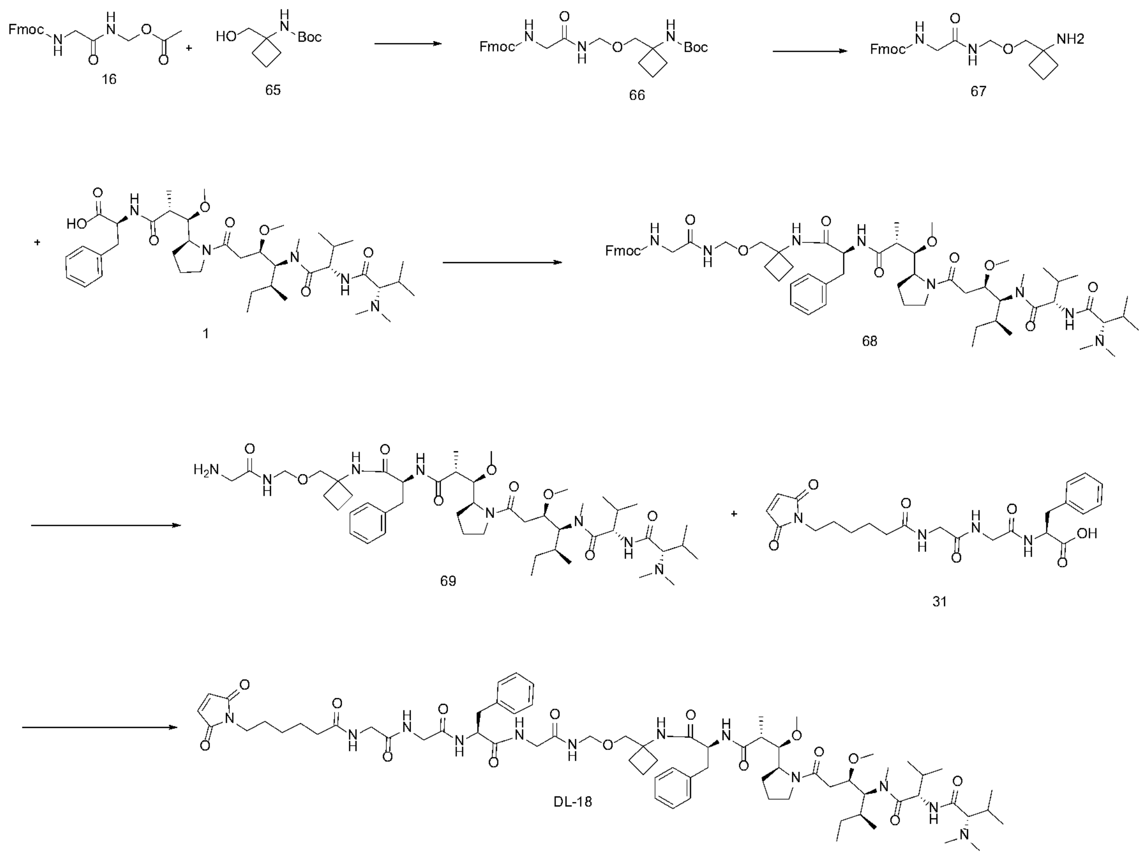

16 65 66 67 1 68 69 31 DL-18

Step 1: Synthesis of tert-butyl (1-(10-(9H-fluoren-9-yl)-5,8-dioxo-2,9-dioxo-4,7-diazadecyl)cyclobutyl) carbamate

Compound 16 (50 mg, 0.136 mmol), Compound 65 (80 mg, 0.398 mmol), and TsOH (5 mg, 0.027 mmol) were added to dichloromethane (4 mL), and the mixture was stirred at room temperature for 3 hours for reaction. The reaction was deemed complete when LCMS monitoring showed the content of Compound 65 in the reaction solution was less than 3%. The solvent of the reaction solution was evaporated to dryness under reduced pressure, and the crude product was isolated and purified by preparative liquid chromatography using acetonitrile/water. Compound 66 (white solid, 22 mg, 31.7% yield) was prepared. MS: 510.60 $[M+H]^+$.

Step 2: Synthesis of (9H-fluoren-9-yl) methyl (2-((((1-aminocyclobutyl) methoxy) methyl) amino)-2-oxoethyl) carbamate

Compound 66 (22 mg, 0.043 mmol) was added into a 25 mL single-neck flask, dichloromethane (4 mL) and trifluoroacetate (2 mL) were added, and the reaction solution reacted at room temperature for 1h. LCMS monitoring showed the complete consumption of starting material Compound 66. The crude product was obtained after rotary evaporation, and then isolated and purified by preparative liquid chromatography using acetonitrile/water to obtain Compound 67 (10 mg, 56.7% yield). MS: 410.49$[M+H]^+$.

Step 3: Synthesis of (9H-fluoren-9-yl) methyl (2-((((1-((S)-2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-3-methyl-2-methylpropanamido)-3-phenylpropanamido)cyclobutyl) methoxy) methyl) amino)-2-oxoethyl) carbamate

Compound 1 (14 mg, 0.018 mmol) and HATU (8.2 mg 0.022 mmol) were added to dichloromethane (5 mL), the mixture was stirred at room temperature for 10 minutes, then Compound 67 (7.5 mg, 0.018 mmol) and DIEA (9.3 mg, 0.072 mmol) were added and the mixture was stirred at room temperature for 3 hours for reaction. The reaction was deemed complete when LCMS monitoring showed the content of Compound 67 in the reaction solution was less than 3%. The solvent of the reaction solution was evaporated to dryness under reduced pressure, and the crude product was isolated and purified by preparative liquid chromatography using acetonitrile/water. Compound 68 was obtained (10 mg, 48.9% yield). MS: 1138.47 $[M+H]^+$.

Step 4: Synthesis of (S)-N-(((3R,4S,5S)-1-((S)-2-((1R,2R)-3-((S)-1-(((2-aminoacetamido) methoxy) methyl)cyclobutyl) amino)-1-oxo-3-phenylpropan-2-yl) amino)-1-methoxy-2-methyl-3-oxopropyl) pyrrolidin-1-yl)-3-methoxy-5-methyl-1-oxoheptan-4-yl)-2-((S)-2-(dimethylamino)-3-methylbutanamido)-N,3-dimethylbutanamide

Compound 68 (10 mg, 0.035 mmol) was added into a 25 mL single-neck flask, N,N-dimethylformamide (3 mL) and TEA (1 mL) were added, and the reaction solution reacted at room temperature for 3h. LCMS monitoring showed the complete consumption of starting material Compound 68. The reaction solution was dried by rotary evaporation, and Compound 69 was obtained. MS: 916.23[M+H]$^+$.

Step 5: Synthesis of N-(((S)-9-benzyl-1-(1-(S)-2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptyl) pyrrolidin-2-yl)-3-methoxy-2-methylpropanamido)-3-phenylpropanamido)cyclobutyl)-5,8,11,14-tetraoxo-2-oxa-4,7,10,13-tetraazapentadecan-15-yl)-6-(2,5-dioxo-2,5-dihydro-1H-pyrrol-1-yl)hexanamide

Compound 31 (4 mg, 0.0088 mmol) and HATU (4 mg, 0.0106 mmol) were added to N,N-dimethylformamide (3 mL), the mixture was stirred at room temperature for 10 minutes for reaction, then Compound 69 (0.0088 mmol) and DIEA (4.5 mg, 0.0352 mmol) were added and the mixture was stirred at room temperature for 3 hours for reaction. The reaction was deemed complete when LCMS monitoring showed the content of Compound 69 in the reaction solution was less than 3%. The solvent of the reaction solution was evaporated to dryness under reduced pressure, and the crude product was isolated and purified by preparative liquid chromatography using acetonitrile/water. Compound DL-18 (2.7 mg, 22.5% yield) was obtained after lyophilization. MS: 1370.71 [M+H]$^+$.

$^1$H NMR (400 MHz, DMSO) δ 8.49 (d, 1H), 8.33 - 8.20 (m, 1H), 8.04 (ddd, 4H), 7.87 (s, 1H), 7.38 - 7.08 (m, 10H), 6.98 (s, 1H), 4.66 (d, 1H), 4.64 - 4.57 (m, 1H), 4.57 - 4.44 (m, 4H), 4.33 (d, 1H), 4.28 - 4.19 (m, 1H), 4.06 (dd, 1H), 3.96 (s, 2H), 3.82 - 3.68 (m, 5H), 3.66 (d, 2H), 3.61 (d, 1H), 3.55 (dd, 4H), 3.46 (s, 4H), 3.18 (dd, 7H), 3.05 (dd, 2H), 2.98 (s, 2H), 2.84 - 2.70 (m, 1H), 2.67 - 2.60 (m, 1H), 2.32 (dd, 1H), 2.19 (d, 7H), 2.09 (dd, 3H), 2.01 (dd, 3H), 1.74 (s, 5H), 1.46 (dd, 4H), 1.33 - 1.11 (m, 4H), 1.01 (dd, 3H), 0.95 - 0.80 (m, 14H), 0.72 (dt, 6H).

**Example 19: Preparation of DL-19**

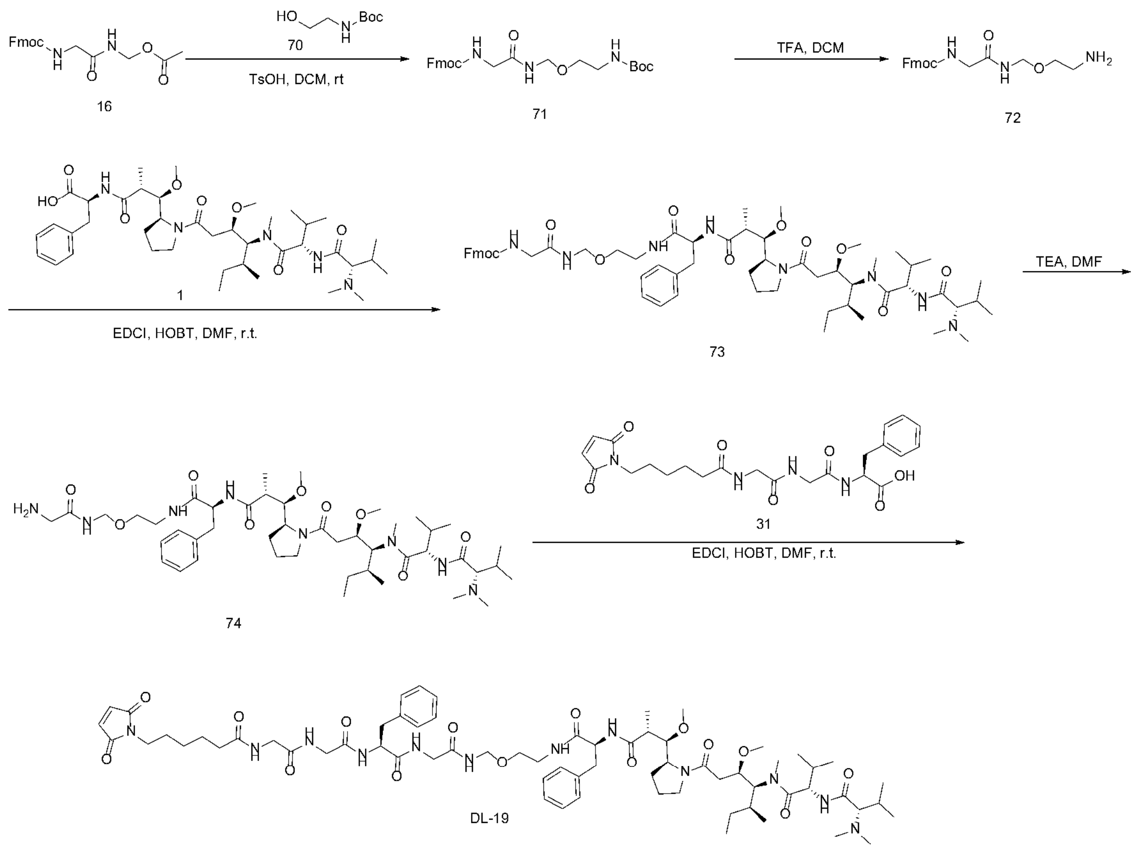

16
70
71
72
1
73
74
31
DL-19

Step 1: Synthesis of tert-butyl (1-(9H-fluoren-9-yl)-3,6-dioxo-2,9-dioxa-4,7-diazaundecan-11-yl) carbamate

Compound **16** (200.0 mg, 0.543 mmol), Compound **70** (350.1 mg, 2.172 mmol), and p-toluenesulfonic acid (18.7 mg, 0.109 mmol) were added to dichloromethane (3 mL), and the mixture was stirred at room temperature for 2 hours for reaction. The reaction was deemed complete when LCMS monitoring showed the content of Compound **16** in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound **71** (white solid, 115.2 mg, 45.2% yield) was prepared. MS: 470.46 $[M+H]^+$.

Step 2: Synthesis of (9H-fluoren-9-yl) methyl (2-(((2-aminoethoxy) methyl) amino)-2-oxoethyl) carbamate

Compound **71** (115.2 mg, 0.245 mmol) was added to dichloromethane (2 mL) and TFA (0.5 mL), and the reaction solution was allowed to react at room temperature for 30 minutes. The LCMS monitoring showed complete consumption of starting material Compound **71.** The reaction solution was subjected to reduced pressure distillation to remove the solvent, followed by isolation and purification via preparative liquid chromatography using acetonitrile/water. Compound **72** (white solid, 60.3 mg, 66.5% yield) was prepared. MS: 370.48 $[M+H]^+$.

Step 3: Synthesis of (9H-fluoren-9-yl) methyl ((3R,4R,7S)-7-benzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8,15-trioxo-2,12-dioxa-6,9,14-triazahexadecan-16-yl) carbamate

Compound 1 (121.8 mg, 0.163 mmol), HOBT (44.1 mg, 0.327 mmol), EDCI (62.6 mg, 0.327 mmol), and Compound 72 (60.3 mg, 0.163 mmol) were added to N,N-dimethylformamide (3 mL), and the mixture was stirred at room temperature for 1 hour for reaction. The reaction was deemed complete when LCMS monitoring showed the content of Compound **72** in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound **73** (white solid, 47.8 mg, 26.7% yield) was prepared. MS: 1098.62 $[M+H]^+$.

Step 4: Synthesis of (S)-N-((3R,4S,5S)-1-((S)-2-((3R,4R,7S)-16-amino-7-benzyl-4-methyl-5,8,15-trioxo-2,12-dioxa-6,9,14-triazahexadecan-3-yl) pyrrolidin-1-yl)-3-methoxy-5-methyl-1-oxocyclopentan-4-yl)-2-((S)-2-(dimethylamino)-3-methylbutanamido)-N,3-dimethylbutanamide

Compound **73** (47.8 mg, 0.0436 mmol) was added to N,N-dimethylformamide (2 mL) and triethylamine (1 mL), and the reaction solution was allowed to react at room temperature for 3 hours. The LCMS monitoring showed complete consumption of starting material Compound **73.** The reaction solution was subjected to reduced pressure distillation to remove the solvent, followed by isolation and purification via preparative liquid chromatography using acetonitrile/water. Compound **74** (white solid, 37.0 mg, 97.1% yield) was prepared. MS: 876.24 $[M+H]^+$.

Step 5: Synthesis of N-((3R,4R,7S,19S)-7,19-dibenzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8,15,18,21,24-hexaoxa-2,12-dioxa-6,9,14,17,20,23-hexaazapentacosan-25-yl)-6-(2,5-dioxo-2,5-dihydro-1H-pyrrol-1-yl)hexanamide

Compound 31 (20.0 mg, 0.0846 mmol), HOBT (11.4 mg, 0.0846 mmol), EDCI (16.2 mg, 0.0846 mmol), and Compound 74 (37.0 mg, 0.0423 mmol) were added to N,N-dimethylformamide (3 mL), and the mixture was stirred at room temperature for 1 hour for reaction. The reaction was deemed complete when LCMS monitoring showed the content of Compound **74** in the reaction solution was less than 3%. The reaction solution was diluted by 2-fold and then washed sequentially with water (15 mL) and saturated brine (15 mL). The mixture was extracted twice, the organic phases were combined and dried over anhydrous sodium sulfate, and the solvent was evaporated to dryness under reduced pressure. The residue was isolated and purified by preparative liquid chromatography using acetonitrile/water. Compound DL-19 (white solid, 17.2 mg, 30.6% yield) was prepared. MS: MS: 676.46 $[M+Na+H]^{2+}$.

$^1H$ NMR (400 MHz, DMSO-$d_6$) $\delta$ 8.27 (dd, 2H), 8.15 - 7.94 (m, 6H), 7.31 - 7.08 (m, 10H), 7.00 (d, 2H), 4.76 - 4.60 (m, 3H), 4.54 (q, 5H), 3.70 (dd, 6H), 3.24 - 3.19 (m, 5H), 3.18 (s, 2H), 3.08 (s, 3H), 3.00 (s, 2H), 2.21 (d, 5H), 2.12 (t, 3H), 1.77 (s, 4H), 1.48 (h, 7H), 1.30 (s, 2H), 1.21 (q, 4H), 1.04 (d, 2H), 1.01 - 0.81 (m, 12H), 0.74 (dt, 6H).

**Example 20: Preparation of DL-20**

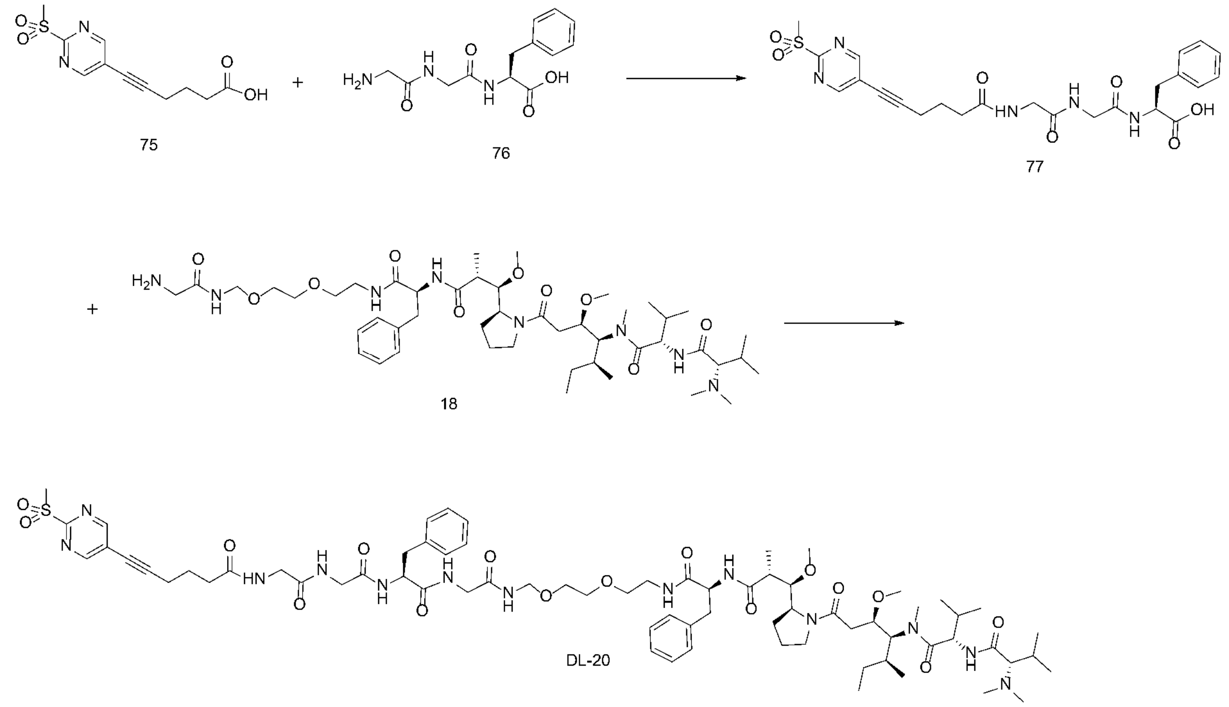

75 76 77 18 DL-20

Step 1: Synthesis of (6-(2-(methylsulfonyl) pyrimidin-5-yl)hex-5-ynyl)diglyceryl-L-phenylalanine

Compound 75 (20 mg, 0.075 mmol) and HATU (28 mg, 0.075 mmol) were added to N,N-dimethylformamide (3 mL), the mixture was stirred at room temperature for 10 minutes, then Compound 76 (21 mg, 0.075 mmol) and DIEA (38 mg 0.3 mmol) were added and the mixture was stirred at room temperature for 3 hours for reaction. The reaction was deemed complete when LCMS monitoring showed the content of Compound 75 in the reaction solution was less than 3%. The solvent of the reaction solution was evaporated to dryness under reduced pressure, and the crude product was isolated and purified by preparative liquid chromatography using acetonitrile/water. Compound 77 was obtained (14 mg, 35.2% yield). MS: 530.57 $[M+H]^+$.

Step 2: Synthesis of N-((3R,4R,7S,22S)-7,22-dibenzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4-methyl-5,8,18,21,24,27-hexaoxo-2,12,15-trioxa-6,9,17,20,23,26-hexaazaoctacosan-28-yl)-6-(2-(methylsulfonyl) pyrimidin-5-yl)hex-5-imide

Compound 77 (5 mg, 0.009 mmol), HOBT (1.8 mg, 0.014 mmol), and EDCI (2.6 mg, 0.014 mmol) were added to N,N-dimethylformamide (3 mL), the mixture was stirred at room temperature for 3 minutes, then Compound 18 (8.7 mg, 0.009 mmol) was added and the mixture was stirred at room temperature for 3 hours for reaction. The reaction was deemed complete when LCMS monitoring showed the content of Compound 18 in N,N- the reaction solution was less than 3%. The solvent of the reaction solution was evaporated to dryness under reduced pressure, and the crude product was isolated and purified by preparative liquid chromatography using acetonitrile/water. Compound DL-20 (5.7 mg, 44.3% yield) was obtained after lyophilization. MS: 1431.77 $[M+H]^+$.

1H NMR (400 MHz, DMSO) δ 9.10 (s, 2H), 8.55 - 7.92 (m, 9H), 7.30 - 7.10 (m, 11H), 4.66 (dd, 1H), 4.58 - 4.47 (m, 4H), 3.97 (s, 1H), 3.80 - 3.68 (m, 6H), 3.60 (dd, 1H), 3.49 (d, 7H), 3.26 - 3.14 (m, 11H), 3.04 (d, 6H), 2.85 - 2.71 (m, 2H), 2.69 - 2.60 (m, 1H), 2.58 (t, 2H), 2.33 (dd, 3H), 2.19 (t, 7H), 1.96 - 1.88 (m, 2H), 1.83 (dt, 3H), 1.77 - 1.73 (m, 1H), 1.46 (dd, 1H), 1.24 (s, 1H), 1.04 (d, 1H), 1.00 - 0.81 (m, 17H), 0.74 (dt, 7H).

**Example 21: Preparation of DL-21**

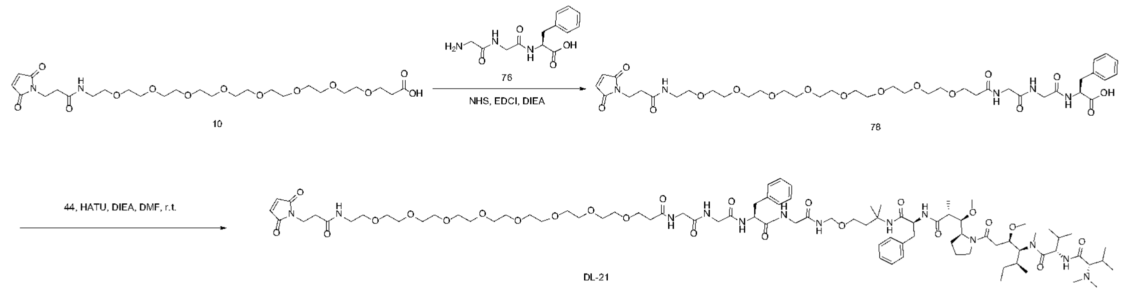


10 78 DL-21

Step 1: Synthesis of (1-(2,5-dioxo-2,5-dihydro-1H-pyrrol-1-yl)-3-oxo-7,10,13,16,19,22,25,28-octaoxa-4-azatriacontan-31-yl)glycylglycyl-L-phenylalanine

Compound 10 (300 mg, 0.507 mmol), NHS (90 mg, 0.783 mmol), and EDCI (150 mg, 0.781 mmol) were added to water (10 mL), and the mixture was stirred at room temperature for 30 minutes for reaction. EDCI (200 mg, 0.781 mmol) was added to the reaction solution, and the resulting mixture was further stirred at room temperature for another 30 minutes for reaction. Subsequently, Compound 76 (300 mg, 0.717 mmol) and DIEA (74.2 mg, 0.575 mmol) were added, and the resulting solution was stirred at room temperature for 30 minutes. The reaction was deemed complete when LCMS monitoring showed the content of Compound 10 in the reaction solution was less than 3%. The solvent of the reaction solution was evaporated to dryness under reduced pressure, and the crude product was isolated and purified by preparative liquid chromatography using acetonitrile/water. Compound 78 (white solid, 150 mg, 34.6% yield) was prepared. MS: 853.40 $[M+H]^+$.

Step 2: Synthesis of N-((3R,4R,7S,20S)-7,20-dibenzyl-3-((S)-1-((3R,4S,5S)-4-((S)-2-((S)-2-(dimethylamino)-3-methylbutanamido)-N,3-dimethylbutanamido)-3-methoxy-5-methylheptanoyl) pyrrolidin-2-yl)-4,10,10-trimethyl-5,8,16,19,22,25-hexaoxo-2,13-dioxa-6,9,15,18,21,24-hexaazahexacosan-26-yl)-1-(3-(2,5-dioxo-2,5-dihydro-1H-pyrrol-1-yl) propanamido)-3,6,9,12,15,18,21,24-octaoxadocosan-27-amide

Compound 78 (22.4 mg, 0.026 mmol) and HATU (12.4 mg, 0.033 mmol) were added to N,N-dimethylformamide (3 mL), the mixture was stirred at room temperature for 10 minutes for reaction, then Compound 44 (20 mg, 0.022 mmol) and DIEA (3.7 mg, 0.028 mmol) were added and the mixture was stirred at room temperature for 3 hours for reaction. The reaction was deemed complete when LCMS monitoring showed the content of Compound 44 in the reaction solution was less than 3%. The solvent of the reaction solution was evaporated to dryness under reduced pressure, and the crude product was isolated and purified by preparative liquid chromatography using acetonitrile/water. Compound DL-21 (5.4 mg, 14.0% yield) was obtained after lyophilization. MS: 1752.01 $[M+H]^+$.

$^1H$ NMR (400 MHz, DMSO) δ 8.48 (m, 1H), 8.30 (m, 1H), 8.16 (m, 1H), 8.13 - 8.06 (m, 1H), 7.98 (m, 2H), 7.29 - 7.18 (m, 5H), 7.15 (m, 1H), 7.01 (s, 1H), 4.71 - 4.57 (m, 2H), 4.57 - 4.41 (m, 3H), 3.79 - 3.66 (m, 3H), 3.63 - 3.58 (m, 2H), 3.53 - 3.47 (m, 14H), 3.37 - 3.27 (m, 53H), 3.24 - 3.13 (m, 6H), 3.11 - 2.91 (m, 5H), 2.85 - 2.71 (m, 2H), 2.70 - 2.60 (m, 2H), 2.40 (m, 2H), 2.36 - 2.29 (m, 2H), 2.21 (m, 5H), 2.01 - 1.81 (m, 4H), 1.76 (s, 2H), 1.63 (m, 1H), 1.46 (s, 1H), 1.32 (s, 2H), 1.26 - 1.17 (m, 4H), 1.04 (m, 2H), 0.97 - 0.82 (m, 8H), 0.74 (m, 4H).

**Example 22: Preparation of ADC-1**

A pre-prepared aqueous solution of the reducing agent tris(2-carboxyethyl) phosphine (4-10 eq) was added to a PBS buffer solution of trastuzumab (0.05M aqueous PBS solution, pH=7.0; 2 mg, 0.014 μmol). The reaction solution was protected with an inert gas, placed in a water bath or metal bath, and subjected to oscillation for reaction at 25°C to 37°C for 0.5 to 2.0 hours. After the reaction ended, the reaction solution was cooled to room temperature and used for the conjugation reaction in the following ADC preparation examples.

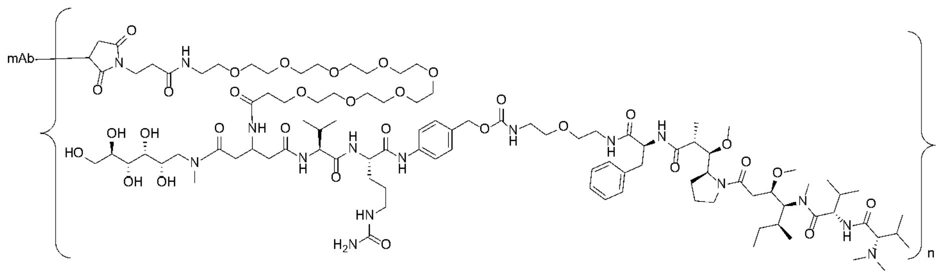

ADC-1

An aqueous solution of compound DL-1 (0.106 mL, 4.0 mg/mL, 0.2 μmol) was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-1 (4.32 mg/mL, 0.43 mL) for storage at -80°C.

The average DAR value (Drug-to-Antibody Ratio) n was calculated to be 6.23 using the reductive reverse-phase chromatography method.

**Example 23: Preparation of ADC-2**

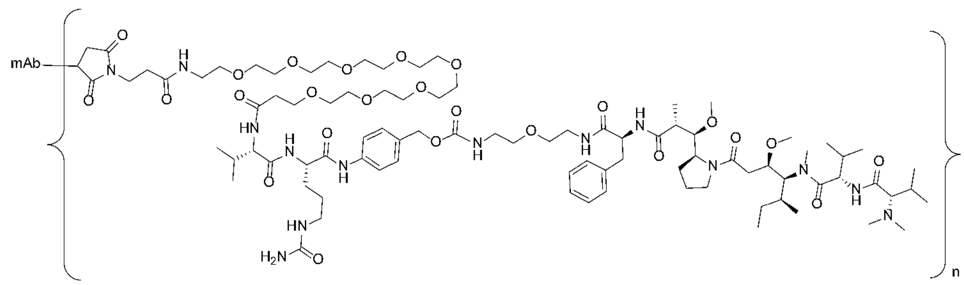

ADC-2

An aqueous solution of compound DL-2 (0.091 mL, 4.0 mg/mL, 0.2 μmol) was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-2 (4.54 mg/mL, 0.4 mL) for storage at -80°C.

The average DAR value n was calculated to be 7.22 using the reductive reverse-phase chromatography method.

**Example 24: Preparation of ADC-3**

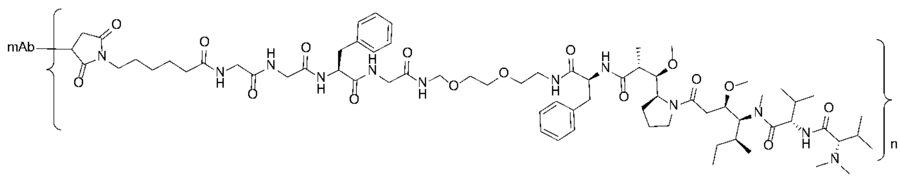

ADC-3

A solution of compound DL-3 (0.091 mL, 4.0 mg/mL, 0.2 μmol) in 10% DMF/water was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and

oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-3 (5.7 mg/mL, 0.32 mL) for storage at -80°C.

The average DAR value n was calculated to be 6.38 using the reductive reverse-phase chromatography method.

**Example 25: Preparation of ADC-4**

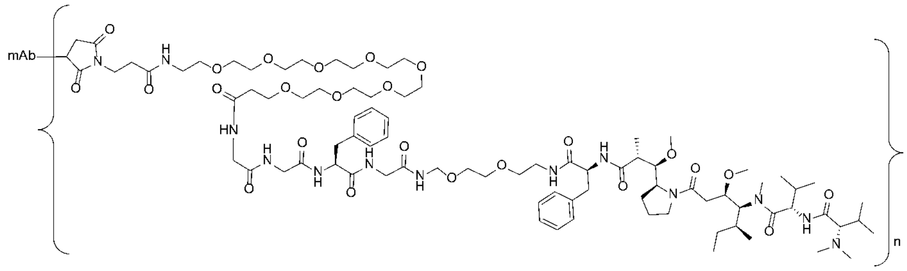

ADC-4

An aqueous solution of compound DL-4 (0.088 mL, 4.0 mg/mL, 0.2 μmol) was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-4 (5.0 mg/mL, 0.37 mL) for storage at -80°C.

The average DAR value n was calculated to be 6.06 using the reductive reverse-phase chromatography method.

**Example 26: Preparation of ADC-5**

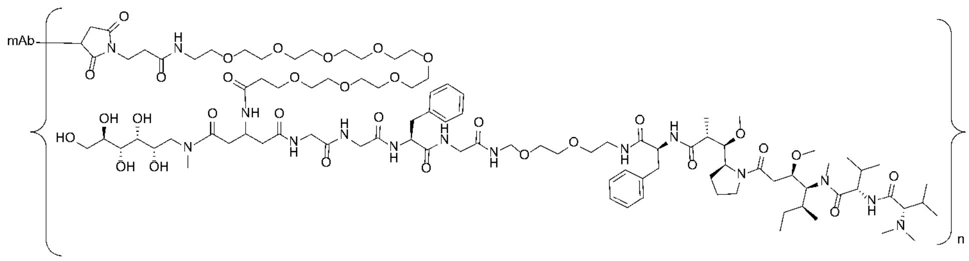

ADC-5

An aqueous solution of compound DL-5 (0.103 mL, 4.0 mg/mL, 0.2 μmol) was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-5 (4.88 mg/mL, 0.38 mL) for storage at -80°C.

The average DAR value n was calculated to be 7.35 using the reductive reverse-phase chromatography method.

**Example 27: Preparation of ADC-6**

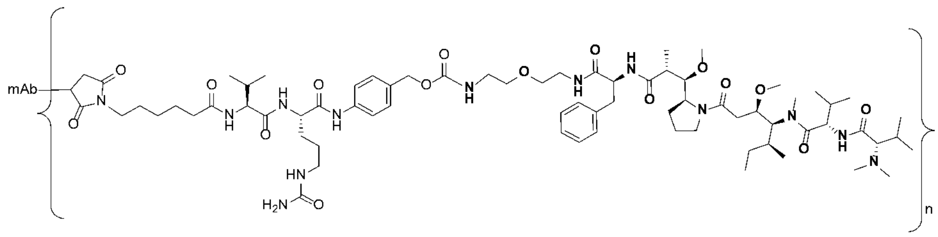

ADC-6

An aqueous solution of compound DL-6 (0.0715 mL, 4.0 mg/mL, 0.2 μmol) was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-6 (4.34 mg/mL, 0.43 mL) for storage at -80°C.

The average DAR value n was calculated to be 7.38 using the reductive reverse-phase chromatography method.

**Example 28: Preparation of ADC-7**

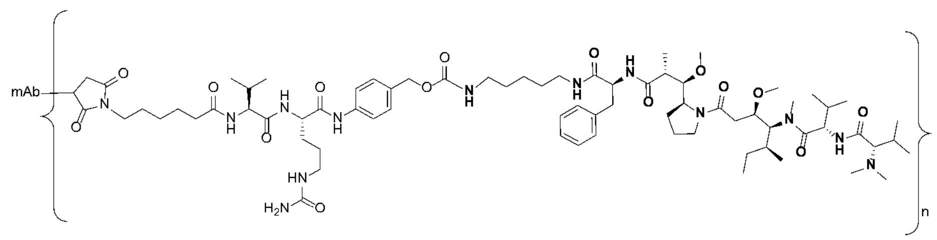

ADC-7

An aqueous solution of compound DL-7 (0.0953 mL, 3.0 mg/mL, 0.2 μmol) was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-7 (5.09 mg/mL, 0.36 mL) for storage at -80°C.

The average DAR value n was calculated to be 7.71 using the reductive reverse-phase chromatography method.

**Example 29: Preparation of ADC-8**

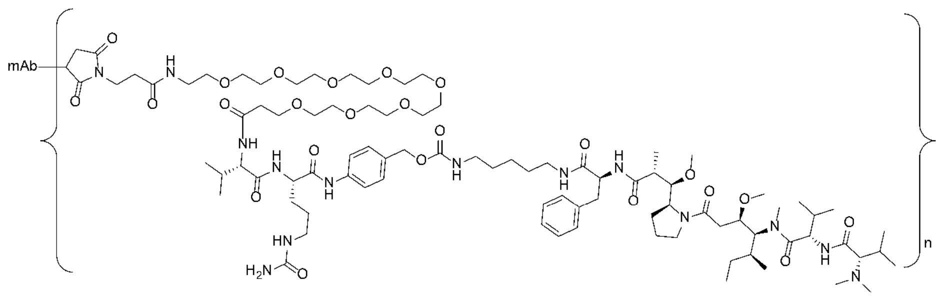

ADC-8

An aqueous solution of compound DL-8 (0.0905 mL, 4.0 mg/mL, 0.2 μmol) was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and

oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-8 (3.69 mg/mL, 0.475 mL) for storage at -80°C.

The average DAR value n was calculated to be 7.35 using the reductive reverse-phase chromatography method.

**Example 30: Preparation of ADC-9**

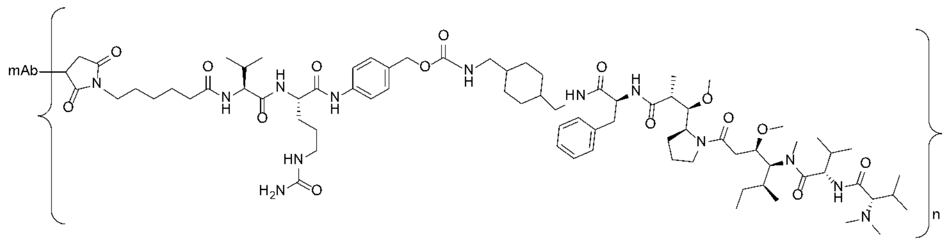

ADC-9

A solution of compound DL-9 (0.074 mL, 4.0 mg/mL, 0.2 μmol) in 20% DMF/water was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the Compound ADC-9 (4.56 mg/mL, 0.4 mL) for storage at -80°C.

The average DAR value n was calculated to be 7.89 using the reductive reverse-phase chromatography method.

**Example 31: Preparation of ADC-10**

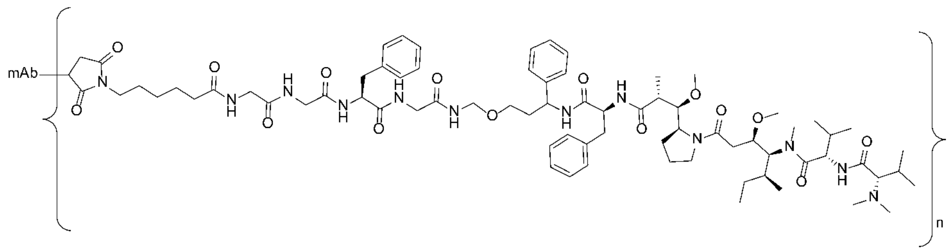

ADC-10

A solution of compound DL-10 (0.071 mL, 4.0 mg/mL, 0.2 μmol) in 20% DMF/water was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-10 (3.1 mg/mL, 0.41 mL) for storage at -80°C.

The average DAR value n was calculated to be 7.81 using the reductive reverse-phase chromatography method.

**Example 32: Preparation of ADC-11**

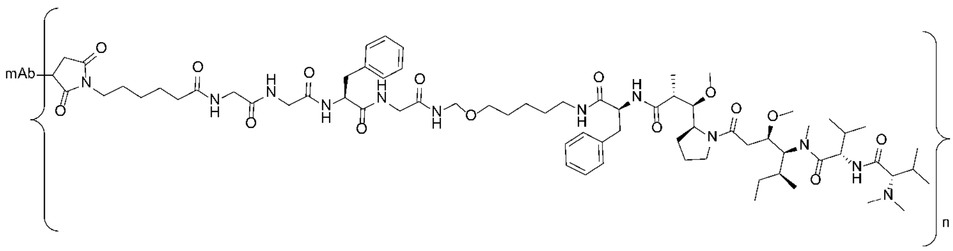

ADC-11

A solution of compound DL-11 (0.069 mL, 4.0 mg/mL, 0.2 μmol) in 20% DMF/water was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-11 (5.5 mg/mL, 0.336 mL) for storage at -80°C.

The average DAR value n was calculated to be 6.2 using the reductive reverse-phase chromatography method.

**Example 33: Preparation of ADC-12**

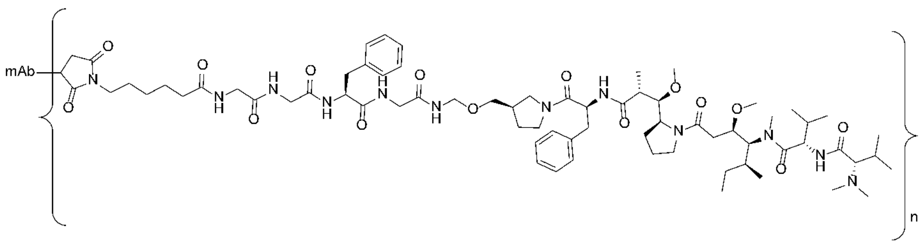

ADC-12

A solution of compound DL-12 (0.069 mL, 4.0 mg/mL, 0.2 μmol) in 20% DMF/water was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-12 (5.14 mg/mL, 0.356 mL) for storage at -80°C.

The average DAR value n was calculated to be 6.5 using the reductive reverse-phase chromatography method.

**Example 34: Preparation of ADC-13**

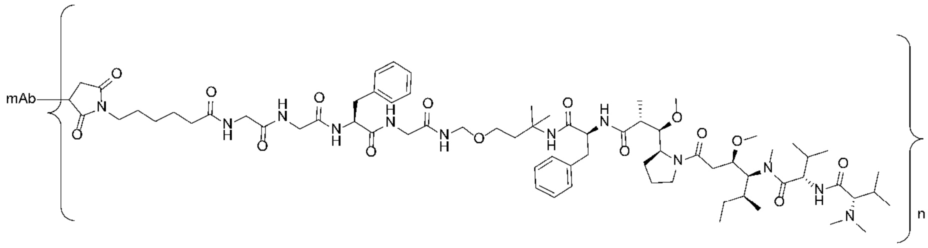

ADC-13

A solution of compound DL-13 (0.068 mL, 4.0 mg/mL, 0.2 μmol) in 20% DMF/water was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to

ultrafiltration and buffer exchange using an Amicon Ultra-15 30 kDa ultrafiltration tube, to obtain a PBS buffer solution of the conjugated product ADC-13 (4.55 mg/mL, 0.4 mL) for storage at -80°C.

The average DAR value n was calculated to be 7.21 using the reductive reverse-phase chromatography method.

**Example 35: Preparation of ADC-14**

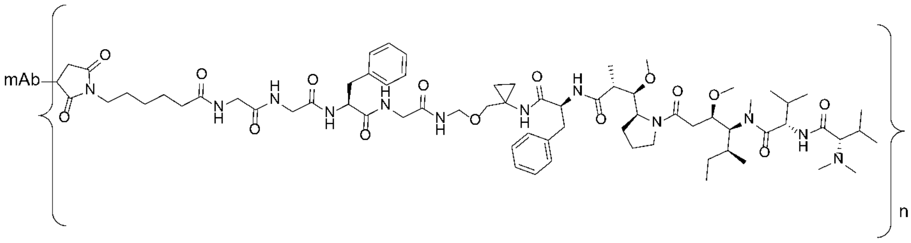

ADC-14

A solution of compound DL-14 (0.068 mL, 4.0 mg/mL, 0.2 μmol) in 20% DMF/water was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration using an Amicon Ultra-15 30 kDa ultrafiltration tube, to obtain a PBS buffer solution of the conjugated product ADC-14 (5.29 mg/mL, 0.35 mL) for storage at -80°C.

The average DAR value n was calculated to be 7.62 using the reductive reverse-phase chromatography method.

**Example 36: Preparation of ADC-15**

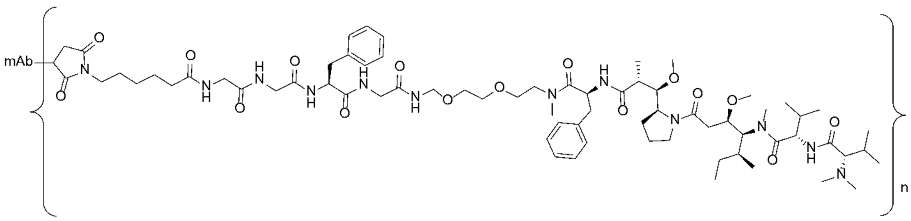

ADC-15

A solution of compound DL-15 (0.07 mL, 4.0 mg/mL, 0.2 μmol) in 20% DMF/water was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube, to obtain a PBS buffer solution of the conjugated product ADC-15 (5.66 mg/mL, 0.34 mL) for storage at -80°C.

The average DAR value n was calculated to be 7.52 using the reductive reverse-phase chromatography method.

**Example 37: Preparation of ADC-16**

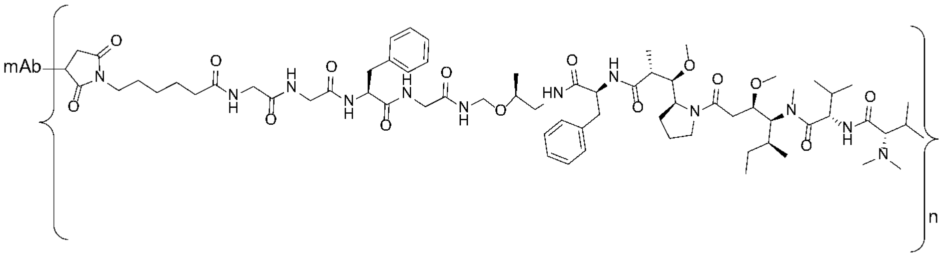

ADC-16

A solution of compound DL-16 (0.07 mL, 4.0 mg/mL, 0.2 μmol) in 20% DMF/water was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube, to obtain a PBS buffer solution of the conjugated product ADC-16 (4.68 mg/mL, 0.4 mL) for storage at -80°C.

The average DAR value n was calculated to be 7.13 using the reductive reverse-phase chromatography method.

**Example 38: Preparation of ADC-17**

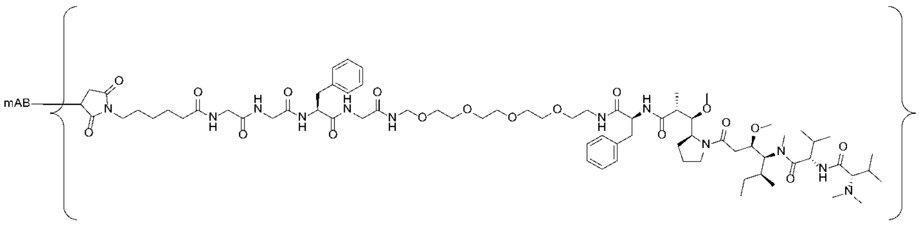

ADC-17

A solution of compound DL-17 (0.076 mL, 4.0 mg/mL, 0.2 μmol) in 20% DMF/water was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube, to obtain a PBS buffer solution of the conjugated product ADC-17 (3.55 mg/mL, 0.55 mL) for storage at -80°C.

The average DAR value n was calculated to be 6.30 using the reductive reverse-phase chromatography method.

**Example 39: Preparation of ADC-18**

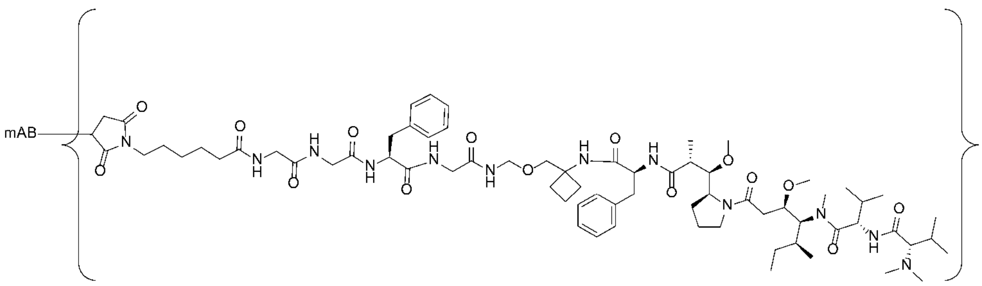

ADC-18

A solution of compound DL-18 (0.071 mL, 4.0 mg/mL, 0.2 μmol) in 20% DMF/water was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and

oscillated for reaction at 25°C for 0.3 hours, and then the reaction ended. The reaction solution was subjected to ultrafiltration and buffer exchange using an Amicon Ultra-15 30 kDa ultrafiltration tube, to obtain a PBS buffer solution of the conjugated product ADC-18 (3.24 mg/mL, 0.52 mL) for storage at -80°C.

The average DAR value n was calculated to be 5.90 using the reductive reverse-phase chromatography method.

**Example 40: Preparation of ADC-19**

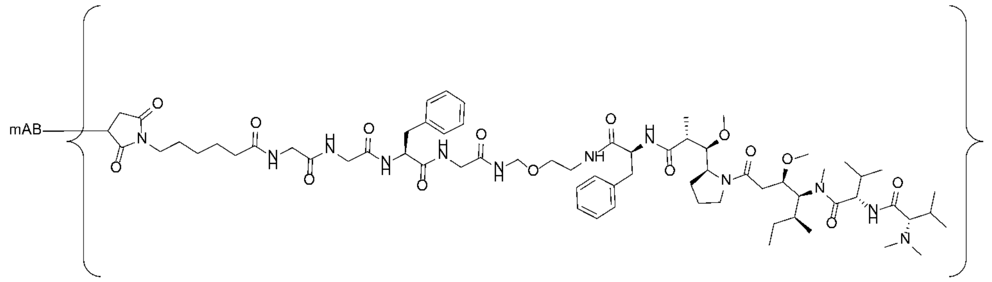

n ADC-19

A solution of compound DL-19 (0.074 mL, 4.0 mg/mL, 0.2 μmol) in 20% DMF/water was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube, to obtain a PBS buffer solution of the conjugated product ADC-19 (3.3 mg/mL, 0.5 mL) for storage at -80°C.

The average DAR value n was calculated to be 6.70 using the reductive reverse-phase chromatography method.

**Example 41: Preparation of ADC-20**

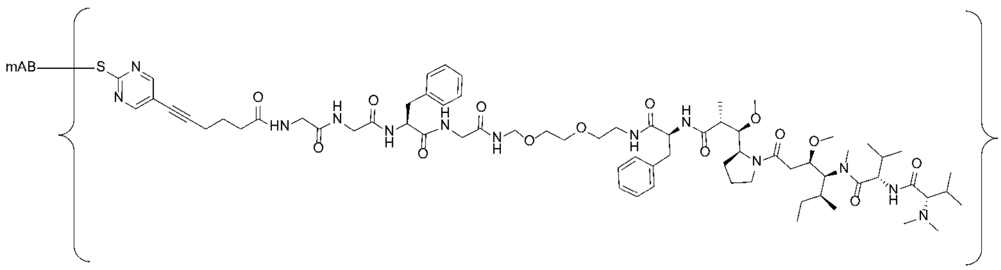

n ADC-20

A solution of compound DL-20 (0.074 mL, 4.0 mg/mL, 0.2 μmol) in 20% DMF/water was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube, to obtain a PBS buffer solution of the conjugated product ADC-20 (3.85 mg/mL, 0.49 mL) for storage at -80°C.

The average DAR value n was calculated to be 7.51 using the reductive reverse-phase chromatography method.

**Example 42: Preparation of ADC-21**

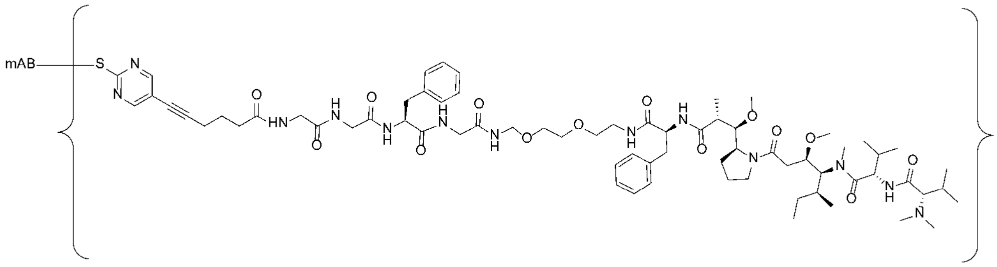

n ADC-21

At 37°C, a pre-prepared aqueous solution of the reducing agent tris(2-carboxyethyl) phosphine (10 mM, 0.014 mL, 0.14 μmol) was added to an aqueous PBS buffer solution of Pertuzumab (0.05M aqueous PBS solution, pH=7.0, 0.092 mL, 21.64 mg/mL, 0.014 μmol). The reaction solution was purged with Ar gas, placed in a metal bath oscillator, and subjected to oscillation for reaction at 37°C for 1.5 hours. After the reaction ended, the reaction solution was allowed to cool naturally to room temperature (25°C) and used for subsequent reaction. A solution of compound DL-20 (0.1 mL, 4.0 mg/mL, 0.28 μmol) in 20% DMF/water was added to 0.106 mL of the foregoing antibody reaction solution. The reaction solution was purged with Ar gas, added into an oscillator, and oscillated for reaction at 25°C for 2.0 hours. After the reaction ended, an aqueous solution of compound NAC (0.004 mL, 100 mM, 0.4 μmol) was added to the reaction solution. The reaction solution was added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-21 (3.69 mg/mL, 0.54 mL) of Pertuzu mAB for storage at -80°C.

The average DAR value n was calculated to be 7.05 using the reductive reverse-phase chromatography method.

**Example 43: Preparation of ADC-22**

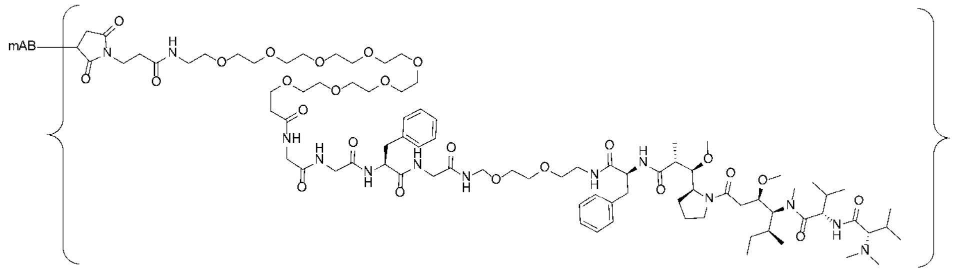

n ADC-22

At 37°C, a pre-prepared aqueous solution of the reducing agent tris(2-carboxyethyl) phosphine (10 mM, 0.008 mL, 0.084 μmol) was added to an aqueous PBS buffer solution of Rituximab (0.05M aqueous PBS solution, pH=7.0, 0.2 mL, 10 mg/mL, 0.014 μmol). The reaction solution was purged with Ar gas, placed in a metal bath oscillator, and subjected to oscillation for reaction at 37°C for 1.5 hours. After the reaction ended, the reaction solution was allowed to cool naturally to room temperature (25°C) and used for subsequent reaction. A solution of compound DL-4 (0.117 mL, 4.0 mg/mL, 0.28 μmol) in 20% DMF/water was added to 0.208 mL of the foregoing antibody reaction solution. The reaction solution was purged with Ar gas, added into an oscillator, and oscillated for reaction at 25°C for 2.0 hours. After the reaction ended, an aqueous solution of compound NAC (0.004 mL, 100 mM, 0.4 μmol) was added to the reaction solution. The reaction solution was added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-22 (5.34 mg/mL, 0.36 mL) of Rituzu mAB for storage at -80°C.

The average DAR value n was calculated to be 7.29 using the reductive reverse-phase chromatography method.

**Example 44: Preparation of ADC-23**

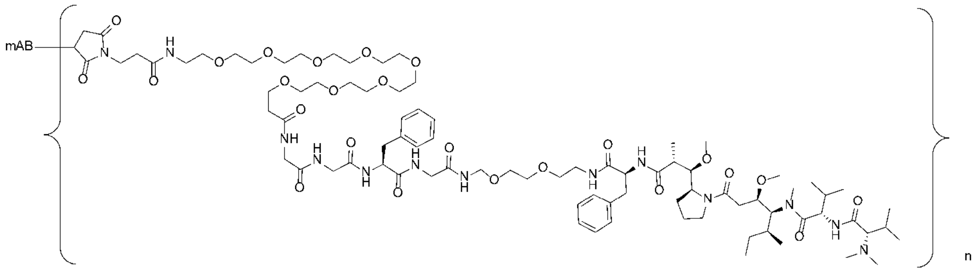

ADC-23

At 37°C, a pre-prepared aqueous solution of the reducing agent tris(2-carboxyethyl) phosphine (10 mM, 0.012 mL, 0.14 μmol) was added to an aqueous PBS buffer solution of mAb-1 (0.05M aqueous PBS solution, pH=7.0, 0.709 mL, 2.82 mg/mL, 0.014 μmol) targeting B7H4 antibody. The reaction solution was purged with Ar gas, placed in a metal bath oscillator, and subjected to oscillation for reaction at 37°C for 1.5 hours. After the reaction ended, the reaction solution was allowed to cool naturally to room temperature (25°C) and used for subsequent reaction. A solution of compound DL-4 (0.117 mL, 4.0 mg/mL, 0.28 μmol) in 20% DMF/water was added to 0.721 mL of the foregoing antibody reaction solution. The reaction solution was purged with Ar gas, added into an oscillator, and oscillated for reaction at 25°C for 2.0 hours. After the reaction ended, an aqueous solution of compound NAC (0.004 mL, 100 mM, 0.4 μmol) was added to the reaction solution. The reaction solution was added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-23 (4.49 mg/mL, 0.45 mL) of AZmAB for storage at -80°C.

The average DAR value n was calculated to be 7.28 using the reductive reverse-phase chromatography method.

**Example 45: Preparation of ADC-24**

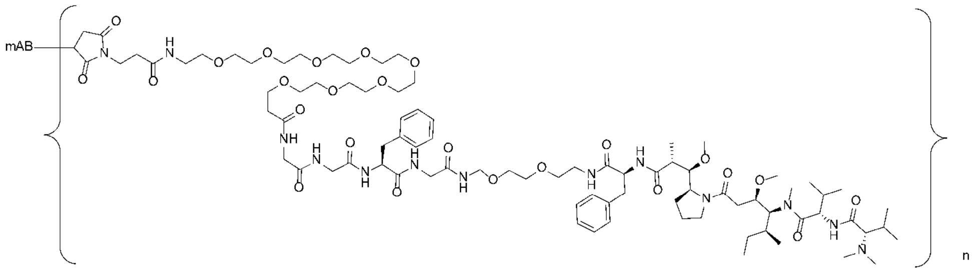

ADC-24

At 37°C, a pre-prepared aqueous solution of the reducing agent tris(2-carboxyethyl) phosphine (10 mM, 0.016 mL, 0.168 μmol) was added to an aqueous PBS buffer solution of Cetuximab (0.05M aqueous PBS solution, pH=7.0, 0.25 mL, 8.0 mg/mL, 0.014 μmol). The reaction solution was purged with Ar gas, placed in a metal bath oscillator, and subjected to oscillation for reaction at 37°C for 1.5 hours. After the reaction ended, the reaction solution was allowed to cool naturally to room temperature (25°C) and used for subsequent reaction. A solution of compound DL-4 (0.117 mL, 4.0 mg/mL, 0.28 μmol) in 20% DMF/water was added to 0.266 mL of the foregoing antibody reaction solution. The reaction solution was purged with Ar gas, added into an oscillator, and oscillated for reaction at 25°C for 2.0 hours. After the reaction ended, an aqueous solution of compound NAC (0.004 mL, 100 mM, 0.4 μmol) was added to the reaction solution. The reaction solution was added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-24 (4.04 mg/mL, 0.51 mL) of Cetuxi mAB for storage at -80°C.

The average DAR value n was calculated to be 7.79 using the reductive reverse-phase chromatography method.

**Example 46: Preparation of ADC-25**

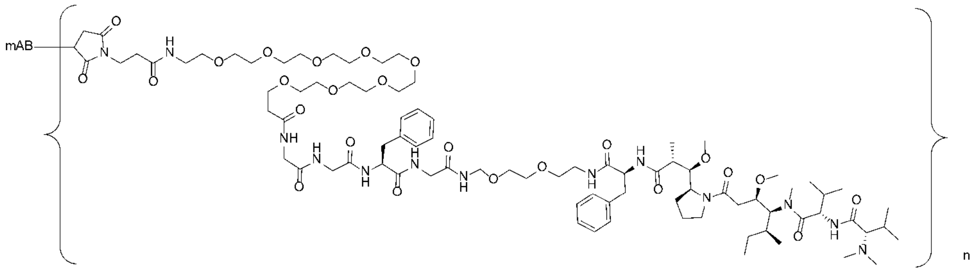

ADC-25

At 37°C, a pre-prepared aqueous solution of the reducing agent tris(2-carboxyethyl) phosphine (10 mM, 0.014 mL, 0.14 μmol) was added to an aqueous PBS buffer solution of Pertuzumab (0.05M aqueous PBS solution, pH=7.0, 0.092 mL, 21.64 mg/mL, 0.014 μmol). The reaction solution was purged with Ar gas, placed in a metal bath oscillator, and subjected to oscillation for reaction at 37°C for 1.5 hours. After the reaction ended, the reaction solution was allowed to cool naturally to room temperature (25°C) and used for subsequent reaction. A solution of compound DL-4 (0.117 mL, 4.0 mg/mL, 0.28 μmol) in 20% DMF/water was added to 0.106 mL of the foregoing antibody reaction solution. The reaction solution was purged with Ar gas, added into an oscillator, and oscillated for reaction at 25°C for 2.0 hours. After the reaction ended, an aqueous solution of compound NAC (0.004 mL, 100 mM, 0.4 μmol) was added to the reaction solution. The reaction solution was added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-25 (3.85 mg/mL, 0.51 mL) of Pertuzu mAB for storage at -80°C.

The average DAR value n was calculated to be 4.42 using the reductive reverse-phase chromatography method.

**Example 47: Preparation of ADC-26**

An aqueous solution of compound DL-21 (0.088 mL, 4.0 mg/mL, 0.2 μmol) was added to 0.208 mL of the foregoing antibody reaction solution. The reaction solution was purged with Ar gas, added into an oscillator, and oscillated for reaction at 25°C for 1.5 hours. After the reaction ended, an aqueous solution of compound NAC (0.004 mL, 100 mM, 0.4 μmol) was added to the reaction solution. The reaction solution was again purged with Ar gas, added into the oscillator, and oscillated for reaction at 25°C for 0.3 hours, and then the reaction ended. The reaction solution was subjected to ultrafiltration and buffer exchange using an Amicon Ultra-15 30 kDa ultrafiltration tube with 0.05M PBS buffer (pH=7.0), to obtain a PBS buffer solution of the conjugated product ADC-26 (5.0 mg/mL, 0.42 mL) for storage at -80°C.

The average DAR value n was calculated to be 6.5 using the reductive reverse-phase chromatography method.

**Biological Evaluation**

**Test Example 1: *In Vitro* Proliferation Inhibition Test of ADC Drugs on Tumor Cells**

I. Test Objective

An objective of this test example is to detect the *in vitro* proliferation inhibitory activity of some ADC drugs in the present disclosure against tumor cells with different expression levels. After 6 days of *in vitro* treatment of the cells with ADC drugs at various concentrations and culturing, the cell viability was detected using the CTG (CellTiter-Glo Luminescent Cell Viability Assay, Promega, Cat. No. G7571) reagent. Administration concentrations were plotted on the abscissa and response values were plotted on the ordinate to fit a four-parameter curve, and proliferation inhibitory effects of the ADC drugs on different tumor cells were evaluated based on $IC_{50}$ values.

II. Test Method

1. Culture of Cells

NCI-N87 cells (Cell Bank of Chinese Academy of Sciences, Cell No. TCHu130) were cultured in RPMI 1640 complete medium supplemented with 10% FBS; JIMT-1 cells (COBIOER, Cell No. CBP60378) were cultured in DMEM complete medium supplemented with 10% FBS; MDA-MB-468 cells (ATCC, Cell No. HTB-132) were cultured in DMEM complete medium supplemented with 10% FBS; SK-BR-3 cells (Cell Bank of Chinese Academy of Sciences, Cell No. TcHu225) were cultured in McCoy's 5A complete medium supplemented with 10% FBS; MDA-MB-231 cells (National Experimental Cell Resource Sharing Platform, Cell No. 1101HUM-PUMC000014) were cultured in RPMI 1640 complete

medium supplemented with 10% FBS; NCI-H2170 cells (Cell Bank of Chinese Academy of Sciences, Cell No. SCSP-5098) were cultured in RPMI 1640 complete medium supplemented with 10% FBS; and A431 cells (COBIOER, Cell No. CBP60330) were cultured in DMEM complete medium supplemented with 10% FBS.

2. Cell Preparation

Stably passaged cells were taken and washed twice with 5-10 mL of DPBS, and then 1-2 mL of 0.25% Trypsin-EDTA for digestion was added. After the cells were completely digested, a complete medium having a volume 2.5-3 times that of trypsin was added into the culture flask to terminate the digestion. The digested cells were transferred into a centrifuge tube and centrifuged at 1000 rpm for 5min. After centrifugation, the supernatant was discarded, the complete medium was added to resuspend the cells, the suspension was pipetted gently to mix the cells uniformly, and then the cells were counted.

3. Cell Seeding

The tumor cells were separately diluted to a concentration of $5.55 \times 10^4$ cells/mL with the complete medium, and 90 μL of the cell suspension was added to each predetermined well of a white opaque 96-well plate. Meanwhile, edge wells were filled with sterile DPBS at a volume of 200 μL per well. The plate was then placed in a 37°C incubator with 5% $CO_2$ for overnight adherent culture.

4. Sample Preparation

The ADC samples were prediluted to 1350 nM with the complete medium separately, followed by 5-fold serial dilution to obtain a total of 9 gradients. The diluted ADC samples were added to the predetermined wells of the 96-well plate at a volume of 10 μL per well with two replicate wells for each concentration, respectively. The complete medium was added to the cultured cells in Column 11 as a control group for normal cell growth.

5. Culture

After addition of the samples, sides of the plate were tapped gently several times to mix the samples and the medium uniformly, and then the plate was placed in a 37°C incubator with 5% $CO_2$ for incubation for 6 days.

6. Cell Viability Assay

After 6 days of culture following sample addition, the CellTiter-Glo® reagent was thawed and equilibrated together with the cell culture plate at room temperature for 30 minutes. Then 50 μL of the reagent was added to each well, the cell plate was oscillated in an oscillator for 2 minutes to lyse the cells, and the plate was left to be incubated at room temperature for 10 minutes to stabilize a luminescent signal.

7. Plate Reading

Luminescence values were measured using a BMG Omega microplate reader.

8. Analysis Method

The four-parameter curve was fitted with the final ADC concentration (nM) as the abscissa and the Luminescence value as the ordinate to calculate the $IC_{50}$ values, and the analytical software was Omega-Data Analysis.

III. Data Analysis

The results are shown in Tables 2 to 4 below.

Table 2 *In Vitro* Proliferation Inhibitory Activity of HER2 ADC Drugs on Tumor Cells

| Sample No. | $IC_{50}$ (nM) | | |
|---|---|---|---|
| | NCI-N87 | JIMT-1 | MDA-MB-468 |
| ADC-1 | 0.029 | 0.069 | N/A |

(continued)

| Sample No. | $IC_{50}$ (nM) | | |
|---|---|---|---|
| | NCI-N87 | JIMT-1 | MDA-MB-468 |
| ADC-2 | 0.032 | - | N/A |
| ADC-3 | 0.022 | 0.067 | N/A |
| ADC-4 | 0.033 | 0.092 | >50 |
| ADC-5 | 0.063 | - | >50 |
| ADC-6 | 0.022 | 0.037 | N/A |
| ADC-7 | 0.029 | - | >50 |
| ADC-8 | 0.010 | 0.083 | - |
| ADC-9 | <0.05 | <0.1 | - |
| ADC-10 | 0.018 | 0.153 | - |
| ADC-11 | 0.014 | 0.092 | - |
| ADC-12 | 0.111 | - | - |
| ADC-13 | 0.015 | 0.106 | - |
| ADC-14 | <0.05 | <0.1 | - |
| ADC-15 | <0.05 | <0.1 | - |
| ADC-16 | <0.05 | <0.1 | - |
| ADC-17 | 0.0205 | - | - |
| ADC-18 | 0.0221 | - | - |
| ADC-19 | 0.0145 | - | - |
| ADC-20 | 0.0172 | - | - |
| ADC-25 | 0.0342 | - | - |
| ADC-22 | N/A | - | - |

Conclusion: The ADC drugs conjugated with trastuzumab in the present disclosure exhibit significant proliferation inhibitory activity against the foregoing NCI-N87 and JIMT-1 cells and a weak proliferation inhibitory effect on HER2-negative MDA-MB-468 cells; and the negative antibody-conjugated ADC-22 shows no cytotoxic effect on NCI-N87 cells, demonstrating good safety profile.

Table 3 *In Vitro* Proliferation Inhibitory Activity of B7H4 ADC Drugs on Tumor Cells

| Sample No. | $IC_{50}$ (nM) | | |
|---|---|---|---|
| | SK-BR-3 | MDA-MB-231 | NCI-H2170 |
| ADC-23 | 0.0642 | 4.0727 | >40 |

Table 4 *In Vitro* Proliferation Inhibitory Activity of EGFR ADC Drugs on Tumor Cells

| Sample No. | $IC_{50}$ (nM) |
|---|---|
| | A431 |
| ADC-24 | 0.3137 |

Conclusion: The ADCs conjugated with antibodies targeting multiple antigens exhibit potent cytotoxic effects on the related cells with both high and low target expression, and show weak cytotoxicity against target-negative cells.

**Test Example 2: Evaluation of Bystander Killing Activity of ADC Drugs**

I. Test Objective

A bystander killing effect of ADC-4 on HER2-negative human breast cancer cell line MDA-MB-468 was studied via co-cultivation of HER2-overexpressing human gastric cancer cell line NCI-N87 and HER2-negative human breast cancer cell line MDA-MB-468.

II. Test Method

NCI-N87 cells and MDA-MB-468 cells were plated at a ratio of 3:1 as a co-cultivation group; NCI-N87 cells were plated independently as the NCI-N87 cell group; and MDA-MB-468 cells were plated independently as the MDA-MB-468 cell group. After the cells in each group adhered to the plate, 1.08 nM ADC-4 was added. Meanwhile, an untreated control group was set up for each group. After 6 days of incubation, the cells were detached by trypsinization and harvested, and the total number of viable cells was counted using a cell counter. Then the labeled HER2/ERBB2 Antibody (PE) (Sino Biological Inc., Cat. No. 10004-MM07T-P) was added as per 5 μL/well, and the cells were incubated at 4°C for 20 minutes. After centrifugation and washing, the cells were resuspended in DPBS, and a proportion of the two types of cells was analyzed by flow cytometry. Dot plot analysis was performed using FlowJo software (FlowJo_v10.8.1_CL). The killing rate of the ADC drug on NCI-N87 cells and MDA-MB-468 cells was calculated using Formula (I) and Formula (II) below, and data of each group were compared to evaluate the bystander killing effect of the ADC drug.

$$\text{NCI-N87 cell killing rate} = 1 - \frac{viable\ cell\ density \times volume \times Q3_{test\ sample}}{viable\ cell\ density \times volume \times Q3_{\text{Untreated}}} \times 100\% \quad \text{Formula I}$$

$$\text{MDA-MB-468 cell killing rate} = 1 - \frac{viable\ cell\ density \times volume \times Q4_{test\ sample}}{viable\ cell\ density \times volume \times Q4_{\text{Untreated}}} \times 100\% \quad \text{Formula II}$$

III. Test Results

Table 5 Results of Bystander Killing Effect Assay

| Group | Co-cultivation group | | NCI-N87 group | MDA-MB-468 group |
|---|---|---|---|---|
| | MDA-MB-468 cell killing rate | NCI-N87 cell killing rate | NCI-N87 cell killing rate | MDA-MB-468 cell killing rate |
| Control group | 0% | 0% | 0% | 0% |
| ADC-4 | 68% | 100% | 100% | -7% |

It can be learned from the results in Table 5 that the toxin conjugated to ADC-4 exhibits excellent transmembrane ability and can easily cross the cell membrane and exert the bystander killing effect. In this study, 1.08 nM ADC-4 exhibited almost no cytotoxic effect on the HER2-negative MDA-MB-468 cells when acting alone. When HER2-overexpressing NCI-N87 cells and HER2-negative MDA-MB-468 cells were mixed at a ratio of 3:1, 1.08 nM ADC-4 not only killed NCI-N87 cells but also achieved a 68% killing rate on MDA-MB-468 cells. These results indicate that the toxin released by cleavage after ADC-4 was internalized into NCI-N87 cells entered adjacent MDA-MB-468 cells via the bystander effect to exert its pharmacological effect, and such killing activity was independent of the antigen expression level of the target cells.

**Test Example 3: Efficacy Evaluation of ADC Drug in JIMT-1 Tumor-Bearing Mice**

I. Objective: This test example is intended to evaluate the anti-tumor effect of the ADC drug in a subcutaneous xenograft animal model of human breast cancer JIMT-1 cell line in female NOD/SCID mice.

II. Test Materials

1. Test Drug

ADC-3: 3 mg/kg

ADC-3: 5 mg/kg
Blank control: Normal saline

2. Preparation Method: All samples were diluted and prepared with normal saline.
3. Test Animals: Female NOD/SCID mice.

III. Test Method

5×$10^6$ JIMT-1 cells (0.1 mL per mouse) were subcutaneously inoculated into the right dorsal regions of NOD/SCID mice, and tumor growth was monitored regularly. When the tumors grew to an average volume of 150 $mm^3$, the mice were randomly grouped for administration based on tumor sizes and body weight. In the experiment, the mice were divided into a blank control group, a group receiving 3 mg/kg ADC-3 and a group receiving 5 mg/kg ADC-3, with 4 mice in each group. Administration was performed via tail vein injection on the day of grouping, which was recorded as Day 0. A single dose was administered, and the experiment lasted for 28 days. Efficacy was evaluated based on the relative tumor growth inhibition (TGI) rate, and safety was assessed according to changes in body weight and mortality of the animals.
After inoculation of the tumor cells, routine monitoring included tumor growth and effects of treatment on the normal behavior of the animals, specifically covering the activity, food and water intake, body weight gain or loss, and any abnormalities in the eyes, fur and other body parts of the experimental animals. All clinical symptoms observed in the experiment were recorded in the raw data. After the start of administration, the body weight and tumor sizes of the mice were measured twice a week.
The calculation formula is as follows:

$$\text{Tumor volume (mm}^3\text{)} = 1/2 \times (a \times b^2)$$

(a represents the longest diameter and b represents the shortest diameter); relative tumor growth inhibition rate (%) = (1 - T/C) × 100% (T/C% is the tumor proliferation rate, referring to the ratio of the relative tumor volume (or body weight) of the treatment group to that of the control group at a specific time point).
In the experiment, StudyDirectorTM (Version 3.1.399.19, Supplier: Studylog System, Inc.) was used for data collection, including the measurement of the longest and shortest diameters of tumors and the weighing of animal body weight. Raw data measured by balance and vernier caliper were directly imported into the software, and any changes in the data were recorded in this software. All procedures including drug administration, tumor measurement, and body weight weighing were performed in a biological safety cabinet or a clean bench.

IV. Test Results:

1. Efficacy Evaluation of the Test Drug in the JIMT-1 Human Breast Cancer Model:
The experimental results are shown in FIG. 1. A single dose was administered via the tail vein, and the experiment was terminated on the 28th day of observation. The tumor inhibition rate of ADC-3 (3 mg/kg) was 84.07%, the tumor inhibition rate of ADC-3 (5 mg/kg) was 99.32%, and 50% of tumors completely regressed (2/4). Both administration groups exhibited excellent tumor-inhibitory effects.
2. Safety Evaluation of the Test Drug in the JIMT-1 Human Breast Cancer Model:
As shown in FIG. 2, no body weight loss was observed in the test drug groups, no animal mortality occurred in the treatment groups, and each treatment group showed good tolerance throughout the treatment period.

The above examples represent only a few embodiments of the present disclosure. Although described in detail, they should not be construed as limiting the scope of the present disclosure. It should be noted that persons skilled in the art may make various modifications and improvements without departing from the spirit of the present disclosure, and such variations are within the scope of protection of the present disclosure

## Claims

**1.** A compound, or an isotopically labeled compound, an optical isomer, a geometric isomer, a tautomer, or a mixture of

isomers thereof, or a pharmaceutically acceptable salt or solvate thereof, wherein the compound comprises a structure represented by Formula (I):

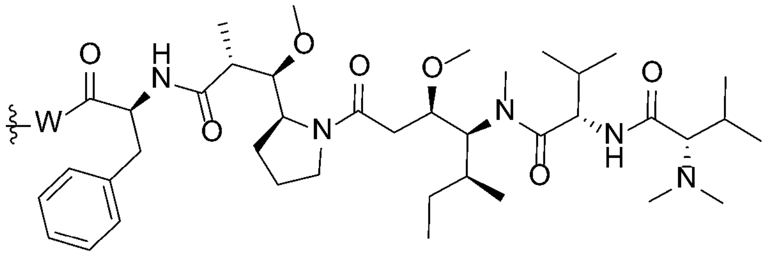

(I)

wherein W is selected from a single bond, $-X-(CH_2)_{m1}-CR_3R_4-NR_5-$, $-X-CR_3R_4-(CH_2)_{m1}-NR_5-$, $-X-(CH_2O)_{m2}-CR_3R_4-NR_5-$, $-X-CR_3R_4-(CH_2O)_{m2}-NR_5-$, $-X-(CH_2CH_2O)_{m3}-CR_3R_4-NR_5-$, $-X-(CH_2CH_2O)_{m3}-(CH_2)_{m1}-NR_5-$, $-X-CR_3R_4-(CH_2CH_2O)_{m3}-NR_5-$, $-X-(CH_2O)_{m2}-R_3R_4N-$, $-X-(CH_2)_{m1}-R_3R_4N-$, $-XR_5-(CH_2)_{m1}-R_3R_4N-$, $-X-(CH_2)_{m1}-(CH_2O)_{m2}-(CH_2)_{m4}-CR_3R_4-NR_5-$, or $-X-CR_3R_4-(CH_2)_{m1}-(CH_2O)_{m2}-(CH_2)_{m4}-NR_5-$;

X is selected from an alkylene group, an imino group, a nitrogen atom, an oxygen atom, or a sulfur atom;

$R_3$, $R_4$, and $R_5$ are each independently selected from a hydrogen atom, a deuterium atom, halogen, a hydroxyl group, an amino group, a cyano group, a nitro group, a sulfonyl group, a $C_1$ to $C_6$ alkyl group, or a $C_3$ to $C_6$ cycloalkyl group; or $R_3$ and $R_4$, together with the carbon atom to which they are attached, form a $C_3$ to $C_6$ cycloalkyl group; or $R_3$ and $R_4$, together with the carbon atom and the nitrogen atom to which they are attached, form a 3- to 6-membered heterocyclic group, wherein in $R_3$, $R_4$, and $R_5$, one or more hydrogen atoms in the $C_1$ to $C_6$ alkyl group or $C_3$ to $C_6$ cycloalkyl group are optionally substituted with a deuterium atom, halogen, a hydroxyl group, a cyano group, an amino group, a $C_1$ to $C_3$ alkyl group, or a phenyl group; m1, m2, m3, and m4 are each independently selected from an integer from 0 to 6; and

the wavy line attached to W in Formula (I) represents a hydrogen atom or a covalent linkage to a linker unit and/or a structure connecting an antibody or an antigen-binding fragment thereof.

2. The compound of claim 1, wherein W is selected from $-X-(CH_2)_{m1}-CR_3R_4-NR_5-$, $-X-CR_3R_4-(CH_2)_{m1}-NR_5-$, $-X-(CH_2CH_2O)_{m3}-(CH_2)_{m1}-NR_5-$, $-X-(CH_2)_{m1}-R_3R_4N-$, $-XR_5-(CH_2)_{m1}-R_3R_4N-$, $-X-(CH_2)_{m1}-(CH_2O)_{m2}-(CH_2)_{m4}-CR_3R_4-NR_5-$, or $-X-CR_3R_4-(CH_2)_{m1}-(CH_2O)_{m2}-(CH_2)_{m4}-NR_5-$; X is selected from an imino group, a nitrogen atom, or an oxygen atom;

$R_3$ and $R_4$ are each independently selected from a hydrogen atom, a deuterium atom, halogen, a $C_1$ to $C_6$ alkyl group, or a $C_3$ to $C_6$ cycloalkyl group; or $R_3$ and $R_4$, together with the carbon atom to which they are attached, form a $C_3$ to $C_6$ cycloalkyl group; or $R_3$ and $R_4$, together with the carbon atom and the nitrogen atom to which they are attached, form a 3- to 6-membered heterocyclic group, wherein in $R_3$ and $R_4$, one or more hydrogen atoms in the $C_1$ to $C_6$ alkyl group or $C_3$ to $C_6$ cycloalkyl group are optionally substituted with a deuterium atom, halogen, a $C_1$ to $C_3$ alkyl group, or a phenyl group;

$R_5$ is selected from a hydrogen atom, a deuterium atom, or halogen; and

m1, m2, and m4 are each independently selected from an integer from 1 to 4.

3. The compound of claim 1, wherein W is selected from any one of the structures shown below:

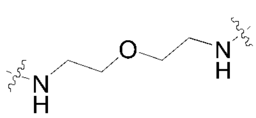 , 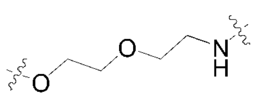 , 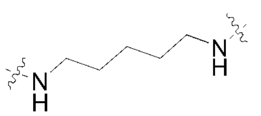 , 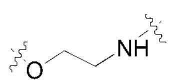 ,

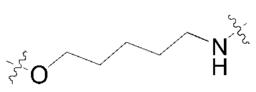 , 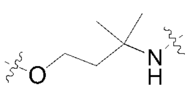 , 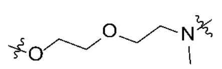 , 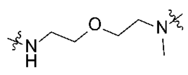 , 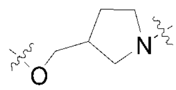 ,

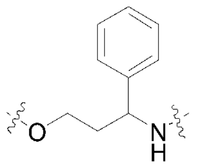, 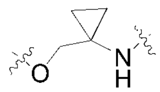, 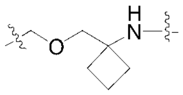, 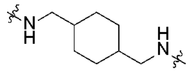, 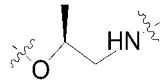,

or

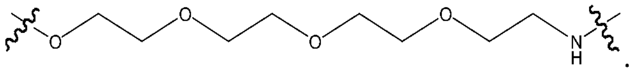.

**4.** The compound of any one of claims 1 to 3, wherein the compound comprises a structure represented by Formula (II):

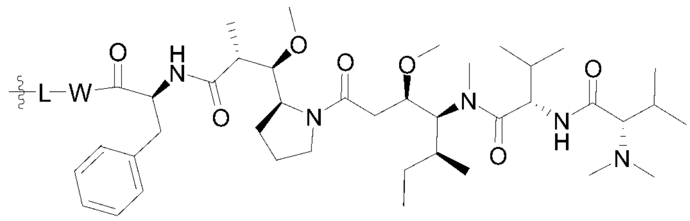

(II)

wherein W is as defined in any one of claims 1 to 3;
L is selected from $^{NH}$-Val-Cit-$^{C=O}$, $^{NH}$-Val-Ala-$^{C=O}$, $^{NH}$-Val-Lys-$^{C=O}$, $^{NH}$-Val-Lys(Ac)-$^{C=O}$, $^{NH}$-Phe-Lys-$^{C=O}$, $^{NH}$-Phe-Lys(Ac)-$^{C=O}$, $^{NH}$-D-Val-Leu-Lys-$^{C=O}$, $^{NH}$-Gly-Gly-Arg-$^{C=O}$, $^{NH}$-Ala-Ala-Asru-$^{C=O}$, $^{NH}$-Gly-Gly-Gly-$^{C=O}$, $^{NH}$-Gly-Gly-Gly-Gly-Gly-$^{C=O}$, or L1 or L2 shown below, preferably L1 or L2;

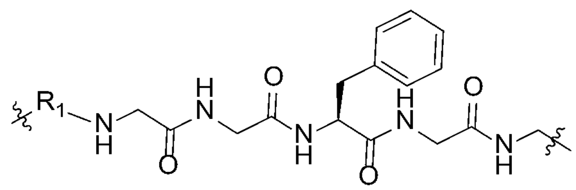

L1

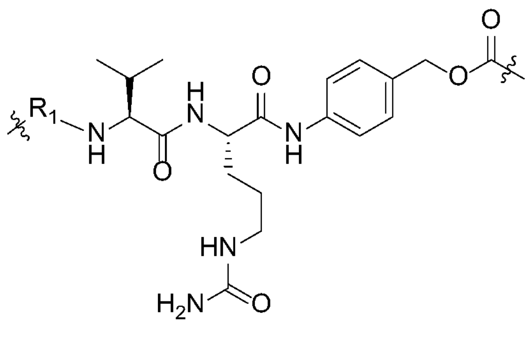

L2

in L1 and L2, $R_1$ is a single bond or has the following structure:

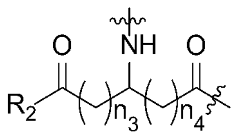

wherein $n_3$ and $n_4$ are each independently an integer from 0 to 6, and $R_2$ is a hydrophilic group or a structure having a hydrophilic group; and
the wavy line attached to L in Formula (II) represents a hydrogen atom or a covalent linkage to a structure

connecting an antibody or an antigen-binding fragment thereof.

5. The compound of claim 4, wherein $R_2$ is a hydroxyl group or a structure having at least three oxygen-containing hydrophilic groups; preferably, the oxygen-containing hydrophilic groups are the same or different and each independently selected from -O- or a hydroxyl group;
preferably, $R_2$ is any one of the following structures:

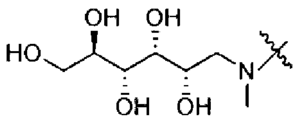 , 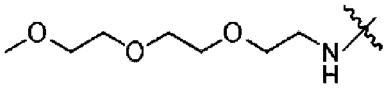 , 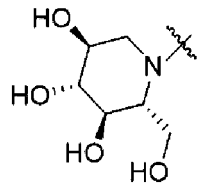 ,

or

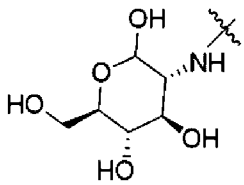 .

6. The compound of claim 4, wherein $R_1$ is a single bond or selected from the following structures:

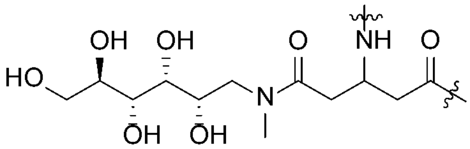 , 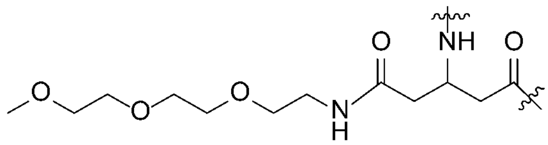 ,

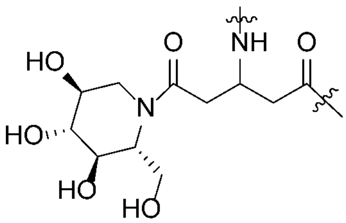 , 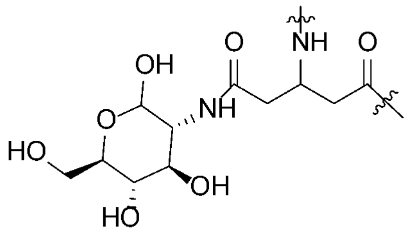 ,

or

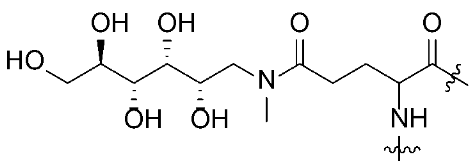 .

7. The compound of any one of claims 4 to 6, wherein when L is L1, X in W is an oxygen atom; and when L is L2, X in W is an imino group.

8. The compound of any one of claims 4 to 7, wherein the compound comprises a structure represented by Formula (III):

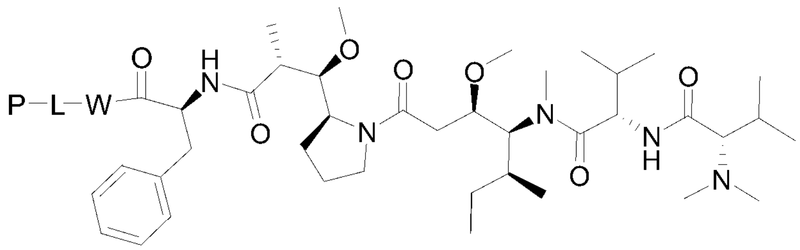

(III)

wherein P is a structure connecting an antibody or an antigen-binding fragment thereof; and L and W are as defined in any one of claims 4 to 7.

**9.** The compound of claim 8, wherein P is selected from P1, P2, or P3:

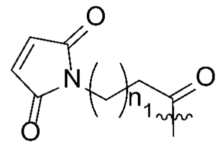

P1

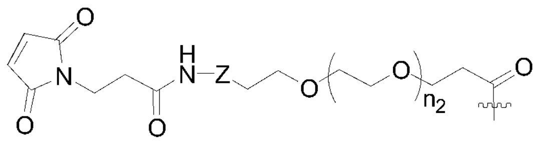

P2

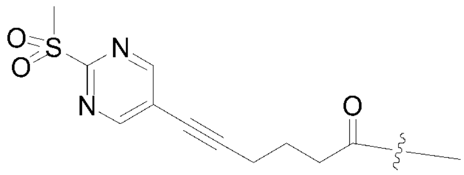

P3

wherein $n_1$ is an integer from 1 to 10, preferably an integer from 2 to 6;
$n_2$ is an integer from 1 to 12, preferably an integer from 5 to 9; and
Z is selected from a bond, a $C_1$ to $C_6$ alkylene group, an imino group, an ester group, an amide group, a sulfonyl group, a sulfonamide group, a ketone group, a urea group, or a phosphate group.

**10.** The compound of claim 9, wherein the compound has a structure as shown in any one of the following:

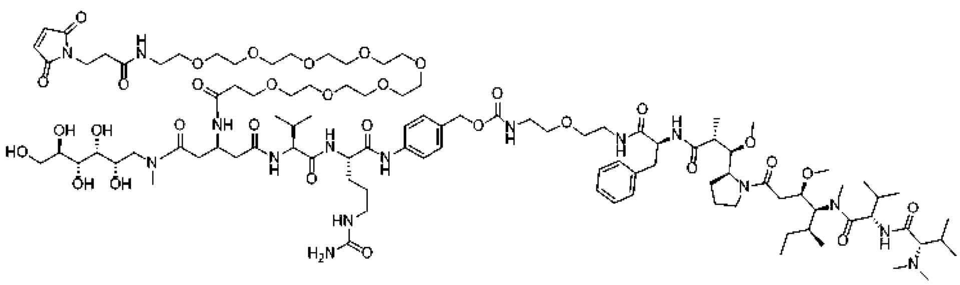

DL-1

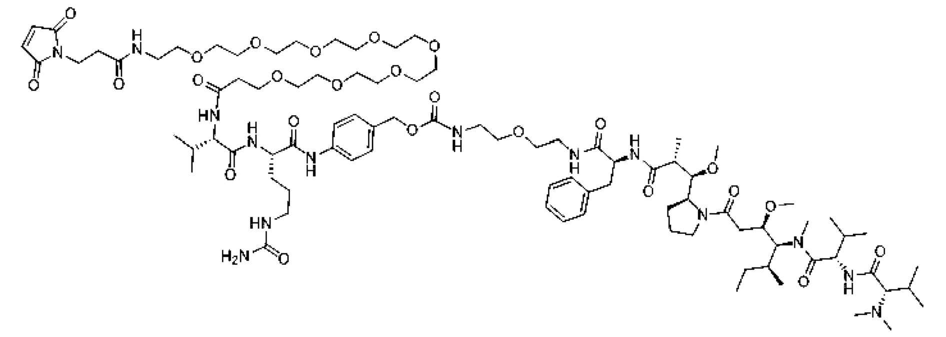

DL-2

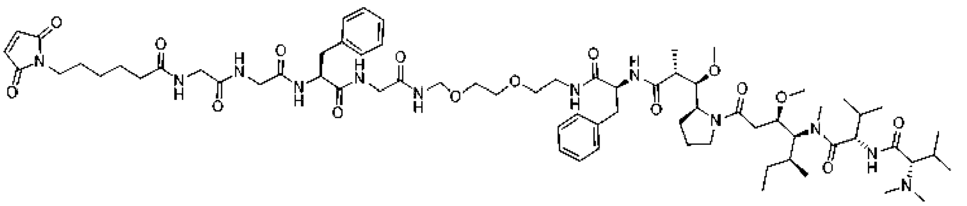

DL-3

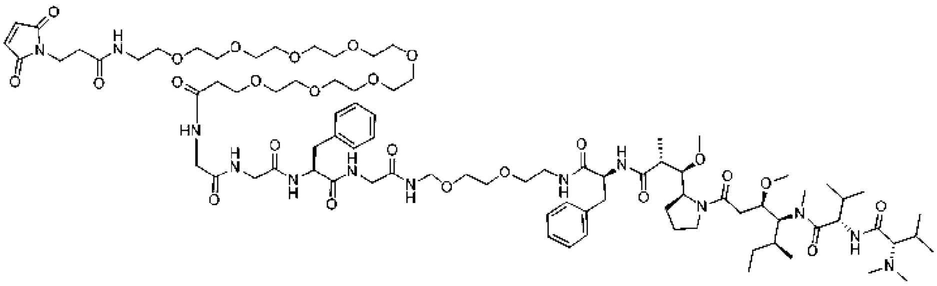

DL-4

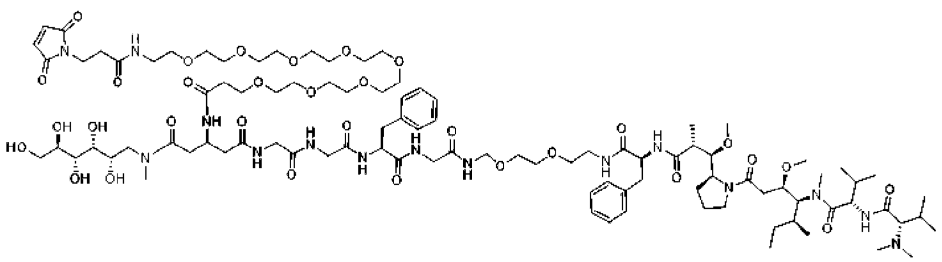

DL-5

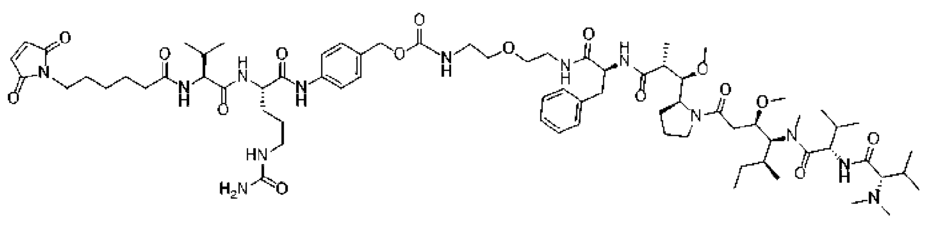

DL-6

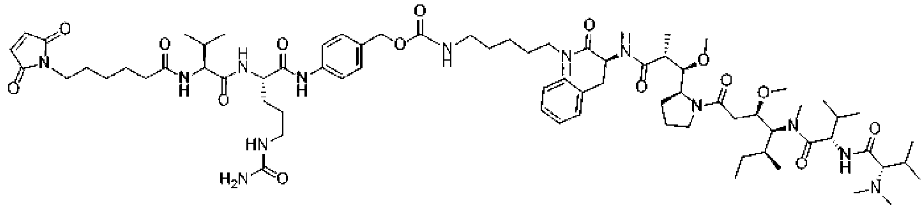

DL-7

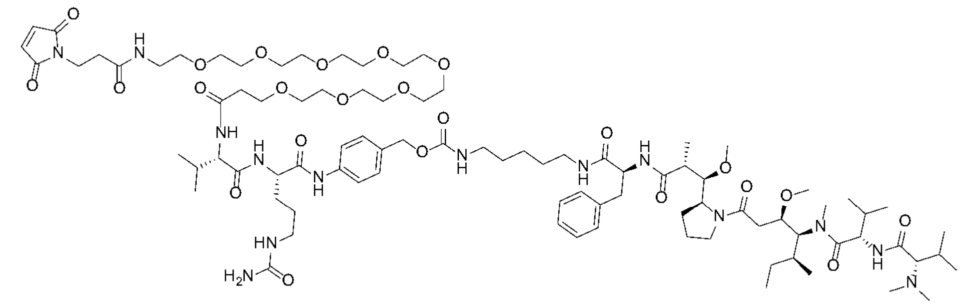

DL-8

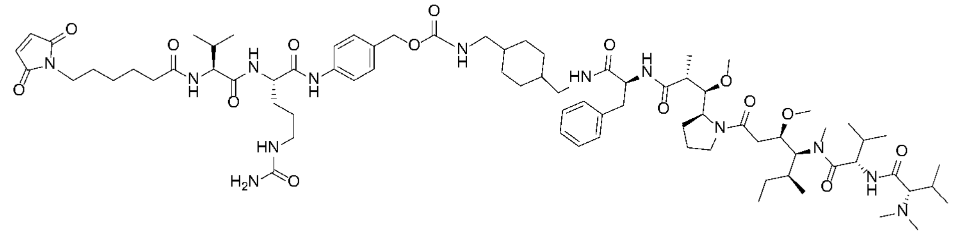

DL-9

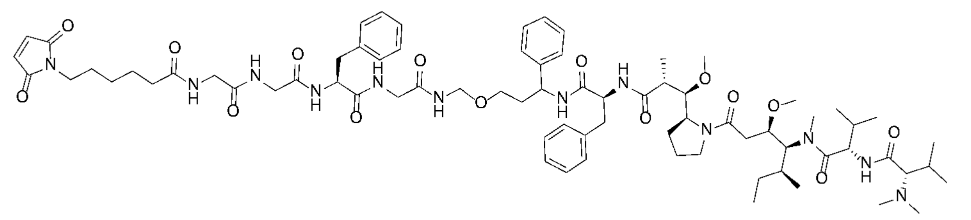

DL-10

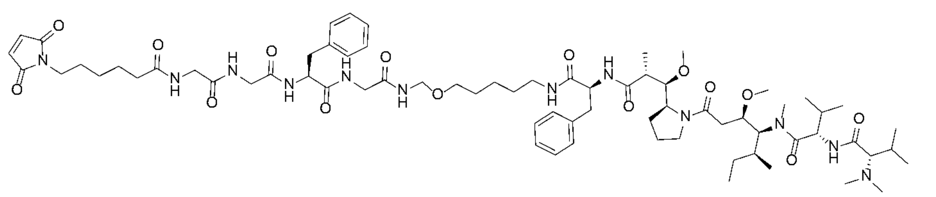

DL-11

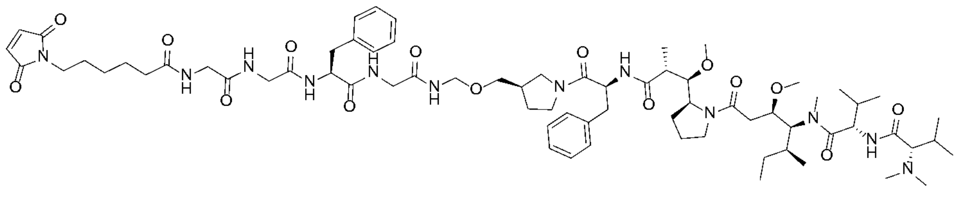

DL-12

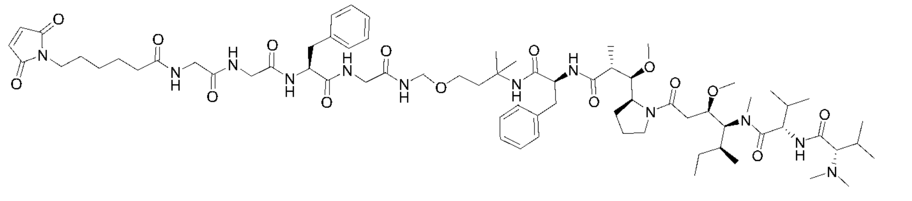

DL-13

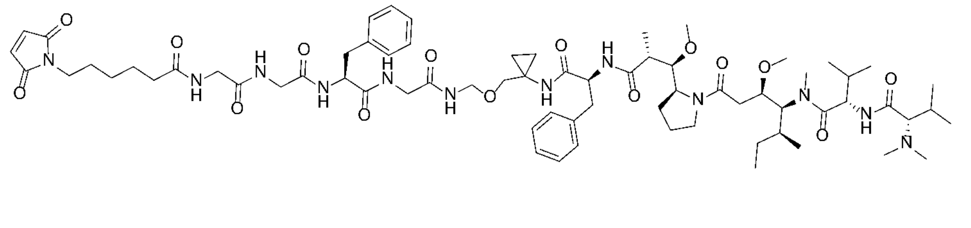

DL-14

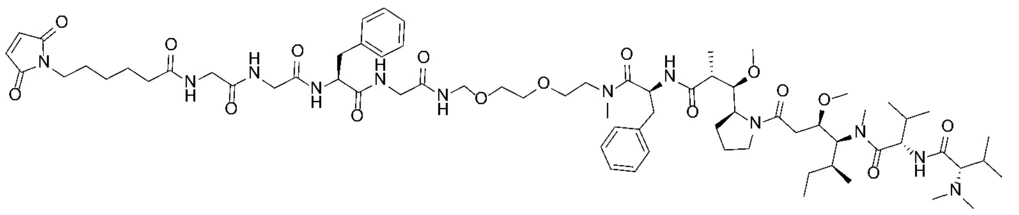

DL-15

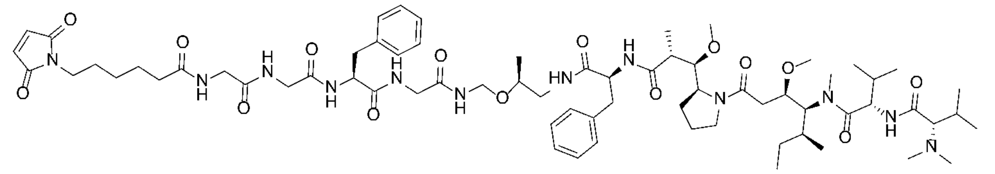

DL-16

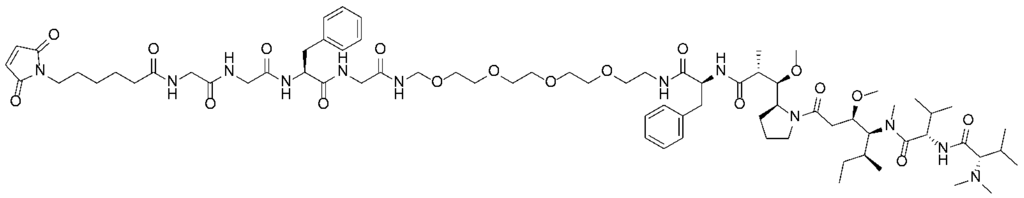

DL-17

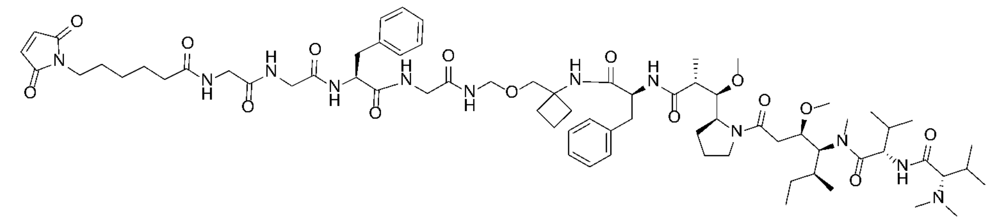

DL-18

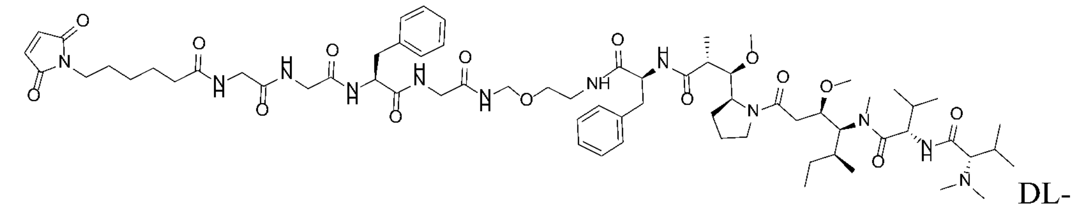

DL-19

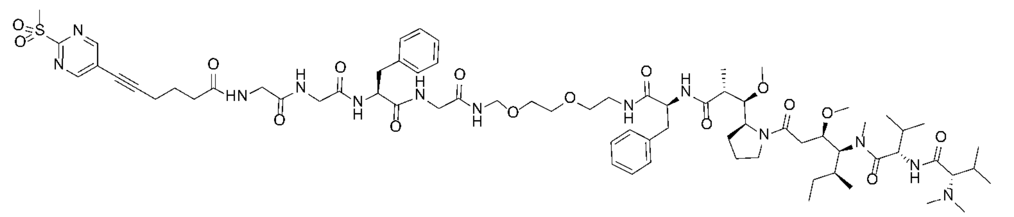

DL-20

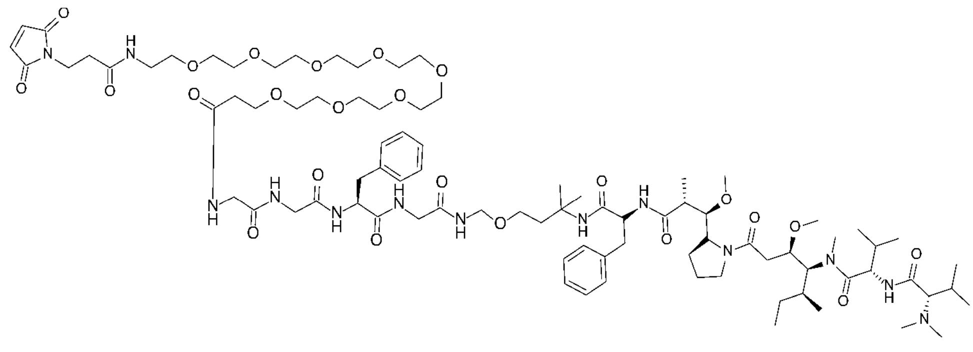

DL-21.

**11.** An antibody-drug conjugate, comprising:

i) an antibody or an antigen-binding fragment thereof; and
ii) the compound, or the isotopically labeled compound, the optical isomer, the geometric isomer, the tautomer, or the mixture of isomers thereof, or the pharmaceutically acceptable salt or solvate thereof of any one of claims 1 to 10.

**12.** The antibody-drug conjugate of claim 11, wherein the antibody-drug conjugate has a structure represented by Formula (IV):

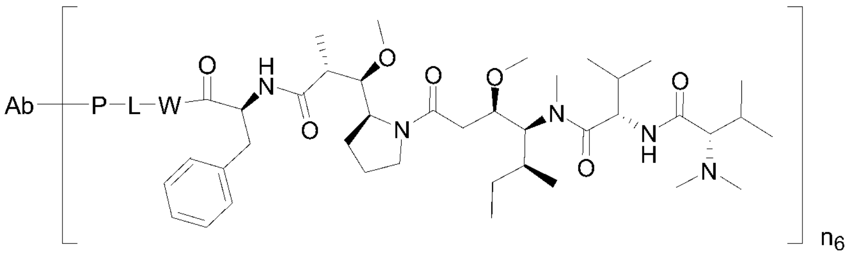

(IV)

wherein Ab is an antibody or an antigen-binding fragment thereof; and
$n_6$ is selected from an integer or a decimal from 1 to 10.

**13.** The antibody-drug conjugate of claim 12, wherein $n_6$ is selected from an integer or a decimal from 1 to 8, preferably, $n_6$ is selected from an integer or a decimal from 6 to 8.

**14.** The antibody-drug conjugate of any one of claims 11 to 13, wherein the antibody targets one or more selected from the group consisting of HER2, HER3, B7H3, B7H4, EGFR, Claudin 18.2, c-MET, Nectin-4, ROR1, GPNMB, CD56, TACSTD2 (TROP2), CEACAM5, folate receptor-$\alpha$, mesothelin, ENPP3, guanylate cyclase C, SLC44A4, NaPi2b, CD70, mucin 1, STEAP1, connexin 4, 5T4, SLTRK6, SC-16, LIV-1, P-cadherin, PSMA, fibronectin extra domain B, endothelin receptor ETB, tenascin C, collagen IV, VEGFR2, periostin, CD30, CD79b, CD19, CD22, CD138, CD37, CD33, CD74, and CD98; and
preferably, the antibody is a monoclonal antibody.

**15.** A pharmaceutical composition comprising the compound, or the isotopically labeled compound, the optical isomer, the geometric isomer, the tautomer, or the mixture of isomers thereof, or the pharmaceutically acceptable salt or solvate thereof of any one of claims 1 to 10, or the antibody-drug conjugate of any one of claims 11 to 14; and one or more pharmaceutically acceptable excipients.

**16.** Use of the compound, or the isotopically labeled compound, the optical isomer, the geometric isomer, the tautomer, or the mixture of isomers thereof, or the pharmaceutically acceptable salt or solvate thereof of any one of claims 1 to 10, or the antibody-drug conjugate of any one of claims 11 to 14 in preparation of a medicament.

**17.** The use of claim 16, wherein the medicament is used for prevention or treatment of a tumor, preferably, the tumor is a cancer associated with expression of one or more selected from the group consisting of HER2, HER3, B7H3, B7H4,

EGFR, Claudin 18.2, c-MET, Nectin-4, ROR1, GPNMB, CD56, TACSTD2 (TROP2), CEACAM5, folate receptor-α, mesothelin, ENPP3, guanylate cyclase C, SLC44A4, NaPi2b, CD70, mucin 1, STEAP1, connexin 4, 5T4, SLTRK6, SC-16, LIV-1, P-cadherin, PSMA, fibronectin extra domain B, endothelin receptor ETB, tenascin C, collagen IV, VEGFR2, periostin, CD30, CD79b, CD19, CD22, CD138, CD37, CD33, CD74, and CD98; more preferably, the tumor is one or more selected from the group consisting of breast cancer, ovarian cancer, cervical cancer, uterine cancer, prostate cancer, renal cancer, urethral cancer, bladder cancer, liver cancer, gastric cancer, endometrial cancer, salivary gland cancer, esophageal cancer, melanoma, glioma, neuroblastoma, sarcoma, lung cancer, colon cancer, rectal cancer, colorectal cancer, leukemia, bone cancer, skin cancer, thyroid cancer, pancreatic cancer, and lymphoma.

**18.** A method for preventing or treating a tumor, comprising: administering an effective amount of the compound, or the isotopically labeled compound, the optical isomer, the geometric isomer, the tautomer, or the mixture of isomers thereof, or the pharmaceutically acceptable salt or solvate thereof of any one of claims 1 to 10, or the antibody-drug conjugate of any one of claims 11 to 14, or the pharmaceutical composition of claim 15.

**19.** The method for preventing or treating a tumor of claim 18, wherein the tumor is a cancer associated with expression of one or more selected from the group consisting of HER2, HER3, B7H3, B7H4, EGFR, Claudin 18.2, c-MET, Nectin-4, ROR1, GPNMB, CD56, TACSTD2 (TROP2), CEACAM5, folate receptor-α, mesothelin, ENPP3, guanylate cyclase C, SLC44A4, NaPi2b, CD70, mucin 1, STEAP1, connexin 4, 5T4, SLTRK6, SC-16, LIV-1, P-cadherin, PSMA, fibronectin extra domain B, endothelin receptor ETB, tenascin C, collagen IV, VEGFR2, periostin, CD30, CD79b, CD19, CD22, CD138, CD37, CD33, CD74, and CD98; preferably, the tumor is one or more selected from the group consisting of breast cancer, ovarian cancer, cervical cancer, uterine cancer, prostate cancer, renal cancer, urethral cancer, bladder cancer, liver cancer, gastric cancer, endometrial cancer, salivary gland cancer, esophageal cancer, melanoma, glioma, neuroblastoma, sarcoma, lung cancer, colon cancer, rectal cancer, colorectal cancer, leukemia, bone cancer, skin cancer, thyroid cancer, pancreatic cancer, and lymphoma.

**20.** A method for preparing the compound of any one of claims 1 to 10, wherein reaction raw materials comprise a compound containing a structure represented by Formula (I);
preferably, the preparation method comprises:

reacting a compound containing an L structure and the structure represented by Formula (I) with a compound containing a P structure to obtain the compound; or
reacting a compound containing the P structure and the L structure with a compound containing the structure represented by Formula (I) to obtain the compound.

**21.** The method of claim 20, comprising:

reacting a compound containing the L structure with a compound containing the structure represented by Formula (I) to obtain a compound containing the L structure and the structure represented by Formula (I), which is then reacted with a compound containing the P structure to obtain the compound; or
reacting a compound containing the P structure with a compound containing the L structure to obtain a compound containing the P structure and the L structure, which is then reacted with a compound containing the structure represented by Formula (I) to obtain the compound.

**22.** A method for preparing the antibody-drug conjugate of any one of claims 11 to 14, comprising: coupling the following i) with ii):

(i) an antibody or an antigen-binding fragment thereof; and
(ii) the compound, or the isotopically labeled compound, the optical isomer, the geometric isomer, the tautomer, or the mixture of isomers thereof, or the pharmaceutically acceptable salt or solvate thereof of any one of claims 1 to 10.

**23.** The method of claim 22, comprising: reducing an interchain disulfide bond of the antibody or the antigen-binding fragment thereof to a free sulfhydryl group, which is then conjugated to a P-terminus of the compound, or the isotopically labeled compound, the optical isomer, the geometric isomer, the tautomer, or the mixture of isomers thereof, or the pharmaceutically acceptable salt or solvate thereof.

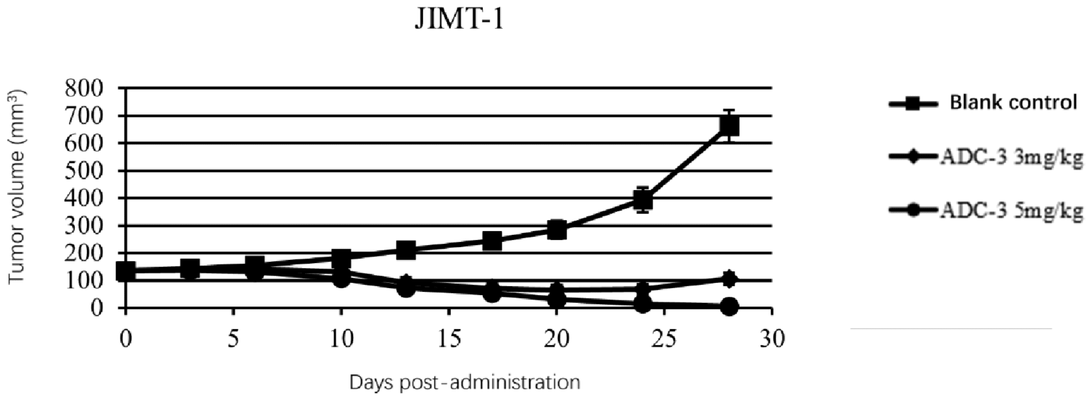
JIMT-1
Tumor volume (mm³)
800
700
600
500
400
300
200
100
0
0
5
10
15
20
25
30
Days post-administration
Blank control
ADC-3 3mg/kg
ADC-3 5mg/kg

FIG. 1

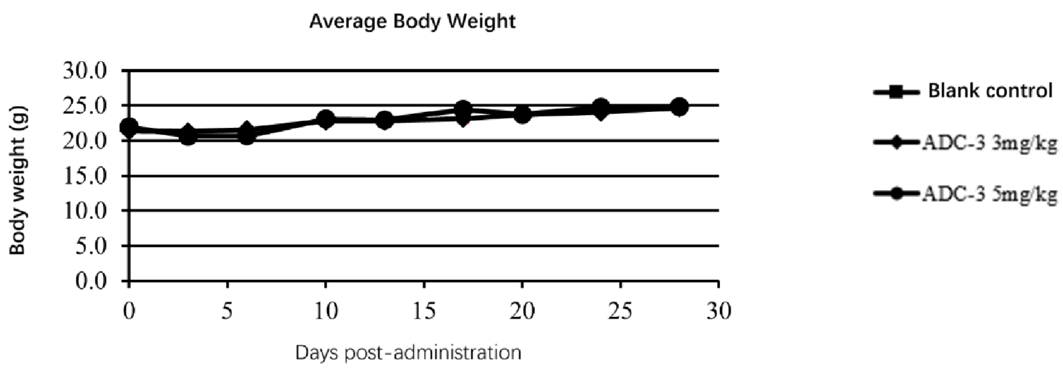
Average Body Weight
Body weight (g)
30.0
25.0
20.0
15.0
10.0
5.0
0.0
0
5
10
15
20
25
30
Days post-administration
Blank control
ADC-3 3mg/kg
ADC-3 5mg/kg

FIG. 2

**INTERNATIONAL SEARCH REPORT**

International application No.
**PCT/CN2024/116631**

**A. CLASSIFICATION OF SUBJECT MATTER**

A61K47/68(2017.01)i; C07K7/02(2006.01)i; A61K38/08(2019.01)i; A61P35/00(2006.01)i; A61P35/02(2006.01)i

According to International Patent Classification (IPC) or to both national classification and IPC

**B. FIELDS SEARCHED**

Minimum documentation searched (classification system followed by classification symbols)

IPC: A61K, C07K, A61P

Documentation searched other than minimum documentation to the extent that such documents are included in the fields searched

Electronic data base consulted during the international search (name of data base and, where practicable, search terms used)

CNABS, CNTXT, DWPI, VEN, ENTXTC, ENTXT, CNKI, PUBMED, ISI_Web of Science, Science Direct, STNext: 诺灵生物医药科技(北京)有限公司, 奥瑞他汀, Auristatin, 偶联物, ADC, conjugat+, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| PX | CN 117430660 A (NUOLING BIOMEDICAL TECHNOLOGY (BEIJING) CO., LTD.) 23 January 2024 (2024-01-23)<br>abstract, and claims 1-24 | 1-23 |
| X | CN 109963620 A (LINXIS B.V.) 02 July 2019 (2019-07-02)<br>abstract, claims 1-29, and description, paragraphs 168, 190, 195, 203 and 209, and figures 1 and 3 | 1-9, 11-23 |
| X | CN 108727466 A (AMBRX INC.) 02 November 2018 (2018-11-02)<br>abstract, and description, embodiment 12 | 1 |
| X | WO 2019125154 A2 (LINXIS B.V.) 27 June 2019 (2019-06-27)<br>abstract, claims 1-19, and description, page 81, embodiment 7 | 1-9, 11-23 |
| X | CN 108201625 A (INDUSTRIAL TECIINOLOGY RESEARCII INSTITUTE) 26 June 2018 (2018-06-26)<br>abstract, claims 1-19, and embodiments 9-10 | 1-9, 11-23 |
| A | WO 2009117531 A1 (SEATTLE GENETICS INC. et al.) 24 September 2009 (2009-09-24)<br>abstract, claims 1-57, and embodiments 10-32 | 1-23 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 December 2024** | **09 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/116631**

**C. DOCUMENTS CONSIDERED TO BE RELEVANT**

| Category* | Citation of document, with indication, where appropriate, of the relevant passages | Relevant to claim No. |
|---|---|---|
| A | CN 106999605 A (ANTIKOR BIOPHARMA LIMITED) 01 August 2017 (2017-08-01)<br>abstract, claims 1-54, and embodiment 50 | 1-23 |
| A | WO 2018192407 A1 (SICHUAN KELUN-BIOTECH BIOPHARMACEUTICAL CO., LTD.) 25 October 2018 (2018-10-25)<br>abstract, and claims 1-82 | 1-23 |

Form PCT/ISA/210 (second sheet) (July 2022)

**INTERNATIONAL SEARCH REPORT**

International application No.
**PCT/CN2024/116631**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **18-19**
because they relate to subject matter not required to be searched by this Authority, namely:

Claims 18-19 relate to a method for preventing or treating tumors, which falls within subject matter for which no search is required by the International Searching Authority as defined in PCT Rule 39.1(iv). A search is performed on the basis that the designation of the subject matter thereof is a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/116631**

| Patent document cited in search report | | | Publication date (day/month/year) | Patent family member(s) | | | Publication date (day/month/year) |
|---|---|---|---|---|---|---|---|
| CN | 117430660 | A | 23 January 2024 | None | | | |
| CN | 109963620 | A | 02 July 2019 | CA | 3026815 | A1 | 14 December 2017 |
| | | | | WO | 2017213494 | A1 | 14 December 2017 |
| | | | | US | 2019298846 | A1 | 03 October 2019 |
| | | | | JP | 2019517565 | A | 24 June 2019 |
| | | | | EP | 3463578 | A1 | 10 April 2019 |
| | | | | AU | 2017278573 | A1 | 03 January 2019 |
| CN | 108727466 | A | 02 November 2018 | JP | 2015521590 | A | 30 July 2015 |
| | | | | JP | 6425650 | B2 | 21 November 2018 |
| | | | | US | 2022033518 | A1 | 03 February 2022 |
| | | | | MX | 2019002732 | A | 23 February 2023 |
| | | | | NZ | 703298 | A | 29 April 2016 |
| | | | | DK | 2858676 | T3 | 13 May 2019 |
| | | | | IL | 258937 | A | 28 June 2018 |
| | | | | IL | 258937 | B | 29 August 2019 |
| | | | | EP | 2858676 | A1 | 15 April 2015 |
| | | | | EP | 2858676 | B1 | 13 March 2019 |
| | | | | ES | 2725329 | T3 | 23 September 2019 |
| | | | | IL | 268538 | A | 26 September 2019 |
| | | | | IL | 268538 | B | 30 November 2020 |
| | | | | SG | 10201702176 | XA | 27 April 2017 |
| | | | | MX | 2023010485 | A | 18 September 2023 |
| | | | | WO | 2013185117 | A1 | 12 December 2013 |
| | | | | IL | 235895 | A0 | 29 January 2015 |
| | | | | IL | 235895 | B | 31 May 2018 |
| | | | | MX | 2014015029 | A | 03 March 2015 |
| | | | | MX | 363116 | B | 11 March 2019 |
| | | | | CA | 2874854 | A1 | 12 December 2013 |
| | | | | CA | 2874854 | C | 14 March 2023 |
| | | | | SG | 11201408153 | YA | 29 January 2015 |
| | | | | US | 2015152190 | A1 | 04 June 2015 |
| | | | | US | 10800856 | B2 | 13 October 2020 |
| | | | | NZ | 713941 | A | 24 February 2017 |
| | | | | AU | 2017200877 | A1 | 02 March 2017 |
| | | | | AU | 2017200877 | B2 | 30 August 2018 |
| | | | | AU | 2013270686 | A1 | 22 January 2015 |
| | | | | AU | 2013270686 | B2 | 10 November 2016 |
| | | | | EP | 3536700 | A1 | 11 September 2019 |
| | | | | KR | 20150023563 | A | 05 March 2015 |
| | | | | KR | 102143664 | B1 | 11 August 2020 |
| WO | 2019125154 | A2 | 27 June 2019 | NL | 2020121 | B1 | 26 June 2019 |
| | | | | US | 2020345862 | A1 | 05 November 2020 |
| | | | | JP | 2021508737 | A | 11 March 2021 |
| | | | | EP | 3727465 | A2 | 28 October 2020 |
| | | | | WO | 2019125154 | A3 | 08 August 2019 |
| CN | 108201625 | A | 26 June 2018 | TW | 201822820 | A | 01 July 2018 |
| | | | | EP | 3335734 | A1 | 20 June 2018 |
| | | | | US | 2018169262 | A1 | 21 June 2018 |
| | | | | US | 10864279 | B2 | 15 December 2020 |
| | | | | JP | 2018138538 | A | 06 September 2018 |

Form PCT/ISA/210 (patent family annex) (July 2022)

**INTERNATIONAL SEARCH REPORT**
**Information on patent family members**

International application No.
**PCT/CN2024/116631**

| Patent document cited in search report | Publication date (day/month/year) | Patent family member(s) | Publication date (day/month/year) |
|---|---|---|---|
| | | JP 6771450 B2 | 21 October 2020 |
| WO 2009117531 A1 | 24 September 2009 | ES 2523033 T3 | 20 November 2014 |
| | | PL 2265283 T3 | 31 March 2015 |
| | | DK 2265283 T3 | 20 October 2014 |
| | | HK 1207976 A1 | 19 February 2016 |
| | | CA 2718942 A1 | 24 September 2009 |
| | | PL 2842575 T3 | 28 February 2018 |
| | | DK 2842575 T3 | 27 November 2017 |
| | | US 2011020343 A1 | 27 January 2011 |
| | | US 8609105 B2 | 17 December 2013 |
| | | EP 2842575 A1 | 04 March 2015 |
| | | EP 2842575 B1 | 27 September 2017 |
| | | US 2014050746 A1 | 20 February 2014 |
| | | US 9463252 B2 | 11 October 2016 |
| | | ES 2647927 T3 | 27 December 2017 |
| | | NO 2842575 T3 | 24 February 2018 |
| | | EP 2265283 A1 | 29 December 2010 |
| | | EP 2265283 A4 | 25 April 2012 |
| | | EP 2265283 B1 | 03 September 2014 |
| CN 106999605 A | 01 August 2017 | JP 2021063124 A | 22 April 2021 |
| | | US 2022233706 A1 | 28 July 2022 |
| | | JP 2018502047 A | 25 January 2018 |
| | | JP 6947630 B2 | 13 October 2021 |
| | | GB 201416960 D0 | 12 November 2014 |
| | | AU 2015323539 A1 | 06 April 2017 |
| | | AU 2015323539 B2 | 21 January 2021 |
| | | EP 3197501 A1 | 02 August 2017 |
| | | EP 3197501 B1 | 10 April 2024 |
| | | WO 2016046574 A1 | 31 March 2016 |
| | | CA 2999384 A1 | 31 March 2016 |
| | | ES 2982012 T3 | 14 October 2024 |
| | | US 2017360951 A1 | 21 December 2017 |
| | | US 11202837 B2 | 21 December 2021 |
| WO 2018192407 A1 | 25 October 2018 | EP 3617221 A1 | 04 March 2020 |
| | | EP 3617221 A4 | 14 April 2021 |
| | | US 2020179529 A1 | 11 June 2020 |
| | | US 11207420 B2 | 28 December 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **STAHL** ; **WERMUTH**. Handbook of Pharmaceutical Salts: Properties, Selection, and Use. Wiley-VCH, 2002
- **T.W. GREENE** ; **20 P.G.M. WUTS**. Protective Groups in Organic Synthesis. John Wiley & Sons, 1991